# EUROPEAN PATENT APPLICATION

(11) **EP 4 834 369 A2**
(43) Date of publication of application: **04.11.2026**
(21) Application number: 26194440.9
(22) Date of filing: 24.04.2020
(51) Int. Cl.: A61K 47/36

(54) **COMPOSITIONS AND METHODS FOR MODULATING COMPLEMENT ACTIVITY**

(30) Priority: 24.04.2019 US 201962837978 P; 12.12.2019 US 201962947188 P; 12.12.2019 US 201962947183 P
(62) Document number(s) of the earlier application(s) in accordance with Art. 76 EPC: 20730497.3 / 3 958 842
(71) Applicant: UCB Holdings, Inc., Smyrna, GA 30080 (US)
(72) Inventor: READ, Simon J., Lexington, Massachusetts, 02421 (US); THACKABERRY, Evan, Groton, Massachusetts, 01450 (US); WANG, Hong, Newton, Massachusetts, 02465 (US)
(74) Representative: Hoffmann Eitle

(57) **Abstract**

Embodiments of the disclosure provide sustained release formulations that include active ingredients and release modulating matrices. Also provided are related methods of preparation and methods of addressing therapeutic indications with sustained release formulations described herein. Included are sustained release formulations with complement inhibitors (e.g., C5 inhibitory cyclic polypeptides, zilucoplan, and/or active metabolites or variants thereof) for treating complement-related indications.

## Description

### CROSS REFERENCE TO RELATED APPLICATIONS

This application claims priority to United States Provisional Patent Application Number 62/837,978 filed on April 24, 2019 entitled COMPOSITIONS AND METHODS FOR MODULATING COMPLEMENT ACTIVITY, United States Provisional Patent Application Number 62/947,188 filed on December 12, 2019 entitled COMPOSITIONS AND METHODS FOR MODULATING COMPLEMENT ACTIVITY, and United States Provisional Patent Application Number 62/947,183 filed on December 12, 2019 entitled COMPOSITIONS AND METHODS FOR MODULATING COMPLEMENT ACTIVITY, the contents of each of which are herein incorporated by reference in their entirety.

### SEQUENCE LISTING

The present application is being filed along with a Sequence Listing in electronic format. The Sequence Listing file, entitled 2011_1027PCT_SL.txt, was created on April 20, 2020 and is 1,191 bytes in size. The information in electronic format of the Sequence Listing is incorporated herein by reference in its entirety.

### BACKGROUND

The vertebrate immune response is comprised of adaptive and innate immune components. While the adaptive immune response is selective for particular pathogens and is slow to respond, components of the innate immune response recognize a broad range of pathogens and respond rapidly upon infection. One such component of the innate immune response is the complement system.

The complement system includes about 20 circulating complement component proteins, synthesized primarily by the liver. Components of this particular immune response were first termed "complement" due to the observation that they complemented the antibody response in the destruction of bacteria. These proteins remain in an inactive form prior to activation in response to infection. Activation occurs by way of a pathway of proteolytic cleavage initiated by pathogen recognition and leading to pathogen destruction. Three such pathways are known in the complement system and are referred to as the classical pathway, the lectin pathway, and the alternative pathway. The classical pathway is activated when an IgG or IgM molecule binds to the surface of a pathogen. The lectin pathway is initiated by the mannan-binding lectin protein recognizing the sugar residues of a bacterial cell wall. The alternative pathway remains active at low levels in the absence of any specific stimuli. While all three pathways differ with regard to initiating events, all three pathways converge with the cleavage of complement component C3. C3 is cleaved into two products termed C3a and C3b. Of these, C3b becomes covalently linked to the pathogen surface while C3a acts as a diffusible signal to promote inflammation and recruit circulating immune cells. Surface-associated C3b forms a complex with other components to initiate a cascade of reactions among the later components of the complement system. Due to the requirement for surface attachment, complement activity remains localized and minimizes destruction to non-target cells.

Pathogen-associated C3b facilitates pathogen destruction in two ways. In one pathway, C3b is recognized directly by phagocytic cells and leads to engulfment of the pathogen. In the second pathway, pathogen-associated C3b initiates the formation of the membrane attack complex (MAC). In the first step, C3b complexes with other complement components to form the C5-convertase complex. Depending on the initial complement activation pathway, the components of this complex may differ. C5-convertase formed as the result of the classical complement pathway comprises C4b and C2a in addition to C3b. When formed by the alternative pathway, C5-convertase comprises two subunits of C3b as well as one Bb component.

Complement component C5 is cleaved by either C5-convertase complex into C5a and C5b. C5a, much like C3a, diffuses into the circulation and promotes inflammation, acting as a chemoattractant for inflammatory cells. C5b remains attached to the cell surface where it triggers the formation of the MAC through interactions with C6, C7, C8 and C9. The MAC is a hydrophilic pore that spans the membrane and promotes the free flow of fluid into and out of the cell, thereby destroying it.

An important component of all immune activity is the ability of the immune system to distinguish between self and non-self cells. Pathology arises when the immune system is unable to make this distinction. In the case of the complement system, vertebrate cells express proteins that protect them from the effects of the complement cascade. This ensures that targets of the complement system are limited to pathogenic cells. Many complement-related disorders and diseases are associated with abnormal destruction of self cells by the complement cascade. In one example, subjects suffering from paroxysmal nocturnal hemoglobinuria (PNH) are unable to synthesize functional versions of the complement regulatory proteins CD55 and CD59 on hematopoietic stem cells. This results in complement-mediated hemolysis and a variety of downstream complications. Experimental evidence suggests that PNH and other complement-related disorders are alleviated through inhibition of complement activity.

While some therapeutic complement inhibitors are available to treat patients, most require frequent administrations by intravenous or subcutaneous delivery. These frequent, often daily, administrations reduce quality of life and patient compliance. There remains a need in the art for therapeutic formulations with less burdensome administration requirements. Embodiments of the present disclosure address this by providing related formulations and methods described herein.

### SUMMARY

In some embodiments, the present disclosure provides a sustained release formulation including: a polypeptide, wherein the polypeptide includes a C5 inhibitory cyclic polypeptide including the amino acid sequence of SEQ ID NO: 1; and a release modulating matrix including a polymer, wherein the polymer includes poly lactic-co-glycolic acid (PLGA). The release modulating matrix may include a particle. The particle may include a microparticle. The PLGA may include a ratio of poly lactic acid to poly glycolic acid of from about 25:75 to about 75:25. The PLGA may include a ratio of poly lactic acid to poly glycolic acid of about 50:50. The release modulating matrix may include an emulsion. The emulsion may include a single emulsion. The emulsion may include a double emulsion. The sustained release formulation may include an excipient. The excipient may include a pore forming excipient (PFE). The pore forming excipient may be selected from the group consisting of medium chain triglycerides, PLURONIC^{®} F-127, and poly (ethylene glycol) (PEG). The particle may include a diameter of from about 5 µm to about 200 µm. The polypeptide may include a cyclic bond. The polypeptide may include a modified lysine residue. The polypeptide may include a lipid moiety. The polypeptide may include a PEG moiety. The polypeptide may include zilucoplan. The sustained release formulation may include from about 10% to about 95% of the release modulating matrix by percent weight. The sustained release formulation may include from about 50% to about 90% of the release modulating matrix by percent weight. The sustained release formulation may include from about 10% to about 50% of the polypeptide by percent weight. The polypeptide may be uniformly distributed in the release modulating matrix.

In some embodiments, the present disclosure provides a method of preparing a sustained release formulation, the method including: preparing an organic phase solution, wherein the organic phase solution is prepared by combining an organic solvent, PLGA, and the polypeptide; preparing an aqueous phase solution, wherein the aqueous phase solution is prepared by combining an aqueous solution with an emulsion stabilizer; and preparing the sustained release formulation by preparing an emulsion of the organic phase solution and the aqueous phase solution. The organic solvent may include dichloromethane (DCM). The aqueous solution may include phosphate buffered saline. The emulsion stabilizer may include polyvinyl alcohol (PVA). The organic solvent may be removed from the emulsion by evaporation. The evaporation may be carried out by quench evaporation. The evaporation may be carried out by rotary evaporation. The particle may be formed as a result of the evaporation. The polypeptide may be uniformly distributed in the particle. The polypeptide may be combined in the organic phase solution at a concentration sufficient to yield from about 10% by weight to about 50% by weight of the polypeptide in the sustained release formulation. The polypeptide may be combined in the organic phase solution at a concentration sufficient to yield from about 36% by weight to about 38% by weight of the polypeptide in the sustained release formulation. The polypeptide may include zilucoplan. The sustained release formulation may include a zilucoplan-PLGA particle.

In some embodiments, the present disclosure provides a sustained release formulation prepared according to any of the methods described herein. The sustained release formulation may include a sustained release profile including low initial burst for release of a polypeptide and/or capability of achieving an effective concentration of a polypeptide in a medium where the polypeptide is released from the sustained release formulation. The capability of achieving the effective concentration of the polypeptide in the medium where the polypeptide is released from the sustained release formulation may be extended over a specific period of time. The specific period of time may include from about one week to about three weeks. The effective concentration may be from about 4,000 ng/mL to about 12,000 ng/mL. The sustained release profile may include an initial burst for release of the polypeptide of from about 0% to about 20% of the total amount of the polypeptide included in the sustained release formulation.

In some embodiments, the present disclosure provides a method of administering a polypeptide, the method including administering a sustained release formulation described herein, wherein the polypeptide includes zilucoplan. The sustained release formulation may be administered by subcutaneous injection. The sustained release formulation may be administered weekly or biweekly. The polypeptide may be released from the sustained release formulation after administration. Release of the polypeptide may exhibit a burst of less than 5%. The sustained release formulation may be administered at a dose sufficient to administer from about 2 mg/kg to about 20 mg/kg of the polypeptide. The sustained release formulation may be administered at a dose sufficient to administer from about 100 mg to about 200 mg of zilucoplan.

In some embodiments, the present disclosure provides a method of reducing hemolysis in a subject, the method including administering a sustained release formulation described herein, wherein the sustained release formulation includes zilucoplan.

In some embodiments, the present disclosure provides a method of treating a complement-related indication, the method including administering a sustained release formulation described herein, wherein the sustained release formulation includes zilucoplan. The complement-related indication may include one or more of paroxysmal nocturnal hemoglobinuria, myasthenia gravis, an inflammatory indication, a wound, a burn, an autoimmune indication, a pulmonary indication, a cardiovascular indication, a neurological indication, a kidney-related indication, a diabetes-related indication, an ocular indication, and a pregnancy-related indication.

In some embodiments, the present disclosure provides a sustained release formulation including: an active ingredient; and a release modulating matrix including a polymer, wherein the polymer includes PLGA. The release modulating matrix may include a particle. The particle may include a nanoparticle or a microparticle. The PLGA may include a ratio of poly lactic acid to poly glycolic acid of from about 25:75 to about 75:25. The PLGA may include a ratio of poly lactic acid to poly glycolic acid of about 50:50. The release modulating matrix may include an emulsion. The emulsion may include a single emulsion. The emulsion may include a double emulsion. The sustained release formulation may include an excipient. The excipient may include a PFE. The PFE may be selected from the group consisting of medium chain triglycerides, PLURONIC^{®} F-127, and PEG. The particle may include a diameter of from about 5 µm to about 200 µm. The active ingredient may include a therapeutic agent. The active ingredient may include at least one non-natural amino acid. The at least one non-natural amino acid may be selected from any of those listed in Table 2 of United States Publication Number US 2011/0172126. The active ingredient may include a cyclic bond. The active ingredient may include a modified lysine residue. The active ingredient may include a lipid moiety. The active ingredient may include a PEG moiety. The active ingredient may be a peptidomimetic. The active ingredient may be a macrocyclic compound. The active ingredient may include a C5 inhibitor. The C5 inhibitor may be selected from the group consisting of any of those listed in Table 1 or any of those listed in Table 1 of United States Publication Number US 2017/0137468. The sustained release formulation may include from about 10% to about 95% of the release modulating matrix by percent weight. The sustained release formulation may include from about 50% to about 90% of the release modulating matrix by percent weight. The sustained release formulation may include from about 10% to about 50% of the active ingredient by percent weight. The active ingredient may be uniformly distributed in the release modulating matrix.

In some embodiments, the present disclosure provides a method of preparing a sustained release formulation described herein, the method comprising: preparing an organic phase solution, wherein the organic phase solution is prepared by combining an organic solvent, PLGA, and an active ingredient; preparing an aqueous phase solution, wherein the aqueous phase solution is prepared by combining an aqueous solution with an emulsion stabilizer; and preparing the sustained release formulation by preparing an emulsion of the organic phase solution and the aqueous phase solution. The organic solvent may include DCM. The aqueous solution may include phosphate buffered saline. The emulsion stabilizer may include PVA. The organic solvent may be removed from the emulsion by evaporation. The evaporation may be carried out by quench evaporation. The evaporation may be carried out by rotary evaporation. A particle may be formed as a result of the evaporation. The active ingredient may be uniformly distributed in the particle. The active ingredient may be combined in the organic phase solution at a concentration sufficient to yield from about 10% by weight to about 50% by weight of the active ingredient in the sustained release formulation. The active ingredient may be combined in the organic phase solution at a concentration sufficient to yield from about 36% by weight to about 38% by weight of the active ingredient in the sustained release formulation.

In some embodiments, the present disclosure provides a sustained release formulation prepared according any of the methods described herein. The sustained release formulation may include a sustained release profile including low initial burst for release of the active ingredient and/or capability of achieving an effective concentration of the active ingredient in a medium where the active ingredient is released from the sustained release formulation. The capability of achieving the effective concentration of the active ingredient in the medium where the active ingredient is released from the sustained release formulation may be extended over a specific period of time. The specific period of time may include from about one week to about three weeks. The effective concentration may be from about 4,000 ng/mL to about 12,000 ng/mL. The sustained release profile may include an initial burst for release of the active ingredient of from about 0% to about 20% of the total amount of the active ingredient included in the sustained release formulation.

In some embodiments, the present disclosure provides a method of administering an active ingredient, the method comprising administering a sustained release formulation according to any of those described herein. The sustained release formulation may be administered by subcutaneous injection. The sustained release formulation may be administered weekly or biweekly. The active ingredient may be released from the sustained release formulation after administration. Release of the active ingredient may exhibit a burst of less than 5%. The sustained release formulation may be administered at a dose sufficient to administer from about 2 mg/kg to about 20 mg/kg of the active ingredient. The sustained release formulation may be administered at a dose sufficient to administer from about 100 mg to about 200 mg of the active ingredient.

**In** some embodiments, the present disclosure provides a method of reducing hemolysis in a subject by administering an active ingredient to the subject according to any of the methods described herein.

In some embodiments, the present disclosure provides a method of treating a complement-related indication in a subject by administering an active ingredient to the subject according to any of the methods described herein, wherein the active ingredient comprises a C5 inhibitor. The complement-related indication may include one or more of paroxysmal nocturnal hemoglobinuria, myasthenia gravis, an inflammatory indication, a wound, a burn, an autoimmune indication, a pulmonary indication, a cardiovascular indication, a neurological indication, a kidney-related indication, a diabetes-related indication, an ocular indication, and a pregnancy-related indication.

### BRIEF DESCRIPTION OF THE FIGURES

The foregoing and other objects, features and advantages of particular embodiments of the disclosure will be apparent from the following description and illustrations in the accompanying figures.
Fig. 1 is a graph of zilucoplan concentration in rat plasma samples obtained at various time points after administration of zilucoplan-PLGA formulations.
Fig. 2 is a graph of zilucoplan concentration in non-human primate plasma samples obtained at various time points after administration of zilucoplan-PLGA formulations.
Fig. 3 is a graph of zilucoplan concentration in non-human primate plasma samples obtained at various time points after administration of zilucoplan-PLGA formulations.

### DETAILED DESCRIPTION

### Introduction

Embodiments of the present disclosure relate to compounds and compositions for modulating complement activity and related methods of use. Complement activity protects the body from foreign pathogens but can lead to self-cell destruction with elevated activity or poor regulation. Complement modulators may be complement inhibitors, such as zilucoplan. Zilucoplan is a synthetic, macrocyclic peptide that binds complement component 5 (C5) with sub-nanomolar affinity and allosterically inhibits its cleavage into C5a and C5b upon activation of the classical, alternative, or lectin pathways (see, e.g., United States Patent Number 10,106,579, the contents of which are herein incorporated by reference in their entirety).

**In** some embodiments, the present disclosure provides formulations for sustained release of C5 inhibitory cyclic polypeptides (e.g., zilucoplan). Such formulations may include poly (D,L-lactic-co-glycolic acid) (PLGA) technology. PLGA sustained release formulations may be used to deliver zilucoplan at desired levels over extended periods of time. PLGA may be combined with zilucoplan to produce particles comprising zilucoplan and PLGA (zilucoplan-PLGA particles). Such particles may include homogenous zilucoplan distribution, which may provide predictable sustained release profiles. Such particles may be used to deliver zilucoplan over an extended period of time and at levels sufficient to treat complement-related indications [e.g., paroxysmal nocturnal hemoglobinuria (PNH) and generalized myasthenia gravis (gMG)]. Zilucoplan-PLGA particles may be used to administer low zilucoplan drug volumes without the need for intravenous loading, on-body infusion devices, tissue-degrading enzymes, or permeation enhancers.

### I. Compounds and compositions

**In** some embodiments, the present disclosure provides compounds (e.g., cyclic polypeptides, zilucoplan, and/or active metabolites or variants thereof) and compositions thereof which function to modulate complement activity. Such compounds and compositions may include inhibitors that block complement activation. As used herein, "complement activity" includes the activation of the complement cascade, the formation of cleavage products from a complement component such as C3 or C5, the assembly of downstream complexes following a cleavage event, or any process or event attendant to, or resulting from, the cleavage of a complement component, e.g., C3 or C5. Complement inhibitors (e.g., complement inhibitory cyclic polypeptides, zilucoplan, and/or active metabolites or variants thereof) may include C5 inhibitors that block complement activation at the level of complement component C5. Such C5 inhibitors (e.g., zilucoplan and/or active metabolites or variants thereof) may bind C5 and prevent its cleavage, by C5 convertase, into the cleavage products C5a and C5b. As used herein, "Complement component C5" or "C5" is defined as a complex which is cleaved by C5 convertase into at least the cleavage products, C5a and C5b. "C5 inhibitors," as referred to herein, include any compound (e.g., cyclic polypeptides, zilucoplan, and/or active metabolites or variants thereof) or composition thereof that inhibit the processing or cleavage of the pre-cleaved complement component C5 complex or the cleavage products of the complement component C5.

In some embodiments, the present disclosure provides cyclic polypeptide compounds (e.g., zilucoplan and/or active metabolites or variants thereof) and compositions thereof which function to modulate complement activity. Such cyclic polypeptide compounds and compositions thereof may include inhibitors that block complement activation. Complement inhibitory cyclic polypeptides (e.g., zilucoplan and/or active metabolites or variants thereof) may include C5 inhibitors that block complement activation at the level of complement component C5. Such C5 inhibitors (e.g., zilucoplan and/or active metabolites or variants thereof) may bind C5 and prevent its cleavage, by C5 convertase, into the cleavage products C5a and C5b. "C5 inhibitory cyclic polypeptides," as referred to herein, include any cyclic polypeptides (e.g., zilucoplan and/or active metabolites or variants thereof) or composition thereof that inhibit the processing or cleavage of the pre-cleaved complement component C5 complex or the cleavage products of the complement component C5.

It is understood that inhibition of C5 cleavage prevents the assembly and activity of the cytolytic membrane attack complex (MAC) on glycosylphosphatidylinositol (GPI) adherent protein-deficient erythrocytes. In some cases, C5 inhibitors presented herein (e.g., C5 inhibitory cyclic polypeptides, zilucoplan, and/or active metabolites or variants thereof) may also bind C5b, preventing C6 binding and subsequent assembly of the C5b-9 MAC.

In some embodiments, C5 inhibitor polypeptides of the present disclosure may include zilucoplan. The core amino acid sequence of zilucoplan ([cyclo(1,6)]Ac-K-V-E-R-F-D-(N-Me)D-Tbg-Y-azaTrp-E-Y-P-Chg-K; SEQ ID NO: 1) includes 15 amino acids (all L-amino acids), including 4 non-natural amino acids [N-methyl-aspartic acid or "(N-Me)D", tert-butylglycine or "Tbg", 7-azatryptophan or "azaTrp", and cyclohexylglycine or "Chg"]; a lactam bridge between K1 and D6 of the polypeptide sequence; and a C-terminal lysine reside with a modified side chain, forming a N-**ε**-(PEG24-γ-glutamic acid-N-α-hexadecanoyl)lysine residue (also referred to herein as "B28"). The C-terminal lysine side chain modification includes a polyethyleneglycol (PEG) spacer (PEG24), with the PEG24 being attached to an L-γ glutamic acid residue that is derivatized with a palmitoyl group.

The free acid form of zilucoplan has a molecular formula of C₁₇₂H₂₇₈N₂₄O₅₅, a molecular weight of 3562.23 Daltons (Da), and an exact mass of 3559.97 amu. The tetra sodium form of zilucoplan has a molecular formula of C₁₇₂H₂₇₈N₂₄O₅₅Na₄. The chemical structure of sodium salt form of zilucoplan is shown in structure I:

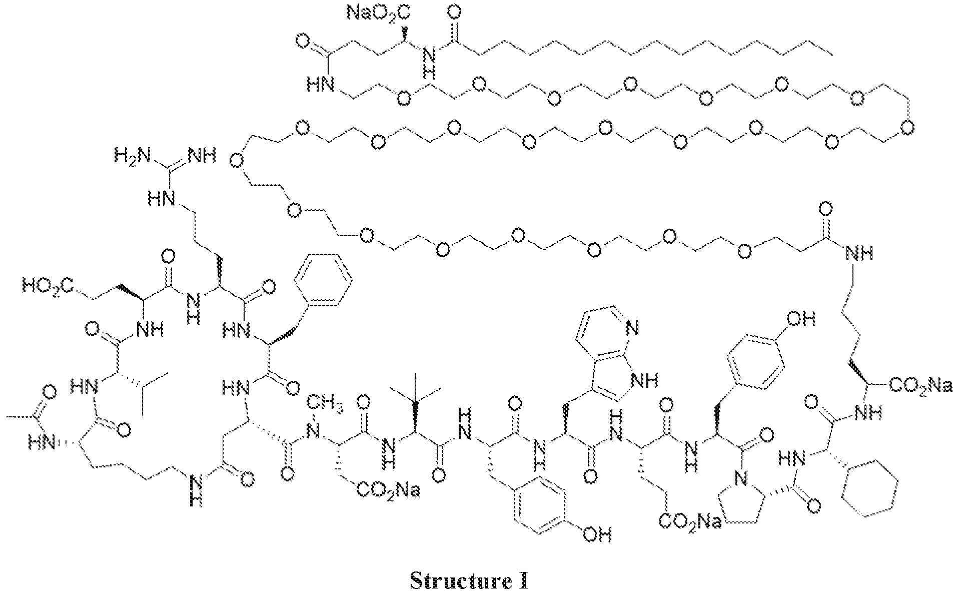

The four sodium ions in the structure are shown associated with designated carboxylates, but they may be associated with any of the acidic groups in the molecule. The zilucoplan drug substance is typically provided as the sodium salt form and is lyophilized.

**In** some embodiments, the present disclosure includes variants of zilucoplan. In some zilucoplan variants, the C-terminal lysine side chain moiety may be altered. In some cases, the PEG24 spacer (having 24 PEG subunits) of the C-terminal lysine side chain moiety may include fewer or additional PEG subunits. In other cases, the palmitoyl group of the C-terminal lysine side chain moiety may be substituted with another saturated or unsaturated fatty acid. In further cases, the L-γ glutamic acid linker of the C-terminal lysine side chain moiety (between PEG and acyl groups) may be substituted with an alternative amino acid or non-amino acid linker.

In some embodiments, C5 inhibitors may include active metabolites or variants of zilucoplan. Metabolites may include ω-hydroxylation of the palmitoyl tail. Such variants may be synthesized or may be formed by hydroxylation of a zilucoplan precursor.

In some embodiments, zilucoplan variants may include modifications to the core polypeptide sequence in zilucoplan that may be used in combination with one or more of the cyclic or C-terminal lysine side chain moiety features of zilucoplan. Such variants may have at least 50%, at least 55%, at least 65%, at least 70%, at least 80%, at least 85%, at least 90%, or at least 95% sequence identity to the core polypeptide sequence of (SEQ ID NO: 1).

In some cases, zilucoplan variants may be cyclized by forming lactam bridges between amino acids other than those used in zilucoplan.

C5 inhibitor compounds may include, but are not limited to, any of those presented in Table 1. References listed and information supporting listed clinical study numbers are incorporated herein by reference in their entirety.

**Table 1. C5 inhibitors**

| **Compound** | **Company** | **Target** | **Compound type** | **Clinical study numbers** | **References** |
|---|---|---|---|---|---|
| Eculizumab (SOLIRIS^{®}) | Alexion Pharmaceuticals, Inc. | C5 | Monoclonal antibody directed against C5 protein. Inhibits C5 cleavage. | NCT01303952; NCT02093533; NCT01567085; NCT01919346; NCT01895127; NCT01399593; NCT02145182; NCT01106027; NCT02301624; NCT01997229; NCT01892345 | US Patent No. 6,355,245; 9,732,149; 9,718,880 |
| ALXN1210 | Alexion Pharmaceuticals, Inc. | C5 | Antibody | NCT02598583; NCT02605993; NCT02946463; NCT03056040; NCT02949128 | US 2016/0168237 |
| Tesidolumab/L FG316 | Novartis | C5 | Antibody | NCT02878616; NCT02763644; NCT01527500; NCT02515942; NCT02534909; NCT01526889 | US 8,241,628; US 8,883,158 |
| ALN-CC5 | Alnylam | C5 | Nucleic acid | NCT02352493 | |
| Zimura | Ophthotech | C5 | Nucleic acid | NCT02397954; NCT02686658 | |
| Coversin | Akari | C5 | Protein | NCT02591862 | |
| ALYN1007 | Alexion | C5a | Antibody | NCT02245412; NCT02128269 | |
| IFX-1 | InflaRx | C5a | Antibody | NCT02246595; NCT02866825; NCT03001622 | |
| MUBODINA^{®} | Adienne Pharma | C5 | Antibody | | US 7,999,081 |
| ALXN5500 | Alexion Pharmaceuticals, Inc. | C5 | Antibody | | |
| ISU305 | ISU ABXIS | C5 | Antibody | | |
| Long-acting coversin | Akari | C5 | Protein | | |
| SOBI005 | Swedish Orphan Biovitrum Ab | C5 | Protein | | |
| IFX-2, TFX-3 | InflaRx | C5a | Antibody | | |
| NOX-D21 | Noxxon | C5a | Spiegelmer | | |
| rEV576 | Volution Immunopharmaceu ticals | C5 | Antibody | | Penabad et al., Lupus, 2014 23(12):1324-6 |
| ARC1005 | Novo Nordisk | C5 | Antibody | | |
| SOMAmers | SomaLogic | C5 | Antibody | | |

### Peptide-based compounds

In some embodiments, C5 inhibitors of the present disclosure are polypeptides. According to the present disclosure, any amino acid-based molecule (natural or non-natural) may be termed a "polypeptide" and this term embraces "peptides," "peptidomimetics," and "proteins." "Peptides" are traditionally considered to range in size from about 4 to about 50 amino acids. Polypeptides larger than about 50 amino acids are generally termed "proteins."

C5 inhibitor polypeptides may be linear or cyclic. Cyclic polypeptides (e.g., zilucoplan and/or active metabolites or variants thereof) include any polypeptides that have as part of their structure one or more cyclic features such as a loop and/or an internal linkage. In some embodiments, such cyclic polypeptides are formed when a molecule acts as a bridging moiety to link two or more regions of the polypeptide. As used herein, the term "bridging moiety" refers to one or more components of a bridge formed between two adjacent or non-adjacent amino acids, non-natural amino acids or non-amino acids in a polypeptide. Bridging moieties may be of any size or composition. In some embodiments, bridging moieties may include one or more chemical bonds between two adjacent or non-adjacent amino acids, non-natural amino acids, non-amino acid residues or combinations thereof. In some embodiments, such chemical bonds may be between one or more functional groups on adjacent or non-adjacent amino acids, non-natural amino acids, non-amino acid residues or combinations thereof. Bridging moieties may include one or more of an amide bond (lactam), disulfide bond, thioether bond, aromatic ring, triazole ring, and hydrocarbon chain. In some embodiments, bridging moieties include an amide bond between an amine functionality and a carboxylate functionality, each present in an amino acid, non-natural amino acid or non-amino acid residue side chain. In some embodiments, the amine or carboxylate functionalities are part of a non-amino acid residue or non-natural amino acid residue.

C5 inhibitor polypeptides (e.g., C5 inhibitory cyclic polypeptides, zilucoplan, and/or active metabolites or variants thereof) may be cyclized through the carboxy terminus, the amino terminus, or through any other convenient point of attachment, such as, for example, through the sulfur of a cysteine (e.g., through the formation of disulfide bonds between two cysteine residues in a sequence) or any side-chain of an amino acid residue. Further linkages forming cyclic loops may include, but are not limited to, maleimide linkages, amide linkages, ester linkages, ether linkages, thiol ether linkages, hydrazone linkages, or acetamide linkages.

In some embodiments, C5 inhibitor polypeptides (e.g., C5 inhibitory cyclic polypeptides, zilucoplan, and/or active metabolites or variants thereof) may be synthesized on solid supports (e.g., rink amide resin) via solid phase peptide synthesis (SPPS). SPPS methods are known in the art and may be performed with orthogonal protecting groups. In some embodiments, peptides of the present disclosure may be synthesized via SPPS with Fmoc chemistry and/or Boc chemistry. Synthesized peptides may be cleaved from solid supports using standard techniques.

C5 inhibitor polypeptides (e.g., C5 inhibitory cyclic polypeptides, zilucoplan, and/or active metabolites or variants thereof) may be purified via chromatography [e.g., size exclusion chromatography (SEC) and/or high performance liquid chromatography (HPLC)]. HPLC may include reverse phase HPLC (RP-HPLC). Such peptides may be freeze-dried after purification. Purified peptides may be obtained as pure peptide or as a peptide salt. Residual salts making up peptide salts may include, but are not limited to, trifluoroacetic acid (TFA), acetate, and/or hydrochloride. In some embodiments, peptides of the present disclosure are obtained as peptide salts. The peptide salts may be peptide salts with TFA. Residual salts may be removed from purified peptides according to known methods (e.g., through use of desalting columns).

In some embodiments, C5 inhibitory cyclic polypeptides of the present disclosure (e.g., zilucoplan and/or active metabolites or variants thereof) are formed using a lactam moiety. Such cyclic polypeptides may be formed, for example, by synthesis on a solid support Wang resin using standard Fmoc chemistry. In some cases, Fmoc-ASP(allyl)-OH and Fmoc-LYS(alloc)-OH are incorporated into polypeptides to serve as precursor monomers for lactam bridge formation.

C5 inhibitor polypeptides (e.g., C5 inhibitory cyclic polypeptides, zilucoplan, and/or active metabolites or variants thereof) may be peptidomimetics. A "peptidomimetic" or "polypeptide mimetic" is a polypeptide in which the molecule contains structural elements that are not found in natural polypeptides *(i.e.,* polypeptides comprised of only the 20 proteinogenic amino acids). In some embodiments, peptidomimetics are capable of recapitulating or mimicking the biological action(s) of a natural peptide. A peptidomimetic may differ in many ways from natural polypeptides, for example through changes in backbone structure or through the presence of amino acids that do not occur in nature. In some cases, peptidomimetics may include amino acids with side chains that are not found among the known 20 proteinogenic amino acids; non-polypeptide-based bridging moieties used to effect cyclization between the ends or internal portions of the molecule; substitutions of the amide bond hydrogen moiety by methyl groups (N-methylation) or other alkyl groups; replacement of a peptide bond with a chemical group or bond that is resistant to chemical or enzymatic treatments; N- and C-terminal modifications; and/or conjugation with a non-peptidic extension (such as polyethylene glycol, lipids, carbohydrates, nucleosides, nucleotides, nucleoside bases, various small molecules, or phosphate or sulfate groups).

As used herein, the term "amino acid" includes the residues of the natural amino acids as well as non-natural amino acids. The 20 natural proteinogenic amino acids are identified and referred to herein by either the one-letter or three-letter designations as follows: aspartic acid (Asp:D), isoleucine (Ile:I), threonine (Thr:T), leucine (Leu:L), serine (Ser:S), tyrosine (Tyr:Y), glutamic acid (Glu:E), phenylalanine (Phe:F), proline (Pro:P), histidine (His:H), glycine (Gly:G), lysine (Lys:K), alanine (Ala:A), arginine (Arg:R), cysteine (Cys:C), tryptophan (Trp:W), valine (Val:V), glutamine (Gln:Q) methionine (Met:M), asparagine (Asn:N). Naturally occurring amino acids exist in their levorotary (L) stereoisomeric forms. Amino acids referred to herein are L-stereoisomers except where otherwise indicated. The term "amino acid" also includes amino acids bearing a conventional amino protecting group (e.g. acetyl or benzyloxycarbonyl), as well as natural and non-natural amino acids protected at the carboxy terminus (e.g., as a (C1-C6) alkyl, phenyl or benzyl ester or amide; or as an alpha-methylbenzyl amide). Other suitable amino and carboxy protecting groups are known to those skilled in the art (See for example, Greene, T. W.; Wutz, P. G. M., Protecting Groups In Organic Synthesis; second edition, 1991, New York, John Wiley & sons, Inc., and documents cited therein, the contents of each of which are herein incorporated by reference in their entirety). Polypeptides and/or polypeptide compositions of the present disclosure may also include modified amino acids.

"Non-natural" amino acids have side chains or other features not present in the 20 naturally-occurring amino acids listed above and include, but are not limited to: N-methyl amino acids, N-alkyl amino acids, alpha, alpha substituted amino acids, beta-amino acids, alpha-hydroxy amino acids, D-amino acids, and other non-natural amino acids known in the art (See, e.g., Josephson et al., (2005) J. Am. Chem. Soc. 127: 11727-11735; Forster, A.C. et al. (2003) Proc. Natl. Acad. Sci. USA 100: 6353-6357; Subtelny et al., (2008) J. Am. Chem. Soc. 130: 6131-6136; Hartman, M.C.T. et al. (2007) PLoS ONE 2:e972; and Hartman et al., (2006) Proc. Natl. Acad. Sci. USA 103:4356-4361). Further non-natural amino acids useful for the optimization of polypeptides and/or polypeptide compositions of the present disclosure include, but are not limited to 1,2,3,4-tetrahydroisoquinoline-1-carboxylic acid, 1-amino-2,3-hydro-1H-indene-1-carboxylic acid, homolysine, homoarginine, homoserine, 2-aminoadipic acid, 3-aminoadipic acid, beta-alanine, aminopropionic acid, 2-aminobutyric acid, 4-aminobutyric acid, 5-aminopentanoic acid, 5-aminohexanoic acid, 6-aminocaproic acid, 2-aminoheptanoic acid, 2-aminoisobutyric acid, 3-aminoisobutyric acid, 2-aminopimelic acid, desmosine, 2,3-diaminopropionic acid, N-ethylglycine, N-ethyl asparagine, homoproline, hydroxylysine, allo-hydroxylysine, 3-hydroxyproline, 4-hydroxyproline, isodesmosine, allo-isoleucine, N-methylpentylglycine, naphthylalanine, ornithine, pentylglycine, thioproline, norvaline, tert-butylglycine, phenylglycine, azatryptophan, 5-azatryptophan, 7-azatryptophan, 4-fluorophenylalanine, penicillamine, sarcosine, homocysteine, 1-aminocyclopropanecarboxylic acid, 1-aminocyclobutanecarboxylic acid, 1-aminocyclopentanecarboxylic acid, 1-aminocyclohexanecarboxylic acid, 4-aminotetrahydro-2*H*-pyran-4-carboxylic acid, (*S*)-2-amino-3-(1*H*-tetrazol-5-yl)propanoic acid, cyclopentylglycine, cyclohexylglycine, cyclopropylglycine, η-ω-methyl-arginine, 4-chlorophenylalanine, 3-chlorotyrosine, 3-fluorotyrosine, 5-fluorotryptophan, 5-chlorotryptophan, citrulline, 4-chloro-homophenylalanine, homophenylalanine, 4-aminomethyl-phenylalanine, 3-aminomethyl-phenylalanine, octylglycine, norleucine, tranexamic acid, 2-amino pentanoic acid, 2-amino hexanoic acid, 2-amino heptanoic acid, 2-amino octanoic acid, 2-amino nonanoic acid, 2-amino decanoic acid, 2-amino undecanoic acid, 2-amino dodecanoic acid, aminovaleric acid, and 2-(2-aminoethoxy)acetic acid, pipecolic acid, 2-carboxy azetidine, hexafluoroleucine, 3-Fluorovaline, 2-amino-4,4-difluoro-3-methylbutanoic acid, 3-fluoro-isoleucine, 4-fluoroisoleucine, 5-fluoroisoleucine, 4-methyl-phenylglycine, 4-ethyl-phenylglycine, 4-isopropyl-phenylglycine, (S)-2-amino-5-azidopentanoic acid (also referred to herein as "X02"), (S)-2-aminohept-6-enoic acid (also referred to herein as "X30''), (S)-2-aminopent-4-ynoic acid (also referred to herein as "X31"), (S)-2-aminopent-4-enoic acid (also referred to herein as "X12"), (*S*)-2-amino-5-(3-methylguanidino) pentanoic acid, (*S*)-2-amino-3-(4-(aminomethyl)phenyl)propanoic acid, (*S*)-2-amino-3-(3-(aminomethyl)phenyl)propanoic acid, (*S*)-2-amino-4-(2-aminobenzo[d]oxazol-5-yl)butanoic acid, (S)-leucinol, (S)-valinol, (*S*)-tert-leucinol, (*R*)-3-methylbutan-2-amine, (*S*)-2-methyl-1-phenylpropan-1-amine, and (*S*)-*N*,2-dimethyl-1-(pyridin-2-yl)propan-1-amine, (*S*)-2-amino-3-(oxazol-2-yl)propanoic acid, (*S*)-2-amino-3-(oxazol-5-yl)propanoic acid, (*S*)-2-amino-3-(1,3 ,4-oxadiazol-2-yl)propanoic acid, (*S*)-2-amino-3-(1,2,4-oxadiazol-3-yl)propanoic acid, (*S*)-2-amino-3-(5-fluoro-1*H-*indazol-3-yl)propanoic acid, and (*S*)-2-amino-3-(1*H*-indazol-3-yl)propanoic acid, (*S*)-2-amino-3-(oxazol-2-yl)butanoic acid, (*S*)-2-amino-3-(oxazol-5-yl) butanoic acid, (*S*)-2-amino-3-(1,3,4-oxadiazol-2-yl) butanoic acid, (*S*)-2-amino-3-(1,2,4-oxadiazol-3-yl) butanoic acid, (*S*)-2-amino-3-(5-fluoro-1*H*-indazol-3-yl) butanoic acid, and (*S*)-2-amino-3-(1*H*-indazol-3-yl) butanoic acid, 2-(2'MeOphenyl)-2-amino acetic acid, tetrahydro 3-isoquinolinecarboxylic acid and stereoisomers thereof (including, but not limited, to D and L isomers).

Additional non-natural amino acids that are useful in the optimization of polypeptides or polypeptide compositions of the present disclosure include but are not limited to fluorinated amino acids wherein one or more carbon bound hydrogen atoms are replaced by fluorine. The number of fluorine atoms included can range from 1 up to and including all of the hydrogen atoms. Examples of such amino acids include but are not limited to 3-fluoroproline, 3,3-difluoroproline, 4-fluoroproline, 4,4-difluoroproline, 3,4-difluroproline, 3,3,4,4-tetrafluoroproline, 4-fluorotryptophan, 5-flurotryptophan, 6-fluorotryptophan, 7-fluorotryptophan, and stereoisomers thereof.

Further non-natural amino acids that are useful in the optimization of polypeptides of the present disclosure include but are not limited to those that are disubstituted at the α-carbon. These include amino acids in which the two substituents on the α-carbon are the same, for example α-amino isobutyric acid, and 2-amino-2-ethyl butanoic acid, as well as those where the substituents are different, for example α-methylphenylglycine and α-methylproline. Further the substituents on the α-carbon may be taken together to form a ring, for example 1-aminocyclopentanecarboxylic acid, 1- aminocyclobutanecarboxylic acid, 1-aminocyclohexanecarboxylic acid, 3-aminotetrahydrofuran-3-carboxylic acid, 3-aminotetrahydropyran-3-carboxylic acid, 4-aminotetrahydropyran-4-carboxylic acid, 3-aminopyrrolidine-3-carboxylic acid, 3-aminopiperidine-3-carboxylic acid, 4-aminopiperidinnne-4-carboxylix acid, and stereoisomers thereof.

Additional non-natural amino acids that are useful in the optimization of polypeptides or polypeptide compositions of the present disclosure include but are not limited to analogs of tryptophan in which the indole ring system is replaced by another 9 or 10 membered bicyclic ring system with 0, 1, 2, 3 or 4 heteroatoms independently selected from N, O, or S. Each ring system may be saturated, partially unsaturated, or fully unsaturated. The ring system may be substituted by 0, 1, 2, 3, or 4 substituents at any substitutable atom. Each substituent may be independently selected from H, F, Cl, Br, CN, COOR, CONRR', oxo, OR, NRR'. Each R and R' may be independently selected from H, C1-C20 alkyl, or C1-C20 alkyl-O-C1-20 alkyl.

In some embodiments, analogs of tryptophan (also referred to herein as "tryptophan analogs") may be useful in the optimization of polypeptides or polypeptide compositions of the present disclosure. Tryptophan analogs may include, but are not limited to, 5-fluorotryptophan [(5-F)W], 5-methyl-O-tryptophan [(5-MeO)W], 1-methyltryptophan [(1-Me-W) or (1-Me)W], D-tryptophan (D-Trp), azatryptophan (including, but not limited to 4-azatryptophan, 7-azatryptophan and 5-azatryptophan,) 5-chlorotryptophan, 4-fluorotryptophan, 6-fluorotryptophan, 7-fluorotryptophan, and stereoisomers thereof. Except where indicated to the contrary, the term "azatryptophan" and its abbreviation, "azaTrp," as used herein, refer to 7-azatryptophan.

Modified amino acid residues useful for the optimization of polypeptides and/or polypeptide compositions of the present disclosure include, but are not limited to those which are chemically blocked (reversibly or irreversibly); chemically modified on their N-terminal amino group or their side chain groups; chemically modified in the amide backbone, as for example, N-methylated, D (non-natural amino acids) and L (natural amino acids) stereoisomers; or residues wherein the side chain functional groups are chemically modified to another functional group. In some embodiments, modified amino acids include without limitation, methionine sulfoxide; methionine sulfone; aspartic acid-(beta-methyl ester), a modified amino acid of aspartic acid; N-ethylglycine, a modified amino acid of glycine; alanine carboxamide; and/or a modified amino acid of alanine. Non-natural amino acids may be purchased from Sigma-Aldrich (St. Louis, MO), Bachem (Torrance, CA) or other suppliers. Non-natural amino acids may further include any of those listed in Table 2 of US patent publication US 2011/0172126, the contents of which are incorporated herein by reference in their entirety.

The present disclosure contemplates variants and derivatives of polypeptides presented herein (e.g., C5 inhibitor polypeptides, C5 inhibitory cyclic polypeptides, zilucoplan, and/or active metabolites or variants thereof). These include substitutional, insertional, deletional, and covalent variants and derivatives. As used herein, the term "derivative" is used synonymously with the term "variant" and refers to a molecule that has been modified or changed in any way relative to a reference molecule or starting molecule.

Polypeptides of the present disclosure may include any of the following components, features, or moieties, for which abbreviations used herein include: "Ac" and "NH2" indicate acetyl and amidated termini, respectively; "Nvl" stands for norvaline; "Phg" stands for phenylglycine; "Tbg" stands for tert-butylglycine; "Chg" stands for cyclohexylglycine; "(N-Me)X" stands for the N-methylated form of the amino acid indicated by the letter or three letter amino acid code in place of variable "X" written as N-methyl-X [e.g. (N-Me)D or (N-Me)Asp stand for the N-methylated form of aspartic acid or N-methyl-aspartic acid]; "azaTrp" stands for azatryptophan; "(4-F)Phe" stands for 4-fluorophenylalanine; "Tyr(OMe)" stands for O-methyl tyrosine, "Aib" stands for amino isobutyric acid; "(homo)F" or "(homo)Phe" stands for homophenylalanine; "(2-OMe)Phg" refers to 2-O-methylphenylglycine; "(5-F)W" refers to 5-fluorotryptophan; "D-X" refers to the D-stereoisomer of the given amino acid "X" [e.g. (D-Chg) stands for D-cyclohexylglycine]; "(5-MeO)W" refers to 5-methyl-O-tryptophan; "homoC" refers to homocysteine; "(1-Me-W)" or "(1-Me)W" refers to 1-methyltryptophan; "Nle" refers to norleucine; "Tiq" refers to a tetrahydroisoquinoline residue; "Asp(T)" refers to (*S*)-2-amino-3-(1*H*-tetrazol-5-yl)propanoic acid; "(3-Cl-Phe)" refers to 3-chlorophenylalanine; "[(N-Me-4-F)Phe]" or "(N-Me-4-F)Phe" refers to N-methyl-4-fluorophenylalanine; "(m-(Cl-homo)Phe" refers to meta-chloro homophenylalanine; "(des-amino)C" refers to 3-thiopropionic acid; "(alpha-methyl)D" refers to alpha-methyl L-aspartic acid; "2Nal" refers to 2-naphthylalanine; "(3-aminomethyl)Phe" refers to 3-aminomethyl-L-phenyalanine; "Cle" refers to cycloleucine; "Ac-Pyran" refers to 4-amino-tetrahydro-pyran-4-carboxylic acid; "(Lys-C16)" refers to N-**ε**-palmitoyl lysine; "(Lys-C12)" refers to N-**ε**-lauryl lysine; "(Lys-C10)" refers to N-**ε**-capryl lysine; "(Lys-C8)" refers to N-**ε**-caprylic lysine; "[*x*Xylyl(*y*, z)]" refers to the xylyl bridging moiety between two thiol containing amino acids where x may be m, p or o to indicate the use of meta-, para- or ortho- dibromoxylenes (respectively) to generate bridging moieties and the numerical identifiers, y and z, place the amino acid position within the polypeptide of the amino acids participating in the cyclization; "[cyclo(*y,z*)]" refers to the formation of a bond between two amino acid residues where the numerical identifiers, y and z, place the position of the residues participating in the bond; "[cyclo-olefinyl(*y,z*)]" refers to the formation of a bond between two amino acid residues by olefin metathesis where the numerical identifiers, y and z, place the position of the residues participating in the bond; "[cyclo-thioalkyl(y,z)]" refers to the formation of a thioether bond between two amino acid residues where the numerical identifiers, *y* and z, place the position of the residues participating in the bond; "[cyclo-triazolyl(y,z)]" refers to the formation of a triazole ring between two amino acid residues where the numerical identifiers, y and z, place the position of the residues participating in the bond. "B20" refers to N-**ε**-(PEG2-γ-glutamic acid-N-α-octadecanedioic acid) lysine [also known as (1*S*,28*S*)-1-amino-7,16,25,30-tetraoxo-9,12,18,21-tetraoxa-6,15,24,29-tetraazahexatetracontane-1,28,46-tricarboxylic acid.]

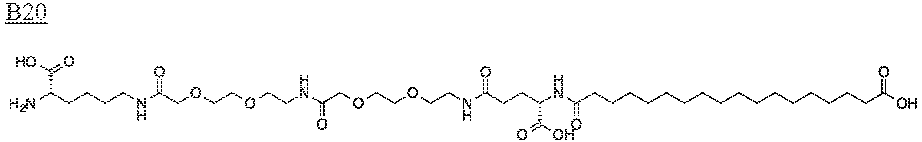

"B28" refers to N-**ε**-(PEG24-γ-glutamic acid-N-α-hexadecanoyl)lysine.

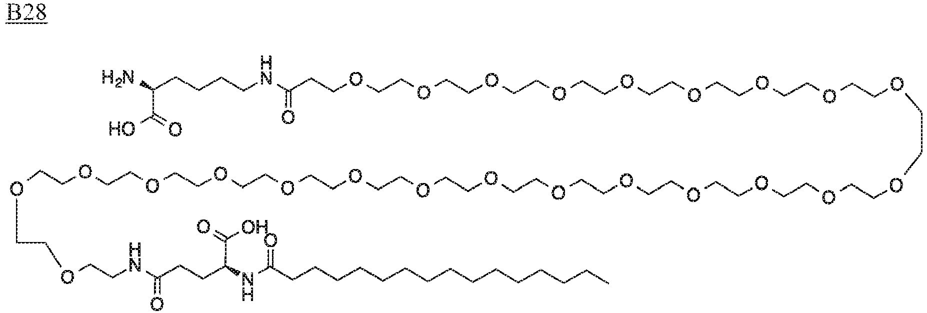

"K14" refers to N-**ε**-1-(4,4-dimethyl-2,6-dioxocyclohex-1-ylidene)-3-methylbutyl-L-lysine. All other symbols refer to the standard one-letter amino acid code.

Some C5 inhibitor polypeptides (e.g., C5 inhibitory cyclic polypeptides, zilucoplan, and/or active metabolites or variants thereof) of the present disclosure include from about 5 amino acids to about 10 amino acids, from about 6 amino acids to about 12 amino acids, from about 7 amino acids to about 14 amino acids, from about 8 amino acids to about 16 amino acids, from about 10 amino acids to about 18 amino acids, from about 12 amino acids to about 24 amino acids, or from about 15 amino acids to about 30 amino acids. In some cases, C5 inhibitor polypeptides (e.g., C5 inhibitory cyclic polypeptides) of the present disclosure include at least 30 amino acids.

Some C5 inhibitor polypeptides (e.g., C5 inhibitory cyclic polypeptides, zilucoplan, and/or active metabolites or variants thereof) include a C-terminal lipid moiety. Such lipid moieties may include fatty acyl groups (e.g., saturated or unsaturated fatty acyl groups). In some cases, the fatty acyl group may be a palmitoyl group.

C5 inhibitor polypeptides (e.g., C5 inhibitory cyclic polypeptides, zilucoplan, and/or active metabolites or variants thereof) having fatty acyl groups may include one or more molecular linkers joining the fatty acids to the peptide. Such molecular linkers may include amino acid residues. In some cases, L-γ glutamic acid residues may be used as molecular linkers. In some cases, molecular linkers may include one or more polyethylene glycol (PEG) linkers. PEG linkers of the present disclosure may include from about 1 to about 5, from about 2 to about 10, from about 4 to about 20, from about 6 to about 24, from about 8 to about 32, or at least 32 PEG units.

C5 inhibitor polypeptides (e.g., C5 inhibitory cyclic polypeptides, zilucoplan, and/or active metabolites or variants thereof) may have molecular weights of from about 200 g/mol to about 600 g/mol, from about 500 g/mol to about 2000 g/mol, from about 1000 g/mol to about 5000 g/mol, from about 3000 g/mol to about 4000 g/mol, from about 2500 g/mol to about 7500 g/mol, from about 5000 g/mol to about 10000 g/mol, or at least 10000 g/mol.

In some embodiments, C5 inhibitor polypeptides (e.g., C5 inhibitory cyclic polypeptides, zilucoplan, and/or active metabolites or variants thereof) of the present disclosure may include any of those listed in Table 1 of United States Publication Number US 2017/0137468, the contents of which are herein incorporated by reference in their entirety.

C5 inhibitor polypeptides (e.g., C5 inhibitory cyclic polypeptides, zilucoplan, and/or active metabolites or variants thereof) of the present disclosure may be developed or modified to achieve specific binding characteristics. Inhibitor binding may be assessed by determining rates of association and/or dissociation with a particular target. In some cases, *compounds* demonstrate strong and rapid association with a target combined with a slow rate of dissociation. In some embodiments, C5 inhibitor polypeptides (e.g., C5 inhibitory cyclic polypeptides, zilucoplan, and/or active metabolites or variants thereof) of the present disclosure demonstrate strong and rapid association with C5. Such inhibitors may further demonstrate slow rates of dissociation with C5.

C5 inhibitor polypeptides (e.g., C5 inhibitory cyclic polypeptides, zilucoplan, and/or active metabolites or variants thereof) disclosed herein, may bind to C5 complement protein with an equilibrium dissociation constant (K_{D}) of from about 0.001 nM to about 0.01 nM, from about 0.005 nM to about 0.05 nM, from about 0.01 nM to about 0.1 nM, from about 0.05 nM to about 0.5 nM, from about 0.1 nM to about 1.0 nM, from about 0.5 nM to about 5.0 nM, from about 2 nM to about 10 nM, from about 8 nM to about 20 nM, from about 15 nM to about 45 nM, from about 30 nM to about 60 nM, from about 40 nM to about 80 nM, from about 50 nM to about 100 nM, from about 75 nM to about 150 nM, from about 100 nM to about 500 nM, from about 200 nM to about 800 nM, from about 400 nM to about 1,000 nM or at least 1,000 nM.

In some embodiments, C5 inhibitor polypeptides (e.g., C5 inhibitory cyclic polypeptides, zilucoplan, and/or active metabolites or variants thereof) of the present disclosure block the formation or generation of C5a from C5. In some case, formation or generation of C5a is blocked following activation of the alternative pathway of complement activation. In some cases, C5 inhibitor polypeptides (e.g., C5 inhibitory cyclic polypeptides, zilucoplan, and/or active metabolites or variants thereof) of the present disclosure block the formation of the membrane attack complex (MAC). Such MAC formation inhibition may be due to C5 inhibitor binding to C5b subunits. C5 inhibitor polypeptide (e.g., C5 inhibitory cyclic polypeptide, zilucoplan, and/or an active metabolite or variant thereof) binding to C5b subunits may prevent C6 binding, resulting in blockage of MAC formation. In some embodiments, this MAC formation inhibition occurs after activation of the classical, alternative, or lectin pathways.

C5 inhibitor polypeptides (e.g., C5 inhibitory cyclic polypeptides, zilucoplan, and/or active metabolites or variants thereof) of the present disclosure may be synthesized using chemical processes. In some cases, such synthesis eliminates risks associated with the manufacture of biological products in mammalian cell lines. In some cases, chemical synthesis may be simpler and more cost-effective than biological production processes.

In some embodiments, C5 inhibitor polypeptide (e.g., C5 inhibitory cyclic polypeptide, zilucoplan, and/or an active metabolite or variant thereof) compositions may be pharmaceutical compositions that include at least one pharmaceutically acceptable excipient. In some embodiments, the pharmaceutically acceptable excipient may include at least one of a salt and a buffering agent. The salt may be sodium chloride. The buffering agent may be sodium phosphate. Sodium chloride may be present at a concentration of from about 0.1 mM to about 1000 mM. In some cases, sodium chloride may be present at a concentration of from about 25 mM to about 100 mM. Sodium phosphate may be present at a concentration of from about 0.1 mM to about 1000 mM. In some cases, sodium phosphate is present at a concentration of from about 10 mM to about 100 mM.

In some embodiments, C5 inhibitor polypeptide (e.g., C5 inhibitory cyclic polypeptide, zilucoplan, and/or an active metabolite or variant thereof) compositions may include from about 0.01 mg/mL to about 4000 mg/mL of a C5 inhibitor. In some cases, C5 inhibitor polypeptides (e.g., C5 inhibitory cyclic polypeptides, zilucoplan, and/or active metabolites or variants thereof) are present at a concentration of from about 1 mg/mL to about 400 mg/mL.

Zilucoplan binds to C5 and inhibits cleavage of C5 by canonical complement pathway convertases as described in International Publication Number WO2018106859, the contents of which are herein incorporated by reference in their entirety. Zilucoplan additionally binds C5b, preventing the formation of the membrane attack complex induced by non-canonical cleavage of C5. Zilucoplan binding and inhibitory activities are not affected by the presence of clinically relevant human C5 polymorphisms (including p.R885>H/C). Unlike eculizumab, an anti-C5 monoclonal antibody inhibitor, zilucoplan does not bind to surface-bound C5b-9 or soluble membrane attack complex (sC5b-9).

### Therapeutic agents

In some embodiments, C5 inhibitors (e.g., C5 inhibitor polypeptides, C5 inhibitory cyclic polypeptides, zilucoplan, and/or active metabolites or variants thereof) of the present disclosure are therapeutic agents. As used herein, the term "therapeutic agent" refers to any substance useful for alleviating, stabilizing, improving, curing, or otherwise addressing any symptom, condition, disorder, or disease. *Therapeutic* agents may include, but are not limited to, natural products, synthetic products, combinations of natural and synthetic products, small molecules, macromolecules, nucleic acids, aptamers, proteins, and polypeptides.

### Formulations

In some embodiments, the present disclosure provides formulations comprising C5 inhibitors (e.g., C5 inhibitor polypeptides, C5 inhibitory cyclic polypeptides, zilucoplan, and/or active metabolites or variants thereof) and/or therapeutic agents described herein. As used herein, a "formulation" is a composition prepared to include a specific component, combination of components, and/or format. Formulation components may include, but are not limited to, solid components, liquid components, and combinations thereof. Formulations of the present disclosure may be prepared to include or to deliver an active ingredient. As used herein, the term "active ingredient" refers to a substance in a composition or formulation that produces a desired effect. Active ingredients may include therapeutic agents. In some embodiments, formulations of the present disclosure are prepared for therapeutic agent delivery. Such formulations may be prepared to deliver therapeutic agents to subjects upon formulation administration.

In some embodiments, formulations of the present disclosure include controlled release formulations. As used herein, a "controlled release formulation" is a formulation that facilitates or is prepared to facilitate diffusion of one or more formulation components to surrounding environments. Some controlled release formulations control the release of therapeutic agents due to interactions between the therapeutic agents (e.g., C5 inhibitors, C5 inhibitor polypeptides, C5 inhibitory cyclic polypeptides, zilucoplan, and/or active metabolites or variants thereof) and surrounding formulation components. "Sustained release formulations" are controlled release formulations that facilitate prolonged diffusion of one or more formulation components. Sustained release formulations may slow diffusion of therapeutic agents (e.g., C5 inhibitors, C5 inhibitor polypeptides, C5 inhibitory cyclic polypeptides, zilucoplan, and/or active metabolites or variants thereof) from the formulation to surrounding environments.

In some embodiments, the present disclosure provides sustained release formulations that include an active ingredient (e.g., C5 inhibitors, C5 inhibitor polypeptides, C5 inhibitory cyclic polypeptides, zilucoplan, and/or active metabolites or variants thereof) and a release modulating matrix. As used herein, the term "release modulating matrix" refers to any network of compounds, chemical bonds, or other chemical barriers that alters the diffusion of an agent. Release modulating matrices may include polymers. Such polymers may be biodegradable. Release modulating matrices may include particles. Such particles may include nanoparticles and/or microparticles. In some embodiments, release modulating matrices may include poly lactic-co-glycolic acid (PLGA). PLGA is a polymer made up of repeating units of poly lactic acid (PLA) and poly glycolic acid (PGA) of different lengths. PLGA included may include a ratio of poly lactic acid to poly glycolic acid of from about 25:75 to about 75:25. The ratio may be about 50:50. Release modulating matrices may include emulsions. Such emulsions may include single emulsions (e.g., water-in-oil emulsions) or double emulsions (e.g., oil-in-water-in-oil emulsions).

In some embodiments, formulations of the present disclosure include controlled release formulations of C5 inhibitory cyclic polypeptides (e.g., zilucoplan and/or active metabolites or variants thereof). Some controlled release formulations control the release of C5 inhibitory cyclic polypeptides (e.g., zilucoplan and/or active metabolites or variants thereof) due to interactions between C5 inhibitory cyclic polypeptides (e.g., zilucoplan and/or active metabolites or variants thereof) and surrounding formulation components. Sustained release formulations may slow diffusion of C5 inhibitory cyclic polypeptides from the formulation to surrounding environments.

Sustained release formulations may include excipients. In some embodiments, pore forming excipients (PFEs) may be included. As used herein, the term "pore forming excipient" refers to a compound or composition used to generate bubbles or pockets within sustained release formulations or release modulating matrices included therein. PFEs may include, but are not limited to, medium chain triglycerides, PLURONIC^{®} F-127, and poly (ethylene glycol) (PEG). PEG PFEs may include, but are not limited to PEG3400.

Release modulating matrices may include particles with a range of diameters. In some embodiments, particle diameters are from about 5 µm to about 200 µm.

Active ingredients included in sustained release formulations may include therapeutic agents (e.g., C5 inhibitors, C5 inhibitor polypeptides, C5 inhibitory cyclic polypeptides, zilucoplan, and/or active metabolites or variants thereof). Such therapeutic agents may include natural and/or non-natural amino acids. Some therapeutic agents may include cyclic bonds. Some therapeutic agents may include modified lysine residues (e.g., with post-translational modifications to free amines of such lysine residues. Some therapeutic agents may include lipid moieties. Some therapeutic agents may include PEG moieties. Some therapeutic agents may include a peptidomimetics. Some therapeutic agents may include macrocyclic compounds. As used herein the term "macrocyclic compound" refers to any macromolecule that includes at least one cyclic bond. Macrocyclic *compounds* may include polypeptides or proteins, including those with natural and/or non-natural amino acids. In some embodiments, sustained release formulations include C5 inhibitor polypeptides that include SEQ ID NO: 1 (e.g., zilucoplan) as a therapeutic agent. Sustained release formulations may include from about 10% by weight to about 50% by weight of therapeutic agent. Therapeutic agents may be uniformly distributed in release modulating matrices.

Active ingredients included in sustained release formulations may include C5 inhibitory cyclic polypeptides (e.g., zilucoplan and/or active metabolites or variants thereof), which may include natural and/or non-natural amino acids. Some C5 inhibitory cyclic polypeptides (e.g., zilucoplan and/or active metabolites or variants thereof) may include modified lysine residues (e.g., with post-translational modifications to free amines of such lysine residues). Some C5 inhibitory cyclic polypeptides (e.g., zilucoplan and/or active metabolites or variants thereof) may include lipid moieties. Some C5 inhibitory cyclic polypeptides (e.g., zilucoplan and/or active metabolites or variants thereof) may include PEG moieties. Some C5 inhibitory cyclic polypeptides (e.g., zilucoplan and/or active metabolites or variants thereof) may include peptidomimetics. Some C5 inhibitory cyclic polypeptides (e.g., zilucoplan and/or active metabolites or variants thereof) may include macrocyclic compounds. In some embodiments, sustained release formulations include C5 inhibitory cyclic polypeptides that include SEQ ID NO: 1. Such polypeptides may include zilucoplan.

Therapeutic agents (e.g., C5 inhibitors, C5 inhibitor polypeptides, C5 inhibitory cyclic polypeptides, zilucoplan, and/or active metabolites or variants thereof) may be present in sustained release formulations at a level of from about 10% by weight to about 50% by weight. Therapeutic agents may be uniformly distributed in release modulating matrices. Sustained release formulations of the present disclosure may include a weight percentage of release modulating matrix of from about 10% to about 95%. In some embodiments, sustained release formulations of the present disclosure include a weight percentage of release modulating matrix of from about 50% to about 90%.

Sustained release formulations may include from about 10% by weight to about 50% by weight of C5 inhibitory cyclic polypeptides (e.g., zilucoplan and/or active metabolites or variants thereof). C5 inhibitory cyclic polypeptides may be uniformly distributed in release modulating matrices.

Sustained release formulations of the present disclosure may have different release profiles of therapeutic agents (e.g., C5 inhibitors, C5 inhibitor polypeptides, C5 inhibitory cyclic polypeptides, zilucoplan, and/or active metabolites or variants thereof). As used herein, the term "release profile" refers to a formulation characterization based on component diffusion properties. Release profiles for sustained release formulations or "sustained release profiles" may include characteristics of active ingredient diffusion from sustained release formulations. Such characteristics may include, but are not limited to, amount of active ingredient (e.g., therapeutic agents, C5 inhibitors, C5 inhibitor polypeptides, C5 inhibitory cyclic polypeptides, zilucoplan, and/or active metabolites or variants thereof) released over a specific time period, rate of active ingredient release over a specific time period, change in the amount or rate of active ingredient release over a specific time period, and total amount of active ingredient released over time. Specific time periods may be of varying lengths of time and may be characterized by different phases of active ingredient release. Such phases may include, but are not limited to, initial burst phases, lag phases, and sustained release phases. "Initial burst" refers to a period of elevated compound release following placement of sustained release formulations in a medium. "Lag phase" refers to a period immediately following an initial burst, where sustained release formulation compound levels are depleted from initial burst release and compound release proceeds at a much lower level. "Sustained release phase" refers to a period following initial burst and lag phases where compound release proceeds at a level below initial burst levels, but above lag phase levels as sustained release formulations equilibrate with surrounding medium and support a more steady release of compound. Sustained release phases may include a period of declining compound release levels as sustained release formulations are depleted of such compounds over time. Each phase of release may vary by length of time and compound release levels depending on multiple factors affecting compound release. Such factors may include, but are not limited to, properties of therapeutic agents (e.g., C5 inhibitors, C5 inhibitor polypeptides, C5 inhibitory cyclic polypeptides, zilucoplan, and/or active metabolites or variants thereof) incorporated into sustained release formulations, amount of therapeutic agents included in sustained release formulations, method of loading therapeutic agents into sustained release formulations, method of sustained release formulation preparation, sustained release formulation preparation method and composition, and properties of the medium into which therapeutic agents are released. Sustained release formulation composition for therapeutic agents (e.g., C5 inhibitors, C5 inhibitor polypeptides, C5 inhibitory cyclic polypeptides, zilucoplan, and/or active metabolites or variants thereof) and/or preparation methods thereof may be varied to achieve optimal sustained release phase lengths and corresponding release levels, all of which contribute to overall sustained release profile of therapeutic agents.

Sustained release formulations of the present disclosure may have different release profiles of C5 inhibitory cyclic polypeptides (e.g., zilucoplan and/or active metabolites or variants thereof). Each phase of release may vary by length of time and compound release levels depending on multiple factors affecting compound release. Such factors may include, but are not limited to, properties of C5 inhibitory cyclic polypeptides (e.g., zilucoplan and/or active metabolites or variants thereof) incorporated into sustained release formulations, amount of C5 inhibitory cyclic polypeptides (e.g., zilucoplan and/or active metabolites or variants thereof) included in sustained release formulations, method of loading C5 inhibitory cyclic polypeptides (e.g., zilucoplan and/or active metabolites or variants thereof) into sustained release formulations, method of sustained release formulation preparation, sustained release formulation preparation method and composition, and properties of the medium into which C5 inhibitory cyclic polypeptides (e.g., zilucoplan and/or active metabolites or variants thereof) are released. Sustained release formulation composition for C5 inhibitory cyclic polypeptides (e.g., zilucoplan and/or active metabolites or variants thereof) and/or preparation methods thereof may be varied to achieve optimal sustained release phase lengths and corresponding release levels, all of which contribute to overall sustained release profile of C5 inhibitory cyclic polypeptides (e.g., zilucoplan and/or active metabolites or variants thereof).

In some embodiments, sustained release formulations are modified to achieve sustained release profiles characterized by one or more of low initial burst, capability of achieving an effective concentration of active ingredient (e.g., therapeutic agents, C5 inhibitors, C5 inhibitor polypeptides, C5 inhibitory cyclic polypeptides, zilucoplan, and/or active metabolites or variants thereof) in a medium where the active ingredient is released from the sustained release formulation, and capability of achieving an effective concentration of the active ingredient in a medium where the active agent is released from the sustained release formulation over a specific period of time. As used herein, the term "effective concentration" refers to the mass per unit volume of an active ingredient (e.g., therapeutic agents, C5 inhibitors, C5 inhibitor polypeptides, C5 inhibitory cyclic polypeptides, zilucoplan, and/or active metabolites or variants thereof) required to achieve a specific effect.

Sustained release formulations including therapeutic agents (e.g., C5 inhibitors, C5 inhibitor polypeptides, C5 inhibitory cyclic polypeptides, zilucoplan, and/or active metabolites or variants thereof) as active ingredients may have sustained release profiles characterized by capability of achieving an effective concentration of the active ingredient for a period of from about one week to about three weeks in a medium where the active ingredient is released from the sustained release formulation. The effective concentration may be from about 4,000 ng/mL to about 12,000 ng/mL. The medium may be a subject or sample obtained from a subject administered such sustained release formulations.

Sustained release formulations including C5 inhibitory cyclic polypeptides (e.g., zilucoplan and/or active metabolites or variants thereof) as active ingredients may have sustained release profiles characterized by capability of achieving an effective concentration of the active ingredient for a period of from about one week to about three weeks in a medium where the active ingredient is released from the sustained release formulation. The effective concentration may be from about 4,000 ng/mL to about 12,000 ng/mL. The medium may be a subject or sample obtained from a subject administered such sustained release formulations.

Sustained release formulations including therapeutic agents (e.g., C5 inhibitors, C5 inhibitor polypeptides, C5 inhibitory cyclic polypeptides, zilucoplan, and/or active metabolites or variants thereof) as active ingredients may have sustained release profiles characterized by low initial burst. In some embodiments, the low initial burst is from about 0% to about 20% of the total amount of the active ingredient included in the sustained release formulation.

Sustained release formulations including C5 inhibitory cyclic polypeptides (e.g., zilucoplan and/or active metabolites or variants thereof) as active ingredients may have sustained release profiles characterized by low initial burst. In some embodiments, the low initial burst is from about 0% to about 20% of the total amount of the active ingredient included in the sustained release formulation.

In some embodiments, the present disclosure provides methods of preparing sustained release formulations (e.g., sustained release formulations that include therapeutic agents, C5 inhibitors, C5 inhibitor polypeptides, C5 inhibitory cyclic polypeptides, zilucoplan, and/or active metabolites or variants thereof). Such methods may include preparing organic phase solutions and aqueous phase solutions for emulsion preparations. Organic phase solutions may be prepared by dissolving PLGA in organic solvents. In some embodiments, active ingredients (e.g., therapeutic agents, C5 inhibitors, C5 inhibitor polypeptides, C5 inhibitory cyclic polypeptides, zilucoplan, and/or active metabolites or variants thereof) are included in organic phase solutions prepared for sustained release formulation preparation. Aqueous phase solutions may be prepared by combining an aqueous solution with an emulsion stabilizer. As used herein, the term "emulsion stabilizer" refers to any compound useful for supporting suspension of organic and aqueous phases in emulsions. In some embodiments, polyvinyl alcohol (PVA) is used as an emulsion stabilizer. Organic solvents may include dichloromethane (DCM). Aqueous solution may include phosphate buffered saline.

In some embodiments, the present disclosure provides methods of preparing sustained release formulations that include C5 inhibitory cyclic polypeptides (e.g., zilucoplan and/or active metabolites or variants thereof) of the present disclosure. Such methods may include preparing organic phase solutions and aqueous phase solutions for emulsion preparations. Organic phase solutions may be prepared by dissolving PLGA in organic solvents. In some embodiments, active ingredients (such as zilucoplan) are included in organic phase solutions prepared for sustained release formulation preparation. Aqueous phase solutions may be prepared by combining an aqueous solution with an emulsion stabilizer. In some embodiments, polyvinyl alcohol (PVA) is used as an emulsion stabilizer. Organic solvents may include dichloromethane (DCM). Aqueous solution may include phosphate buffered saline.

In some embodiments, organic solvents are removed from sustained release formulations after emulsification. Organic solvents may be removed by evaporation. The evaporation may be carried out by quench evaporation. As used herein, the term "quench evaporation" refers to an evaporation process wherein a formulation being subjected to evaporation is emersion in a solution that draws out organic solvent. Evaporation may be carried out by rotary evaporation. Rotary evaporation (rotavap) refers to an evaporation process utilizing a vacuum pump to reduce ambient pressure and speed up the evaporation process. Particles may be formed as a result of the evaporation, wherein remaining formulation components become condensed. Active ingredients (e.g., therapeutic agents, C5 inhibitors, C5 inhibitor polypeptides, C5 inhibitory cyclic polypeptides, zilucoplan, and/or active metabolites or variants thereof) may be unifonnly distributed in particles. Active ingredients (e.g., therapeutic agents, C5 inhibitors, C5 inhibitor polypeptides, C5 inhibitory cyclic polypeptides, zilucoplan, and/or active metabolites or variants thereof) may be included in solutions used for emulsion formation at concentrations sufficient to yield from about 10% by weight to about 50% by weight of the active ingredient in resulting sustained release formulations. Active ingredients (e.g., therapeutic agents, C5 inhibitors, C5 inhibitor polypeptides, C5 inhibitory cyclic polypeptides, zilucoplan, and/or active metabolites or variants thereof) may be included in solutions used for emulsion formation at concentrations sufficient to yield from about 36% by weight to about 38% by weight of such active ingredients in final sustained release formulations. Such active ingredients may include therapeutic agents, C5 inhibitors, and/or C5 inhibitor polypeptides. C5 inhibitor polypeptides may include SEQ ID NO: 1 (e.g., zilucoplan and/or active metabolites or variants thereof). Sustained release formulations may include zilucoplan-PLGA particles. C5 inhibitory cyclic polypeptides (e.g., zilucoplan and/or active metabolites or variants thereof) may be uniformly distributed in particles. C5 inhibitory cyclic polypeptides (e.g., zilucoplan and/or active metabolites or variants thereof) may be included in solutions used for emulsion formation at concentrations sufficient to yield from about 10% by weight to about 50% by weight of the C5 inhibitory cyclic polypeptides in resulting sustained release formulations. C5 inhibitory cyclic polypeptides (e.g., zilucoplan and/or active metabolites or variants thereof) may be included in solutions used for emulsion formation at concentrations sufficient to yield from about 36% by weight to about 38% by weight of such C5 inhibitory cyclic polypeptides in final sustained release formulations. Such C5 inhibitory cyclic polypeptides may include SEQ ID NO: 1. The C5 inhibitory cyclic polypeptides may include zilucoplan.

For treatment of human subjects, compounds and compositions of the present disclosure may be formulated as pharmaceutical compositions. Such pharmaceutical compositions may include any of the sustained release formulations described herein. Depending on the subject to be treated, the mode of administration, and the type of treatment desired (e.g., prevention, prophylaxis, or therapy) pharmaceutical compositions may be formulated in ways consonant with these parameters. A summary of such techniques is found in Remington: The Science and Practice of Pharmacy, 21 st Edition, Lippincott Williams & Wilkins, (2005); and Encyclopedia of Pharmaceutical Technology, eds. J. Swarbrick and J. C. Boylan, 1988-1999, Marcel Dekker, New York, each of which is incorporated herein by reference.

### Isotopic variations

Compounds (e.g., C5 inhibitors, C5 inhibitor polypeptides, C5 inhibitory cyclic polypeptides, zilucoplan, and/or active metabolites or variants thereof) of the present disclosure may include one or more atoms that are isotopes. As used herein, the term "isotope" refers to a chemical element that has one or more additional neutrons. In some embodiments, compounds of the present disclosure or components of formulations thereof may be deuterated. As used herein, the term "deuterated" refers to a substance that has had one or more hydrogen atoms replaced by deuterium isotopes. Deuterium isotopes are isotopes of hydrogen. The nucleus of hydrogen contains one proton while deuterium nuclei contain both a proton and a neutron. Compounds and compositions of the present disclosure may be deuterated in order to change a physical property, such as stability, or to allow for use in diagnostic and experimental applications.

C5 inhibitory cyclic polypeptides (e.g., zilucoplan and/or active metabolites or variants thereof) of the present disclosure may include one or more atoms that are isotopes. In some embodiments, C5 inhibitory cyclic polypeptides (e.g., zilucoplan and/or active metabolites or variants thereof) of the present disclosure or components of formulations thereof may be deuterated. C5 inhibitory cyclic polypeptides (e.g., zilucoplan and/or active metabolites or variants thereof) and compositions of the present disclosure may be deuterated in order to change a physical property, such as stability, or to allow for use in diagnostic and experimental applications.

### II. Methods

In some embodiments, the present disclosure provides methods related to preparing, using, and evaluating compounds (e.g., therapeutic agents, C5 inhibitors, C5 inhibitor polypeptides, C5 inhibitory cyclic polypeptides, zilucoplan, and/or active metabolites or variants thereof) described herein and compositions thereof. Such methods may include methods of using compounds of the present disclosure and compositions thereof for therapeutic applications. As used herein, the term "therapeutic application" refers to any activity conducted to alleviate, stabilize, improve, cure, or otherwise address any symptom, condition, disorder, or disease. Therapeutic applications may include modulating complement activity using complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof.

In some embodiments, the present disclosure provides methods related to preparing, using, and evaluating C5 inhibitory cyclic polypeptides (e.g., zilucoplan and/or active metabolites or variants thereof) and compositions thereof described herein. Such methods may include methods of using C5 inhibitory cyclic polypeptides (e.g., zilucoplan and/or active metabolites or variants thereof) and compositions of the present disclosure for therapeutic applications. Therapeutic applications may include modulating complement activity using C5 inhibitory cyclic polypeptides (e.g., zilucoplan and/or active metabolites or variants thereof) and/or compositions described herein.

### Therapeutic indications

In some embodiments, methods of the present disclosure include methods of treating therapeutic indications using compounds (e.g., C5 inhibitors, C5 inhibitor polypeptides, C5 inhibitory cyclic polypeptides, zilucoplan, and/or active metabolites or variants thereof) described herein and/or compositions thereof. As used herein, the term "therapeutic indication" refers to any symptom, condition, disorder, or disease that may be alleviated, stabilized, improved, cured, or otherwise addressed by some form of treatment or other therapeutic intervention (e.g., through complement inhibitor administration). Therapeutic indications may include, but are not limited to, inflammatory indications, wounds, injuries, autoimmune indications, vascular indications, neurological indications, kidney-related indications, ocular indications, cardiovascular indications, pulmonary indications, and pregnancy-related indications. Therapeutic indications associated with complement activity and/or dysfunction are referred to herein as "complement-related indications." In some embodiments, methods of the present disclosure may include treating complement-related indications by administering complement inhibitor compounds described herein and/or compositions thereof. Administration may include the use of sustained release formulations described herein, including those formulated for sustained release of zilucoplan.

In some embodiments, methods of the present disclosure include methods of treating therapeutic indications using C5 inhibitory cyclic polypeptides (e.g., zilucoplan and/or active metabolites or variants thereof) and/or compositions disclosed herein. Therapeutic indications may include, but are not limited to, inflammatory indications, wounds, injuries, autoimmune indications, vascular indications, neurological indications, kidney-related indications, ocular indications, cardiovascular indications, pulmonary indications, and pregnancy-related indications. In some embodiments, methods of the present disclosure may include treating complement-related indications by administering C5 inhibitory cyclic polypeptides (e.g., zilucoplan and/or active metabolites or variants thereof) and/or compositions thereof disclosed herein (e.g., complement inhibitor compounds).

In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be useful in the treatment of complement-related indications where complement activation leads to progression of a disease, disorder and/or condition. Such complement-related indications may include, but are not limited to inflammatory indications, wounds, injuries, autoimmune indications, vascular indications, neurological indications, kidney-related indications, ocular indications, cardiovascular indications, pulmonary indications, and pregnancy-related indications. Complement-related indications may include, but are not limited to, any of those listed in United States Patent Number 10,106,579, the contents of which are herein incorporated by reference in their entirety.

In some embodiments, C5 inhibitory cyclic polypeptides (e.g., zilucoplan and/or active metabolites or variants thereof) may be useful in the treatment of complement-related indications where complement activation leads to progression of a disease, disorder and/or condition. Such complement-related indications may include, but are not limited to inflammatory indications, wounds, injuries, autoimmune indications, vascular indications, neurological indications, kidney-related indications, ocular indications, cardiovascular indications, pulmonary indications, and pregnancy-related indications. Complement-related indications may include, but are not limited to, any of those listed in United States Patent Number 10,106,579, the contents of which are herein incorporated by reference in their entirety.

Complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be useful in the treatment of infectious diseases, disorders and/or conditions, for example, in a subject having an infection. In some embodiments, subjects having an infection or that are at risk of developing sepsis or a septic syndrome may be treated with complement inhibitors described herein. In some cases, complement inhibitor compounds may be used in the treatment of sepsis.

C5 inhibitory cyclic polypeptides (e.g., zilucoplan and/or active metabolites or variants thereof) and compositions thereof may be useful in the treatment of infectious diseases, disorders and/or conditions, for example, in a subject having an infection. In some embodiments, subjects having an infection or that are at risk of developing sepsis or a septic syndrome may be treated with C5 inhibitory cyclic polypeptides (e.g., zilucoplan and/or active metabolites or variants thereof) described herein. In some cases, C5 inhibitory cyclic polypeptides (e.g., zilucoplan and/or active metabolites or variants thereof) may be used in the treatment of sepsis.

Complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may also be administered to improve the outcome of clinical procedures wherein complement inhibition is desired. Such procedures may include, but are not limited to grafting, transplantation, implantation, catheterization, intubation and the like. In some embodiments, complement inhibitor compounds and compositions are used to coat devices, materials and/or biomaterials used in such procedures. In some embodiments, the inner surface of a tube may be coated with compounds and compositions to prevent complement activation within a bodily fluid that passes through the tube, either in vivo or ex vivo, e.g., extracorporeal shunting, e.g., dialysis and cardiac bypass.

C5 inhibitory cyclic polypeptides (e.g., zilucoplan and/or active metabolites or variants thereof) and compositions thereof may also be administered to improve the outcome of clinical procedures wherein complement inhibition is desired. Such procedures may include, but are not limited to grafting, transplantation, implantation, catheterization, intubation and the like. In some embodiments, C5 inhibitory cyclic polypeptides (e.g., zilucoplan and/or active metabolites or variants thereof) and compositions thereof are used to coat devices, materials and/or biomaterials used in such procedures. In some embodiments, the inner surface of a tube may be coated with C5 inhibitory cyclic polypeptides (e.g., zilucoplan and/or active metabolites or variants thereof) and compositions thereof to prevent complement activation within a bodily fluid that passes through the tube, either in vivo or ex vivo, e.g., extracorporeal shunting, e.g., dialysis and cardiac bypass.

As used herein the terms "treat," "treatment," and the like, refer to relief from or alleviation of pathological processes. In the context of the present disclosure insofar as it relates to any of the other conditions recited herein below, the terms "treat," "treatment," and the like mean to relieve or alleviate at least one symptom associated with such condition, or to slow or reverse the progression or anticipated progression of such condition.

By "lower" or "reduce" in the context of a disease marker or symptom is meant a significant decrease in such a level, often statistically significant. The decrease may be, for example, at least 10%, at least 20%, at least 30%, at least 40% or more, and is preferably down to a level accepted as within the range of normal for an individual without such a disorder.

By "increase" or "raise" in the context of a disease marker or symptom is meant a significant rise in such level, often statistically significant. The increase may be, for example, at least 10%, at least 20%, at least 30%, at least 40% or more, and is preferably up to a level accepted as within the range of normal for an individual without such disorder.

Efficacy of treatment or amelioration of disease can be assessed, for example by measuring disease progression, disease remission, symptom severity, reduction in pain, quality of life, dose of a medication required to sustain a treatment effect, level of a disease marker or any other measurable parameter appropriate for a given disease being treated or targeted for prevention. It is well within the ability of one skilled in the art to monitor efficacy of treatment or prevention by measuring any one of such parameters, or any combination of parameters. In connection with the administration of a polypeptide or pharmaceutical composition thereof, "effective against" a disease or disorder indicates that administration in a clinically appropriate manner results in a beneficial effect for at least a fraction of patients, such as an improvement of symptoms, a cure, a reduction in disease load, reduction in tumor mass or cell numbers, extension of life, improvement in quality of life, a reduction in the need for blood transfusions or other effect generally recognized as positive by medical doctors familiar with treating the particular type of disease or disorder.

A treatment or preventive effect is evident when there is a significant improvement, often statistically significant, in one or more parameters of disease status, or by a failure to worsen or to develop symptoms where they would otherwise be anticipated. As an example, a favorable change of at least 10% in a measurable parameter of disease, and preferably at least 20%, 30%, 40%, 50% or more may be indicative of effective treatment. Efficacy for a given compound or composition may also be judged using an experimental animal model for the given disease as known in the art. When using an experimental animal model, efficacy of treatment is evidenced when a statistically significant modulation in a marker or symptom is observed.

Compounds of the present disclosure and additional therapeutic agents can be administered in combination. Such combinations may be in the same composition, or the additional therapeutic agents can be administered as part of a separate composition or by another method described herein.

### Paroxysmal nocturnal hemoglobinuria (PNH)

Complement-related indications may include paroxysmal nocturnal hemoglobinuria (PNH). In some embodiments, complement inhibitor compounds and compositions may be used to treat, prevent or delay development of PNH. In some embodiments, the treatment may be involved with the prevention of hemolysis of PNH erythrocytes in a dose dependent manner.

An acquired mutation in the phosphatidylinositol glycan anchor biosynthesis, class A (PIG-A) gene that originates from a multipotent hematopoietic stem cell results in a rare disease known as paroxysmal nocturnal hemoglobinuria (PNH) (Pu, J.J. et al., Paroxysmal nocturnal hemoglobinuria from bench to bedside. Clin Transl Sci. 2011 Jun;4(3):219-24). PNH is characterized by bone marrow disorder, hemolytic anemia and thrombosis. The PIG-A gene product is necessary for the production of a glycolipid anchor, glycosylphosphatidylinositol (GPI), utilized to tether proteins to the plasma membrane. Two complement-regulatory proteins, CD55 and CD59, become nonfunctional in the absence of GPI. This leads to complement-mediated destruction of these cells. Complement inhibitors are particularly useful in the treatment of PNH. In some embodiments, compounds and compositions may be used to treat, prevent or delay development of Paroxysmal nocturnal hemoglobinuria (PNH) or anemias associated with complement. Subjects with PNH are unable to synthesize functional versions of the complement regulatory proteins CD55 and CD59 on hematopoietic stem cells. This results in complement-mediated hemolysis and a variety of downstream complications. As used herein, the term "downstream" or "downstream complication" refers to any event occurring after and as a result of another event. In some cases, downstream events are events occurring after and as a result of C5 cleavage and/or complement activation.

PNH is characterized by low hemoglobin, increased levels of lactate dehydrogenase and bilirubin, and decreased level of haptoglobin. Symptoms of PNH include symptoms of anemia, such as tiredness, headaches, dyspnea, chest pain, dizziness, and feeling of lightheadedness.

Current treatments for PNH include the use of eculizumab (Alexion Pharmaceuticals, Cheshire, CT). In some cases, eculizumab may be ineffective due to mutation in C5, short half-life, immune reaction, or other reason. In some embodiments, methods of the present disclosure include methods of treating subjects with PNH, wherein such subjects have been treated previously with eculizumab. In some cases, eculizumab is ineffective in such subjects, making treatment with compounds of the present disclosure important for therapeutic relief. In some embodiments, compounds of the present disclosure may be used to treat subjects that are resistant to eculizumab treatment. Such subjects may include subjects with the R885H/C polymorphism, which confers resistance to eculizumab. In some cases, compounds of the present disclosure are administered simultaneously or in conjunction with eculizumab therapy. In such cases, subjects may experience one or more beneficial effects of such combined treatment, including, but not limited to more effective relief, faster relief and/or fewer side effects.

### Inflammatory indications

Therapeutic indications that may be addressed with compounds (e.g., C5 inhibitors, C5 inhibitor polypeptides, C5 inhibitory cyclic polypeptides, zilucoplan, and/or active metabolites or variants thereof) described herein and/or compositions thereof may include inflammatory indications. As used herein, the term "inflammatory indication" refers to therapeutic indications that involve immune system activation. Inflammatory indications may include complement-related indications. Inflammation may be upregulated during the proteolytic cascade of the complement system. Although inflammation may have beneficial effects, excess inflammation may lead to a variety of pathologies (Markiewski et al. 2007. Am J Pathol. 17: 715-27). In some embodiments, complement inhibitor compounds and compositions of the present disclosure may be used to treat, prevent, or delay development of inflammatory indications.

Inflammatory indications may include, but are not limited to, Acute Disseminated Encephalomyelitis (ADEM), Acute necrotizing hemorrhagic leukoencephalitis, Addison's disease, Agammaglobulinemia, Alopecia areata, Amyloidosis, Ankylosing spondylitis, Acute antibody-mediated rejection following organ transplantation, Anti-GBM/Anti-TBM nephritis, Antiphospholipid syndrome (APS), Autoimmune angioedema, Autoimmune aplastic anemia, Autoimmune dysautonomia, Autoimmune hepatitis, Autoimmune hyperlipidemia, Autoimmune immunodeficiency, Autoimmune inner ear disease (AIED), Autoimmune myocarditis, Autoimmune pancreatitis, Autoimmune retinopathy, Autoimmune thrombocytopenic purpura (ATP), Autoimmune thyroid disease, Autoimmune urticaria, Axonal & neuronal neuropathies, Bacterial sepsis and septic shock, Balo disease, Behcet's disease, Bullous pemphigoid, Cardiomyopathy, Castleman disease, Celiac disease, Chagas disease, Chronic fatigue syndrome, Chronic inflammatory demyelinating polyneuropathy (CIDP), Chronic recurrent multifocal osteomyelitis (CRMO), Churg-Strauss syndrome, Cicatricial pemphigoid/benign mucosal pemphigoid, Crohn's disease, Cogans syndrome, Cold agglutinin disease, Congenital heart block, Coxsackie myocarditis, CREST disease, Essential mixed cryoglobulinemia, Demyelinating neuropathies, Dermatitis herpetiformis, Dermatomyositis, Devic's disease (neuromyelitis optica), Diabetes Type I, Discoid lupus, Dressler's syndrome, Endometriosis, Eosinophilic esophagitis, Eosinophilic fasciitis, Erythema nodosum, Experimental allergic encephalomyelitis, Evans syndrome, Fibromyalgia, Fibrosing alveolitis, Giant cell arteritis (temporal arteritis), Glomerulonephritis, Goodpasture's syndrome, Granulomatosis with Polyangiitis (GPA) see Wegener's, Graves' disease, Guillain-Barre syndrome, Hashimoto's encephalitis, Hashimoto's thyroiditis, Hemolytic anemia (including atypical hemolytic uremic syndrome and plasma therapy-resistant atypical hemolytic-uremic syndrome), Henoch-Schonlein purpura, Herpes gestationis, Hypogammaglobulinemia, Idiopathic thrombocytopenic purpura (ITP), IgA nephropathy, IgG4-related sclerosing disease, Immunoregulatory lipoproteins, Inclusion body myositis, Insulin-dependent diabetes (type1), Interstitial cystitis, Juvenile arthritis, Juvenile diabetes, Kawasaki syndrome, Lambert-Eaton syndrome, Large vessel vasculopathy, Leukocytoclastic vasculitis, Lichen planus, Lichen sclerosus, Ligneous conjunctivitis, Linear IgA disease (LAD), Lupus (SLE), Lyme disease, Meniere's disease, Microscopic polyangiitis, Mixed connective tissue disease (MCTD), Mooren's ulcer, Mucha-Habermann disease, Multiple endocrine neoplasia syndromes, Multiple sclerosis, Multifocal motor neuropathy, Myositis, Myasthenia gravis, Narcolepsy, Neuromyelitis optica (Devic's), Neutropenia, Ocular cicatricial pemphigoid, Optic neuritis, Osteoarthritis, Palindromic rheumatism, PANDAS (Pediatric Autoimmune Neuropsychiatric Disorders Associated with Streptococcus), Paraneoplastic cerebellar degeneration, Paroxysmal nocturnal hemoglobinuria (PNH), Parry Romberg syndrome, Parsonnage-Turner syndrome, Pars planitis (peripheral uveitis), Pemphigus, Peripheral neuropathy, Perivenous encephalomyelitis, Pernicious anemia, POEMS syndrome, Polyarteritis nodosa, Type I, II, & III autoimmune polyglandular syndromes, Polyendocrinopathies, Polymyalgia rheumatica, Polymyositis, Postmyocardial infarction syndrome, Postpericardiotomy syndrome, Progesterone dermatitis, Primary biliary cirrhosis, Primary sclerosing cholangitis, Psoriasis, Psoriatic arthritis, Idiopathic Pulmonary fibrosis, Pyoderma gangrenosum, Pure red cell aplasia, Raynauds phenomenon, Reactive arthritis, Reflex sympathetic dystrophy, Reiter's syndrome, Relapsing polychondritis, Restless legs syndrome, Retroperitoneal fibrosis, Rheumatic fever, Rheumatoid arthritis, Sarcoidosis, Schmidt syndrome, Scleritis, Scleroderma, Shiga-Toxin producing Escherichia Coli Hemolytic-Uremic Syndrome (STEC-HUS), Sjogren's syndrome, Small vessel vasculopathy, Sperm & testicular autoimmunity, Stiff person syndrome, Subacute bacterial endocarditis (SBE), Susac's syndrome, Sympathetic ophthalmia, Takayasu's arteritis, Temporal arteritis/Giant cell arteritis, Thrombocytopenic purpura (TTP), Tolosa-Hunt syndrome, Transverse myelitis, Tubular autoimmune disorder, Ulcerative colitis, Undifferentiated connective tissue disease (UCTD), Uveitis, Vesiculobullous dermatosis, Vasculitis, Vitiligo, and Wegener's granulomatosis (also known as Granulomatosis with Polyangiitis (GPA)).

### Sterile inflammation

Inflammatory indications may include sterile inflammation. Sterile inflammation is inflammation that occurs in response to stimuli other than infection. Sterile inflammation may be a common response to stress such as genomic stress, hypoxic stress, nutrient stress or endoplasmic reticulum stress caused by a physical, chemical, or metabolic noxious stimuli. Sterile inflammation may contribute to pathogenesis of many diseases such as, but not limited to, ischemia-induced injuries, rheumatoid arthritis, acute lung injuries, drug-induced liver injuries, inflammatory bowel diseases and/or other diseases, disorders or conditions. Mechanism of sterile inflammation and methods and compounds for treatment, prevention and/or delaying of symptoms of sterile inflammation may include any of those taught by Rubartelli et al. in Frontiers in Immunology, 2013, 4:398-99, Rock et al. in Annu Rev Immunol. 2010, 28:321-342 or in United States Patent No. 8,101,586, the contents of each of which are herein incorporated by reference in their entirety. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent or delay development of sterile inflammation. In some embodiments, C5 inhibitory cyclic polypeptides (e.g., zilucoplan and/or active metabolites or variants thereof) and compositions of the present disclosure may be used to treat, prevent or delay development of sterile inflammation.

### Systemic inflammatory response (SIRS) and sepsis

Inflammatory indications may include systemic inflammatory response syndrome (SIRS). SIRS is inflammation affecting the whole body. Where SIRS is caused by an infection, it is referred to as sepsis. SIRS may also be caused by non-infectious events such as trauma, injury, burns, ischemia, hemorrhage and/or other conditions. During sepsis and SIRS, complement activation leads to excessive generation of complement activation products which may cause multi organ failure (MOF) in subjects. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat and/or prevent SIRS. In some embodiments, C5 inhibitory cyclic polypeptides (e.g., zilucoplan and/or active metabolites or variants thereof) and compositions of the present disclosure may be used to treat and/or prevent SIRS. Complement inhibitor compounds and compositions may be used to control and/or balance complement activation for prevention and treatment of SIRS, sepsis and/or MOF. C5 inhibitory cyclic polypeptides (e.g., zilucoplan and/or active metabolites or variants thereof) and compositions may be used to control and/or balance complement activation for prevention and treatment of SIRS, sepsis and/or MOF. The methods of applying complement inhibitors to treat SIRS and sepsis may include those taught by Rittirsch et al. in Clin Dev Immunol, 2012, 962927, in U.S. publication No. US2013/0053302 or in United States Patent No. 8,329,169, the contents of each of which are herein incorporated by reference in their entirety.

### Acute respiratory distress syndrome (ARDS)

Inflammatory indications may include acute respiratory distress syndrome (ARDS). ARDS is a widespread inflammation of the lungs and may be caused by trauma, infection (e.g., sepsis), severe pneumonia and/or inhalation of harmful substances. ARDS is typically a severe, life-threatening complication. Studies suggest that neutrophils may contribute to development of ARDS by affecting the accumulation of polymorphonuclear cells in the injured pulmonary alveoli and interstitial tissue of the lungs. In some embodiments, complement inhibitor compounds (e.g., C5 inhibitors, C5 inhibitor polypeptides, C5 inhibitory cyclic polypeptides, zilucoplan and/or active metabolites or variants thereof) described herein and/or compositions thereof may be used to treat and/or prevent development of ARDS. Complement inhibitor compounds and compositions thereof may be administered to reduce and/or prevent tissue factor production in alveolar neutrophils. In some embodiments, C5 inhibitory cyclic polypeptides (e.g., zilucoplan and/or active metabolites or variants thereof) and compositions of the present disclosure may be used to treat and/or prevent development of ARDS. C5 inhibitory cyclic polypeptides (e.g., zilucoplan and/or active metabolites or variants thereof) and compositions thereof may be administered to reduce and/or prevent tissue factor production in alveolar neutrophils. Complement inhibitor compounds and compositions thereof may further be used for treatment, prevention and/or delaying of ARDS, in some cases according to any of the methods taught in International Publication No. WO2009/014633, the contents of which are herein incorporated by reference in their entirety. C5 inhibitory cyclic polypeptides (e.g., zilucoplan and/or active metabolites or variants thereof) and compositions thereof may further be used for treatment, prevention and/or delaying of ARDS, in some cases according to any of the methods taught in International Publication No. WO2009/014633, the contents of which are herein incorporated by reference in their entirety.

### Periodontitis

Inflammatory indications may include periodontitis. Periodontitis is a widespread, chronic inflammation leading to the destruction of periodontal tissue which is the tissue supporting and surrounding the teeth. The condition also involves alveolar bone loss (bone that holds the teeth). Periodontitis may be caused by a lack of oral hygiene leading to accumulation of bacteria at the gum line, also known as dental plaque. Certain health conditions such as diabetes or malnutrition and/or habits such as smoking may increase the risk of periodontitis. Periodontitis may increase the risk of stroke, myocardial infarction, atherosclerosis, diabetes, osteoporosis, pre-term labor, as well as other health issues. Studies demonstrate a correlation between periodontitis and local complement activity. Periodontal bacteria may either inhibit or activate certain components of the complement cascade. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat or prevent development of periodontitis and/or associated conditions. In some embodiments, C5 inhibitory cyclic polypeptides (e.g., zilucoplan and/or active metabolites or variants thereof) and compositions of the present disclosure may be used to treat or prevent development of periodontitis and/or associated conditions. Complement activation inhibitors and treatment methods may include any of those taught by Hajishengallis in Biochem Pharmacol. 2010, 15; 80(12): 1 and Lambris or in US publication No. US2013/0344082, the contents of each of which are herein incorporated by reference in their entirety.

### Dermatomyositis

Inflammatory indications may include dermatomyositis. Dermatomyositis is an inflammatory myopathy characterized by muscle weakness and chronic muscle inflammation. Dermatomyositis often begins with a skin rash that is associated concurrently or precedes muscle weakness. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of dermatomyositis. In some embodiments, C5 inhibitory cyclic polypeptides (e.g., zilucoplan and/or active metabolites or variants thereof) and compositions of the present disclosure may be used to treat, prevent, or delay development of dermatomyositis.

### Rheumatoid arthritis

Inflammatory indications may include rheumatoid arthritis. Rheumatoid arthritis is an autoimmune condition affecting the wrists and small joints of the hands. Typical symptoms include pain, stiffness of the joints, swelling, and feeling of warmth. Activated components of the complement system affect development of rheumatoid arthritis, as products of complement cascade mediate proinflammatory activities, such as vascular permeability and tone, leukocyte chemotaxis and the activation and lysis of multiple cell types (see Wang, et al., Proc. Natl. Acad. Sci., 1995; 92: 8955-8959). Wang et al. demonstrated that inhibition of C5 complement cascade in animals prevented the onset of arthritis and ameliorated established condition. Complement activation inhibitors and treatment methods may include any of those taught by Wang, et al., Proc. Natl. Acad. Sci., 1995; 92: 8955-8959, the contents of which are herein incorporated by reference in their entirety. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat or prevent development of rheumatoid arthritis. In some embodiments, C5 inhibitory cyclic polypeptides (e.g., zilucoplan and/or active metabolites or variants thereof) and compositions of the present disclosure may be used to treat or prevent development of rheumatoid arthritis.

### Asthma

Inflammatory indications may include asthma. Asthma is a chronic inflammation of the bronchial tubes, which are the airways allowing air to pass in and out of the lungs. The condition is characterized by narrowing, inflammation and hyperresponsiveness of the tubes. Typical symptoms include periods of wheezing, chest tightness, coughing and shortness of breath. Asthma the most common respiratory disorder. Complement proteins C3 and C5 are associated with many pathophysiological features of asthma, such as inflammatory cell infiltration, mucus secretion, increased vascular permeability, and smooth muscle cell contraction, and therefore it has been suggested that downregulation of complement activation may be used to treat, manage or prevent asthma. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of asthma. In some embodiments, C5 inhibitory cyclic polypeptides (e.g., zilucoplan and/or active metabolites or variants thereof) and compositions of the present disclosure may be used to treat, prevent, or delay development of asthma. Complement activation inhibitors and treatment methods may include any of those taught by Khan et al., Respir Med. 2014 April ; 108(4): 543-549, the contents of which are herein incorporated by reference in their entirety.

### Anaphylaxis

Inflammatory indications may include anaphylaxis. Anaphylaxis is a severe and potentially life-threatening allergic reaction. Anaphylaxis may lead to a shock characterized e.g. by sudden drop of blood pressure, narrowing of airways, breathing difficulties, rapid and weak pulse, a rash, nausea and vomiting. The cardiopulmonary collapse during anaphylaxis has been associated with complement activation and generation of C3a and C5a anaphylatoxins. Balzo et al. report animal studies indicating that complement activation markedly enhance cardiac dysfunction during anaphylaxis (Balzo et al., Circ Res. 1989 Sep;65(3):847-57). Complement activation inhibitors and treatment methods may include any of those taught by Balzo et al., the contents of which are herein incorporated by reference in their entirety. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of anaphylaxis. In some embodiments, C5 inhibitory cyclic polypeptides (e.g., zilucoplan and/or active metabolites or variants thereof) and compositions of the present disclosure may be used to treat, prevent, or delay development of anaphylaxis.

### Bowel inflammation

Inflammatory indications may include inflammatory bowel disease (IBD). IBD is a reoccurring condition with periods of mild to severe inflammation or periods of remission. Common symptoms include diarrhea, fatigue and fever, abdominal pain, weight loss, reduced appetite and bloody stool. Types of IBD include ulcerative proctitis, dextran sulfate sodium colitis, proctosigmoitidis, left-sided colitis, panconlitis, acute severe ulcerative colitis. IBD, such as dextran sulfate sodium colitis and ulcerative colitis, have been associated with complement activity (Webb et al., Int J Med Pharm Case Reports. 2015; 4(5): 105-112 and Aomatsu et al., J Clin Biochem Nutr. 2013;52(1):72-5). Complement activation inhibitors and treatment methods may include any of those taught by Webb et al. or Aomatsu et al, the contents of each of which are herein incorporated by reference in their entirety. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of IBD. In some embodiments, C5 inhibitory cyclic polypeptides (e.g., zilucoplan and/or active metabolites or variants thereof) and compositions of the present disclosure may be used to treat, prevent, or delay development of IBD.

### Systemic inflammation during cardiopulmonary bypass

Inflammatory indications may include inflammatory response induced by cardiopulmonary bypass (CBP). CBP is a technique used during surgery to take over the function of heart and lungs to maintain blood circulation and oxygen concentration of the blood. CBD provokes a systemic inflammatory response that may lead to complications of the surgical patients. The suggested cause may be due to contact activation of blood with artificial surfaces during extracorporeal circulation. The inflammation response may lead to SIRS and be life-threatening.

Complement activation has been associated with the inflammatory response induced by CBP. Studies have suggested that terminal components C5a and C5b-9 directly contribute to platelet and neutrophil activation during the extracorporeal blood circulation and C5 has been identified as a therapeutic site for prevention and treatment of inflammatory response induced by CBP (Rinder et al. J Clin Invest. 1995; 96(3): 1564-1572). Complement activation inhibitors and treatment methods may include any of those taught by Rinder et al. J Clin Invest. 1995; 96(3): 1564-1572, the contents of which are herein incorporated by reference in their entirety. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of inflammatory response induced by CBP. In some embodiments, C5 inhibitory cyclic polypeptides (e.g., zilucoplan and/or active metabolites or variants thereof) and compositions of the present disclosure may be used to treat, prevent, or delay development of inflammatory response induced by CBP.

### Rejection in organ or tissue transplant

Inflammatory indications may include immune rejection of transplants. Transplants may be organs (e.g. heart, kidneys, liver, lungs, intestine, thymus and pancreas) or tissues (e.g. bones, tendons, skin, cornea, veins). Different types of transplants include autograft (transplanting patient's own tissue), allograft (transplant between two members of the same species) or xenograft (transplant between members of different species, e.g. from an animal to a human). Complications after organ transplant arise as the recipient's immune system attacks the transplanted tissue. The rejection may be hyperacute referring to a reaction occurring within few minutes after the transplant is performed, and typically occurs when the antigens are unmatched. Acute rejection occurs within a week or few months after transplant. Some rejections are chronic and take place over many years.

Transplant rejection and related inflammation has been associated with complement system. The complement cascade is relevant to transplantation in a number of ways, e.g. as an effector mechanism of antibody-initiated allograft injury, promotion of ischemia-reperfusion injury, and formation and function of alloantibodies (Sheen and Heeger, Curr Opin Organ Transplant. 2015;20(4):468-75). Therapy targeting complement has been suggested to have significance for the survival and health of transplant patients As an example, studies have shown that C5 blockage of C5 with eculizumab reduces the incidence of early antibody-mediated rejection (AMR) of organ allografts (Stegall et al., Nature Reviews Nephrology 8(11):670-8, 2012) and inhibition of C5 may prevent acute cardiac tissue injury in an ex vivo model of pig-to-human xenotransplantation (Kroshus et al, Transplantation. 1995,15;60(11):1194-202.) Complement activation inhibitors and treatment methods may include any of those taught by Stegall et al., Nature Reviews Nephrology 8(11):670-8, 2012 and Kroshus et al, Transplantation. 1995,15;60(11): 1194-202, and (Sheen and Heeger, Curr Opin Organ Transplant. 2015;20(4):468-75, the contents of each of which are herein incorporated by reference in their entirety. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat subjects with or receiving transplanted organs or tissues. In some embodiments, C5 inhibitory cyclic polypeptides (e.g., zilucoplan and/or active metabolites or variants thereof) and/or compositions of the present disclosure may be used to treat subjects with or receiving transplanted organs or tissues.

### Wounds and injuries

Therapeutic indications that may be addressed with compounds and/or compositions of the present disclosure may include wounds and injuries. As used herein, the term "injury" typically refers to physical trauma, but may include localized infection or disease processes. Injuries may be characterized by harm, damage or destruction caused by external events affecting body parts and/or organs. Non-limiting examples of injuries include head trauma and crush injuries. Wounds are associated with cuts, blows, burns and/or other impacts to the skin, leaving the skin broken or damaged. Wounds and injuries may include complement-related indications. Wounds and injuries are often acute but if not healed properly they may lead to chronic complications and/or inflammation. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat and/or promote healing of different types of wounds and/or injuries. In some embodiments, C5 inhibitory cyclic polypeptides (e.g., zilucoplan and/or active metabolites or variants thereof) and compositions of the present disclosure may be used to treat and/or promote healing of different types of wounds and/or injuries.

### Wounds and burn wounds

**In** some embodiments, complement inhibitor compounds and compositions of the present disclosure may be used to treat and/or to promote healing of wounds. Healthy skin provides a waterproof, protective barrier against pathogens and other environmental effectors. The skin also controls body temperature and fluid evaporation. When skin is wounded these functions are disrupted making skin healing challenging. Wounding initiates a set of physiological processes related to the immune system that repair and regenerate tissue. Complement activation is one of these processes. Complement activation studies have identified several complement components involved with wound healing as taught by van de Goot et al. in J Burn Care Res 2009, 30:274-280 and Cazander et al. Clin Dev Immunol, 2012, 2012:534291, the contents of each of which are herein incorporated by reference in their entirety. In some cases, complement activation may be excessive, causing cell death and enhanced inflammation (leading to impaired wound healing and chronic wounds). In some cases, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to reduce or eliminate such complement activation to promote wound healing. Treatment with complement inhibitor compounds and compositions may be carried out according to any of the methods for treating wounds disclosed in International Publication No. WO2012/174055, the contents of which are herein incorporated by reference in their entirety. In some cases, C5 inhibitory cyclic polypeptides (e.g., zilucoplan and/or active metabolites or variants thereof) and compositions may be used to reduce or eliminate such complement activation to promote wound healing. Treatment with C5 inhibitory cyclic polypeptides (e.g., zilucoplan and/or active metabolites or variants thereof) and compositions thereof may be carried out according to any of the methods for treating wounds disclosed in International Publication No. WO2012/174055, the contents of which are herein incorporated by reference in their entirety.

### Head trauma

Wounds and/or injuries may include head trauma. Head traumas include injuries to the scalp, the skull or the brain. Examples of head trauma include, but are not limited to concussions, contusions, skull fracture, traumatic brain injuries and/or other injuries. Head traumas may be minor or severe. In some cases, head trauma may lead to long term physical and/or mental complications or death. Studies indicate that head traumas may induce improper intracranial complement cascade activation, which may lead to local inflammatory responses contributing to secondary brain damage by development of brain edema and/or neuronal death (Stahel et al. in Brain Research Reviews, 1998, 27: 243-56, the contents of which are herein incorporated by reference in their entirety). In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat head trauma and/or prevent or delay development of diseases, disorders, and/or conditions associated with head trauma. In some embodiments, complement inhibitor compounds and compositions may be used to treat, prevent, reduce, or delay development of secondary complications of head trauma. Methods of using complement inhibitor compounds and compositions to control complement cascade activation in head trauma may include any of those taught by Holers et al. in United States Patent No. 8,911,733, the contents of which are herein incorporated by reference in their entirety. In some embodiments, C5 inhibitory cyclic polypeptides (e.g., zilucoplan and/or active metabolites or variants thereof) and compositions of the present disclosure may be used to treat head trauma and/or prevent or delay development of diseases, disorders, and/or conditions associated with head trauma. In some embodiments, C5 inhibitory cyclic polypeptides (e.g., zilucoplan and/or active metabolites or variants thereof) and compositions may be used to treat, prevent, reduce, or delay development of secondary complications of head trauma. Methods of using C5 inhibitory cyclic polypeptides (e.g., zilucoplan and/or active metabolites or variants thereof) and compositions to control complement cascade activation in head trauma may include any of those taught by Holers et al. in United States Patent No. 8,911,733, the contents of which are herein incorporated by reference in their entirety.

### Crush injury

Wounds and/or injuries may include crush injuries. Crush injuries are injuries caused by a force or a pressure put on the body causing bleeding, bruising, fractures, nerve injuries, wounds and/or other damages to the body. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat and/or promote healing of crush injuries. In some embodiments, C5 inhibitory cyclic polypeptides (e.g., zilucoplan and/or active metabolites or variants thereof) and compositions of the present disclosure may be used to treat and/or promote healing of crush injuries. Treatment may be used to reduce complement activation following crush injuries, thereby promoting healing after crush injuries (e.g., by promoting nerve regeneration, promoting fracture healing, preventing or treating inflammation, and/or other related complications). Complement inhibitor compounds described herein and/or compositions thereof may be used to promote healing according to any of the methods taught in United States Patent No. 8,703,136; International Publication Nos. WO2012/162215; WO2012/174055; or US publication No. US2006/0270590, the contents of each of which are herein incorporated by reference in their entirety. C5 inhibitory cyclic polypeptides (e.g., zilucoplan and/or active metabolites or variants thereof) and compositions thereof may be used to promote healing according to any of the methods taught in United States Patent No. 8,703,136; International Publication Nos. WO2012/162215; WO2012/174055; or US publication No. US2006/0270590, the contents of each of which are herein incorporated by reference in their entirety.

### Autoimmune indications

Therapeutic indications addressed with complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may include autoimmune indications. As used herein, the term "autoimmune indication" refers to any therapeutic indication relating to immune targeting of a subject's tissues and/or substances by the subject's own immune system. Autoimmune indications may include complement-related indications. Autoimmune indications may involve certain tissues or organs of the body. The immune system may be divided into innate and adaptive systems, referring to nonspecific immediate defense mechanisms and more complex antigen-specific systems, respectively. The complement system is part of the innate immune system, recognizing and eliminating pathogens. Additionally, complement proteins may modulate adaptive immunity, connecting innate and adaptive responses. Complement inhibitor compounds and compositions of the present disclosure may be used to modulate complement in the treatment and/or prevention of autoimmune diseases. In some cases, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used according to the methods presented in Ballanti et al. Immunol Res (2013) 56:477-491, the contents of which are herein incorporated by reference in their entirety. In some embodiments, autoimmune indications include myasthenia gravis.

Therapeutic indications addressed with C5 inhibitory cyclic polypeptides (e.g., zilucoplan and/or active metabolites or variants thereof) and/or compositions of the present disclosure may include autoimmune indications. In some cases, C5 inhibitory cyclic polypeptides (e.g., zilucoplan and/or active metabolites or variants thereof) may be used according to the methods presented in Ballanti et al. Immunol Res (2013) 56:477-491, the contents of which are herein incorporated by reference in their entirety. In some embodiments, autoimmune indications include myasthenia gravis.

### Anti-phospholipid syndrome (APS) and catastrophic anti-phospholipid syndrome (CAPS)

Autoimmune indications may include anti-phospholipid syndrome (APS). APS is an autoimmune condition caused by anti-phospholipid antibodies that cause the blood to clot. APS may lead to recurrent venous or arterial thrombosis in organs, and complications in placental circulations causing pregnancy-related complications such as miscarriage, still birth, preeclampsia, premature birth and/or other complications. Catastrophic anti-phospholipid syndrome (CAPS) is an extreme and acute version of a similar condition leading to occlusion of veins in several organs simultaneously. Studies suggest that complement activation may contribute to APS-related complications including pregnancy-related complications, thrombotic (clotting) complications, and vascular complications. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of APS and/or APS-related complications. In some embodiments, complement inhibitor compounds described herein and/or compositions thereof may be used to prevent and/or treat APS by complement activation control. In some cases, complement inhibitor compounds described herein and/or compositions thereof may be used to treat APS and/or APS-related complications according to the methods taught by Salmon et al. Ann Rheum Dis 2002;61(Suppl II):ii46-ii50 and Mackworth-Young in Clin Exp Immunol 2004, 136:393-401, the contents of which are herein incorporated by reference in their entirety. In some embodiments, C5 inhibitory cyclic polypeptides (e.g., zilucoplan and/or active metabolites or variants thereof) and compositions of the present disclosure may be used to treat, prevent, or delay development of APS and/or APS-related complications. In some embodiments, C5 inhibitory cyclic polypeptides (e.g., zilucoplan and/or active metabolites or variants thereof) and compositions of the present disclosure may be used to prevent and/or treat APS by complement activation control. In some cases, C5 inhibitory cyclic polypeptides (e.g., zilucoplan and/or active metabolites or variants thereof) and compositions thereof may be used to treat APS and/or APS-related complications according to the methods taught by Salmon et al. Ann Rheum Dis 2002;61(Suppl II):ii46-ii50 and Mackworth-Young in Clin Exp Immunol 2004, 136:393-401, the contents of which are herein incorporated by reference in their entirety.

### Cold agglutinin disease

Autoimmune indications may include cold agglutinin disease (CAD), also referred to as cold agglutinin-mediated hemolysis. CAD is an autoimmune disease resulting from a high concentration of IgM antibodies interacting with red blood cells at low range body temperatures (Engelhardt et al. Blood, 2002, 100(5):1922-23). CAD may lead to conditions such as anemia, fatigue, dyspnea, hemoglobinuria and/or acrocyanosis. CAD is related to robust complement activation and studies have shown that CAD may be treated with complement inhibitor therapies. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of CAD. Such uses may treat CAD by inhibiting complement activity. In some cases, complement inhibitor compounds described herein and/or compositions thereof may be used to treat CAD according to the methods taught by Roth et al in Blood, 2009, 113:3885-86 or in International publication No. WO2012/139081, the contents of each of which are herein incorporated by reference in their entirety. In some embodiments, C5 inhibitory cyclic polypeptides (e.g., zilucoplan and/or active metabolites or variants thereof) and compositions of the present disclosure may be used to treat, prevent, or delay development of CAD. Such uses may treat CAD by inhibiting complement activity. In some cases, C5 inhibitory cyclic polypeptides (e.g., zilucoplan and/or active metabolites or variants thereof) and compositions may be used to treat CAD according to the methods taught by Roth et al in Blood, 2009, 113:3885-86 or in International publication No. WO2012/139081, the contents of each of which are herein incorporated by reference in their entirety.

### Dermatological diseases

Autoimmune indications may include dermatological disease. Skin has a role in a spectrum of immunological reactions and are associated with abnormal or overactivated complement protein functions. Autoimmune mechanisms with autoantibodies and cytotoxic functions of the complement affect epidermal or vascular cells causing tissue damage and skin inflammation (Palenius and Meri, Front Med (Lausanne). 2015; 2: 3). Dermatological diseases associated with autoimmune and complement abnormality include, but are not limited to, hereditary and acquired angioedema, autoimmune urticarial (hives), systemic lupus erythematosus, vasculitis syndromes and urticarial vasculitis, bullous skin diseases (e.g. pemphigus, bullous pemphigioid, mucous membrane pemphigoid, epidermolysis bullosa acquisita, dermatitis herpetiformis, pemphigoides festationis), and partial lipodustrophy. In some cases, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat autoimmune dermatological diseases according to the methods taught by Palenius and Meri, Front Med (Lausanne). 2015; 2: 3, the contents of which are herein incorporated by reference in their entirety. In some embodiments, complement inhibitor compounds described herein and/or compositions thereof may be used to treat, prevent, or delay development of dermatological diseases. In some cases, C5 inhibitory cyclic polypeptides (e.g., zilucoplan and/or active metabolites or variants thereof) and compositions may be used to treat autoimmune dermatological diseases according to the methods taught by Palenius and Meri, Front Med (Lausanne). 2015; 2: 3, the contents of which are herein incorporated by reference in their entirety. In some embodiments, C5 inhibitory cyclic polypeptides (e.g., zilucoplan and/or active metabolites or variants thereof) and compositions of the present disclosure may be used to treat, prevent, or delay development of dermatological diseases.

### Pulmonary indications

Therapeutic indications addressed with compounds and/or compositions of the present disclosure may include pulmonary indications. As used herein, the term "pulmonary indication" refers to any therapeutic indication related to the lungs and/or related airways. Pulmonary indications may include complement-related indications. Pulmonary indications may include, but are not limited to, asthma, pulmonary fibrosis, chronic obstructive pulmonary disease (COPD), and acute respiratory distress syndrome. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of pulmonary indications. In some embodiments, C5 inhibitory cyclic polypeptides (e.g., zilucoplan and/or active metabolites or variants thereof) and compositions of the present disclosure may be used to treat, prevent, or delay development of pulmonary indications.

### Chronic obstructive pulmonary disease (COPD)

Pulmonary indications may include chronic obstructive pulmonary disease (COPD). COPD refers to a class of disorders related to progressive lung dysfunction. They are most often characterized by breathlessness. Complement dysfunction has been indicated as a contributor to some pulmonary indications related to COPD (Pandya, P.H. et al. 2013. Translational Review. 51(4): 467-73, the contents of which are herein incorporated by reference in their entirety). In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of COPD. In some embodiments, C5 inhibitory cyclic polypeptides (e.g., zilucoplan and/or active metabolites or variants thereof) and compositions of the present disclosure may be used to treat, prevent, or delay development of COPD.

### Cardiovascular indications

Therapeutic indications addressed with compounds and/or compositions of the present disclosure may include cardiovascular indications. As used herein, the term "cardiovascular indication" refers to any therapeutic indication relating to the heart and/or vasculature. Cardiovascular indications may include complement-related indications. Cardiovascular indications may include, but are not limited to, atherosclerosis, myocardial infarction, stroke, vasculitis, trauma and conditions arising from cardiovascular intervention (including, but not limited to cardiac bypass surgery, arterial grafting and angioplasty). In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of cardiovascular indications. In some embodiments, C5 inhibitory cyclic polypeptides (e.g., zilucoplan and/or active metabolites or variants thereof) and compositions of the present disclosure may be used to treat, prevent, or delay development of cardiovascular indications.

Vascular indications are cardiovascular indications related to blood vessels (e.g., arteries, veins, and capillaries). Such indications may affect blood circulation, blood pressure, blood flow, organ function, and/or other bodily functions. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of vascular indications. In some embodiments, C5 inhibitory cyclic polypeptides (e.g., zilucoplan and/or active metabolites or variants thereof) and compositions of the present disclosure may be used to treat, prevent, or delay development of vascular indications.

### Coagulation

In some embodiments, cardiovascular indications include therapeutic indications associated with coagulation, the coagulation cascade, and/or coagulation cascade components. Historically, the complement activation pathway was viewed separately from the coagulation cascade; however, interplay between these two systems has more recently been appreciated. Coagulation and complement are coordinately activated in an overlapping spatiotemporal manner in response to common pathophysiologic stimuli to maintain homeostasis. Disease may emerge with unchecked activation of the innate immune and coagulation responses. Examples include, for example, atherosclerosis, stroke, coronary heart disease, diabetes, ischemia-reperfusion injury, trauma, paroxysmal nocturnal hemoglobinuria, age-related macular degeneration, and atypical hemolytic-uremic syndrome.

Several molecular links between complement and coagulation are currently appreciated. For example, thrombin was found to promote complement activation by cleaving C5 (Huber-Lang, et al., 2006. Nature Med. 12(6):682-687; the contents of which are herein incorporated by reference in their entirety). While thrombin is capable of cleaving C5 at R751 (yielding C5a and C5b), it more efficiently cleaves C5 at a highly conserved R947 site, generating C5_{T} and C5b_{T} intermediates. C5b_{T} interacts with other complement proteins to form the C5b_{T}-9 membrane attack complex with significantly more lytic activity than with C5b-9 (Krisinger, et al., (2014). Blood. 120(8):1717-1725).

Complement may be activated by additional components of the coagulation and/or inflammation cascades. For example, other serine proteases with slightly different substrate specificity may act in a similar way. Huber-Lang et al. (2006) showed that thrombin not only cleaved C5 but also generated C3a *in vitro* when incubated with native C3 (Huber-Lang, et al., 2006. Nature Med. 12(6):682-687). Similarly, other components of the coagulation pathway, such as FXa, FXIa and plasmin, have been found to cleave both C5 and C3.

Specifically, in a mechanism similar to the one observed via thrombin activation, it has been observed that plasmin, FXa, FIXa and FXIa are able to cleave C5 to generate C5a and C5b [Amara, et al., (2010). J. Immunol. 185:5628-5636; Amara, et al., (2008) Current Topics in Complement II, J.D. Lambris (ed.), pp. 71-79]. The anaphylatoxins produced were found to be biologically active as shown by a dose-dependent chemotactic response of neutrophils and HMC-1 cells, respectively. Plasmin-induced cleavage activity could be dose-dependently blocked by the serine protease inhibitor aprotinin and leupeptine. These findings suggest that various serine proteases belonging to the coagulation system are able to activate the complement cascade independently of the established pathways. Moreover, functional C5a and C3a are generated (as detected by immunoblotting and ELISA), both of which are known to be crucially involved in the inflammatory response.

In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat cardiovascular indications related to coagulation, the coagulation cascade, and/or coagulation cascade components. The coagulation cascade components may include, but are not limited to, tissue factor, thrombin, FXa, FIXa, FXIa, plasmin, or other coagulation proteases. Compounds described herein and/or compositions thereof may be used to treat complement activity and/or coagulation (e.g., thrombosis) associated with such cardiovascular indications. In some embodiments, C5 inhibitory cyclic polypeptides (e.g., zilucoplan and/or active metabolites or variants thereof) and compositions of the present disclosure may be used to treat cardiovascular indications related to coagulation, the coagulation cascade, and/or coagulation cascade components. The coagulation cascade components may include, but are not limited to, tissue factor, thrombin, FXa, FIXa, FXIa, plasmin, or other coagulation proteases. C5 inhibitory cyclic polypeptides (e.g., zilucoplan and/or active metabolites or variants thereof) and/or compositions of the present disclosure may be used to treat complement activity and/or coagulation (e.g., thrombosis) associated with such cardiovascular indications.

### Thrombotic microangiopathy (TMA)

Vascular indications may include thrombotic microangiopathy (TMA) and associated diseases. Microangiopathies affect small blood vessels (capillaries) of the body causing capillary walls to become thick, weak, and prone to bleeding and slow blood circulation. TMAs tend to lead to the development of vascular thrombi, endothelial cell damage, thrombocytopenia, and hemolysis. Organs such as the brain, kidney, muscles, gastrointestinal system, skin, and lungs may be affected. TMAs may arise from medical operations and/or conditions that include, but are not limited to, hematopoietic stem cell transplantation (HSCT), renal disorders, diabetes and/or other conditions. TMAs may be caused by underlying complement system dysfunction, as described by Meri et al. in European Journal of Internal Medicine, 2013, 24: 496-502, the contents of which are herein incorporated by reference in their entirety. Generally, TMAs may result from increased levels of certain complement components leading to thrombosis. In some cases, this may be caused by mutations in complement proteins or related enzymes. Resulting complement dysfunction may lead to complement targeting of endothelial cells and platelets leading to increased thrombosis. In some embodiments, TMAs may be prevented and/or treated with complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof. In some cases, methods of treating TMAs with complement inhibitor compounds described herein and/or compositions thereof may be carried out according to those described in US publication Nos. US2012/0225056 or US2013/0246083, the contents of each of which are herein incorporated by reference in their entirety. In some embodiments, TMAs may be prevented and/or treated with C5 inhibitory cyclic polypeptides (e.g., zilucoplan and/or active metabolites or variants thereof) and compositions of the present disclosure. In some cases, methods of treating TMAs with C5 inhibitory cyclic polypeptides (e.g., zilucoplan and/or active metabolites or variants thereof) and compositions thereof may be carried out according to those described in US publication Nos. US2012/0225056 or US2013/0246083, the contents of each of which are herein incorporated by reference in their entirety.

### Disseminated intravascular coagulation (DIC)

Vascular indications may include disseminated intravascular coagulation (DIC). DIC is a pathological condition where the clotting cascade in blood is widely activated and results in formation of blood clots especially in the capillaries. DIC may lead to an obstructed blood flow of tissues and may eventually damage organs. Additionally, DIC affects the normal process of blood clotting that may lead to severe bleeding. Complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent or reduce the severity of DIC by modulating complement activity. In some cases, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used according to any of the methods of DIC treatment taught in US Patent No. 8,652,477, the contents of which are herein incorporated by reference in their entirety. C5 inhibitory cyclic polypeptides (e.g., zilucoplan and/or active metabolites or variants thereof) and compositions of the present disclosure may be used to treat, prevent or reduce the severity of DIC by modulating complement activity. In some cases, C5 inhibitory cyclic polypeptides (e.g., zilucoplan and/or active metabolites or variants thereof) and compositions may be used according to any of the methods of DIC treatment taught in US Patent No. 8,652,477, the contents of which are herein incorporated by reference in their entirety.

### vasculitis

Vascular indications may include vasculitis. Generally, vasculitis is a disorder related to inflammation of blood vessels, including veins and arteries, characterized by white blood cells attacking tissues and causing swelling of the blood vessels. Vasculitis may be associated with an infection, such as in Rocky Mountain spotted fever, or autoimmunity. An example of autoimmunity associated vasculitis is Anti-Neutrophil Cytoplasmic Autoantibody (ANCA) vasculitis. ANCA vasculitis is caused by abnormal antibodies attacking the body's own cells and tissues. ANCAs attack the cytoplasm of certain white blood cells and neutrophils, causing them to attack the walls of the vessels in certain organs and tissues of the body. ANCA vasculitis may affect skin, lungs, eyes and/or kidney. Studies suggest that ANCA disease activates an alternative complement pathway and generates certain complement components that create an inflammation amplification loop resulting in a vascular injury (Jennette et al. 2013, Semin Nephrol. 33(6): 557-64, the contents of which are herein incorporated by reference in their entirety). In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to prevent and/or treat vasculitis. In some cases, complement inhibitor compounds described herein and/or compositions thereof may be used to prevent and/or treat ANCA vasculitis by inhibiting complement activation. In some embodiments, C5 inhibitory cyclic polypeptides (e.g., zilucoplan and/or active metabolites or variants thereof) and compositions of the present disclosure may be used to prevent and/or treat vasculitis. In some cases, C5 inhibitory cyclic polypeptides (e.g., zilucoplan and/or active metabolites or variants thereof) and compositions may be used to prevent and/or treat ANCA vasculitis by inhibiting complement activation.

### Neurological indications

Therapeutic indications addressed with compounds (e.g., C5 inhibitors, C5 inhibitor polypeptides, C5 inhibitory cyclic polypeptides, zilucoplan, and/or active metabolites or variants thereof) described herein and/or compositions thereof may include neurological indications. As used herein, the term "neurological indication" refers to any therapeutic indication relating to the nervous system. Neurological indications may include complement-related indications. Neurological indications may include neurodegeneration. Neurodegeneration generally relates to a loss of structure or function of neurons, including death of neurons. In some embodiments, complement inhibitor compounds described herein and/or compositions thereof may be used to treat, prevent, or delay development of neurological indications, including, but not limited to neurodegenerative diseases and related disorders. In some embodiments, C5 inhibitory cyclic polypeptides (e.g., zilucoplan and/or active metabolites or variants thereof) and compositions of the present disclosure may be used to treat, prevent, or delay development of neurological indications, including, but not limited to neurodegenerative diseases and related disorders. Treatment may include inhibiting the effect of complement activity on neuronal cells using compounds and compositions of the present disclosure. Neurodegenerative related disorders include, but are not limited to, Amyelotrophic Lateral Sclerosis (ALS), Multiple Sclerosis (MS), Parkinson's disease, Alzheimer's disease, and Lewy body dementia. In some embodiments, complement-related neurological indications include myasthenia gravis.

### Amyotrophic lateral sclerosis (ALS)

Neurological indications may include ALS. ALS is a fatal motor neuron disease characterized by the degeneration of spinal cord neurons, brainstems and motor cortex. ALS causes loss of muscle strength leading eventually to a respiratory failure. Complement dysfunction may contribute to ALS, and therefore ALS may be prevented, treated and/or the symptoms may be reduced by therapy with complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof targeting complement activity. In some embodiments, complement inhibitor compounds described herein and/or compositions thereof may be used to treat, prevent, or delay development of ALS and/or promote nerve regeneration. In some cases, complement inhibitor compounds described herein and/or compositions thereof may be used as complement inhibitors according to any of the methods taught in US publication No. US2014/0234275 or US2010/0143344, the contents of each of which are herein incorporated by reference in their entirety. In some embodiments, C5 inhibitory cyclic polypeptides (e.g., zilucoplan and/or active metabolites or variants thereof) and compositions of the present disclosure may be used to treat, prevent, or delay development of ALS and/or promote nerve regeneration. In some cases, C5 inhibitory cyclic polypeptides (e.g., zilucoplan and/or active metabolites or variants thereof) and compositions may be used as complement inhibitors according to any of the methods taught in US publication No. US2014/0234275 or US2010/0143344, the contents of each of which are herein incorporated by reference in their entirety.

### Alzheimer's disease

Neurological indications may include Alzheimer's disease. Alzheimer's disease is a chronic neurodegenerative disease with symptoms that may include disorientation, memory loss, mood swings, behavioral problems and eventually loss of bodily functions. Alzheimer's disease is thought to be caused by extracellular brain deposits of amyloid that are associated with inflammation-related proteins such as complement proteins (Sjoberg et al. 2009. Trends in Immunology. 30(2): 83-90, the contents of which are herein incorporated by reference in their entirety). In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of Alzheimer's disease by controlling complement activity. In some cases, complement inhibitor compounds described herein and/or compositions thereof may be used according to any of the Alzheimer's treatment methods taught in US publication No. US2014/0234275, the contents of which are herein incorporated by reference in their entirety. In some embodiments, C5 inhibitory cyclic polypeptides (e.g., zilucoplan and/or active metabolites or variants thereof) and compositions of the present disclosure may be used to treat, prevent, or delay development of Alzheimer's disease by controlling complement activity. In some cases, C5 inhibitory cyclic polypeptides (e.g., zilucoplan and/or active metabolites or variants thereof) and compositions thereof may be used according to any of the Alzheimer's treatment methods taught in US publication No. US2014/0234275, the contents of which are herein incorporated by reference in their entirety.

### Multiple sclerosis and neuromyelitis optica

Neurological indications may include multiple sclerosis (MS) or neuromyelitis optica (NMO). MS is an inflammatory condition affecting the central nervous system as the immune system launches an attack against the body's own tissues, and in particular against nerve-insulating myelin. The condition may be triggered by an unknown environmental agent, such as a virus. MS is progressive and eventually results in disruption of the communication between the brain and other parts of the body. Typical early symptoms include blurred vision, partial blindness, muscle weakness, difficulties in coordination and balance, impaired movement, pain and speech impediments. NMO (also known as Devic's disease) is an inflammatory demyelinating disease affecting the optic nerves and spinal cord as the immune system attacks the astrocytes. NMO is sometimes considered as a variant of MS. Typical symptoms of NMO include muscle weakness of the legs or paralysis, loss of senses (e.g. blindness) and dysfunctions of the bladder and bowel.

MS and NMO have been associated with complement component regulation e.g. by pathological and animal model studies (Ingram et al., Clin Exp Immunol. 2009 Feb; 155(2): 128-139). In the central nervous system glial cells and neurons produce the majority of complement proteins and the expression is increased in response to inflammation. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of MS or NMO. Treatment methods may include any of those taught by Ingram et al., Clin Exp Immunol. 2009 Feb; 155(2): 128-139, the contents of which are herein incorporated by reference in their entirety. In some embodiments, C5 inhibitory cyclic polypeptides (e.g., zilucoplan and/or active metabolites or variants thereof) and compositions of the present disclosure may be used to treat, prevent, or delay development of MS or NMO. Treatment methods may include any of those taught by Ingram et al., Clin Exp Immunol. 2009 Feb; 155(2): 128-139, the contents of which are herein incorporated by reference in their entirety.

### Myasthenia gravis

Neurological indications may include myasthenia gravis. Myasthenia gravis (MG) is a rare complement-mediated autoimmune disease characterized by the production of autoantibodies targeting proteins that are critical for the normal transmission of chemical or neurotransmitter signals from nerves to muscles, e.g., acetylcholine receptor (AChR) proteins. The presence of AChR autoantibodies in patient samples can be used as an indicator of disease. As used herein, the term "MG" embraces any form of MG. While about 15% of patients have symptoms that are confined to ocular muscles, the majority of patients experience generalized myasthenia gravis. As used herein, the term "generalized myasthenia gravis" or "gMG" refers to MG that affects multiple muscle groups throughout the body. Although the prognosis of MG is generally benign, 10% to 15% of patients have refractory MG. As used herein, the term "refractory MG" or "rMG" refers to MG where disease control either cannot be achieved with current therapies, or results in severe side effects of immunosuppressive therapy. This severe form of MG affects approximately 9,000 individuals in the United States.

Patients with MG present with muscle weakness that characteristically becomes more severe with repeated use and recovers with rest. Muscle weakness can be localized to specific muscles, such as those responsible for eye movements, but often progresses to more diffuse muscle weakness. MG may even become life-threatening when muscle weakness involves the diaphragm and the other chest wall muscles responsible for breathing. This is the most feared complication of MG, known as myasthenic crisis or MG crisis, and requires hospitalization, intubation, and mechanical ventilation. Approximately 15% to 20% of patients with gMG experience a myasthenic crisis within two years of diagnosis.

The most common target of autoantibodies in MG is the acetylcholine receptor, or AChR, located at the neuromuscular junction, the point at which a motor neuron transmits signals to a skeletal muscle fiber. Current therapies for gMG focus on either augmenting the AChR signal or nonspecifically suppressing the autoimmune response. First-line therapy for symptomatic gMG is treatment with acetylcholinesterase inhibitors such as pyridostigmine, which is the only approved therapy for MG. Although sometimes adequate for control of mild ocular symptoms, pyridostigmine monotherapy is usually insufficient for the treatment of generalized weakness, and dosing of this therapy may be limited by cholinergic side effects. Therefore, in patients who remain symptomatic despite pyridostigmine therapy, corticosteroids with or without systemic immunosuppressives are indicated (Sanders DB, et al. 2016. Neurology. 87(4):419-25). Immunosuppressives used in gMG include azathioprine, cyclosporine, mycophenolate mofetil, methotrexate, tacrolimus, cyclophosphamide, and rituximab. To date, efficacy data for these agents are sparse and no steroidal or immunosuppressive therapy has been approved for the treatment of gMG. Moreover, all of these agents are associated with well-documented long-term toxicities. Surgical removal of the thymus may be recommended in patients with nonthymomatous gMG and moderate to severe symptoms in an effort to reduce the production of AChR autoantibodies (Wolfe GI, et al. 2016. N Engl J Med. 375(6):511-22). Intravenous (IV) immunoglobulin and plasma exchange are usually restricted to short-term use in patients with myasthenic crisis or life-threatening signs such as respiratory insufficiency or dysphagia (Sanders et al., 2016).

There is substantial evidence that supports the role of terminal complement cascade in the pathogenesis of AChR autoantibody-positive gMG. Results from animal models of experimental autoimmune MG have demonstrated that autoantibody immune complex formation at the neuromuscular junction triggers activation of the classical complement pathway, resulting in local activation of C3 and deposition of the membrane attack complex (MAC) at the neuromuscular junction, resulting in loss of signal transduction and eventual muscle weakness (Kusner LL, et al., 2012. Ann N Y Acad Sci. 1274(1):127-32).

Binding of anti-AChR autoantibodies to the muscle endplate results in activation of the classical complement cascade and deposition of MAC on the post-synaptic muscle fiber leading to local damage to the muscle membrane, and reduced responsiveness of the muscle to stimulation by the neuron.

In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of MG (e.g., gMG and/or rMG). Inhibition of complement activity may be used to block complement-mediated damage resulting from MG (e.g., gMG and/or rMG). In some embodiments, C5 inhibitory cyclic polypeptides (e.g., zilucoplan and/or active metabolites or variants thereof) and compositions of the present disclosure may be used to treat, prevent, or delay development of MG (e.g., gMG and/or rMG). Inhibition of complement activity may be used to block complement-mediated damage resulting from MG (e.g., gMG and/or rMG).

### Kidney-related indications

Therapeutic indications addressed with compounds (e.g., C5 inhibitors, C5 inhibitor polypeptides, C5 inhibitory cyclic polypeptides, zilucoplan, and/or active metabolites or variants thereof) described herein and/or compositions thereof may include kidney-related indications. As used herein, the term "kidney-related indication" refers to any therapeutic indication involving kidneys. Kidney-related indications may include complement-related indications. Kidneys are organs responsible for removing metabolic waste products from the blood stream. Kidneys regulate blood pressure, the urinary system, and homeostatic functions and are therefore essential for a variety of bodily functions. Kidneys may be more seriously affected by inflammation (as compared to other organs) due to unique structural features and exposure to blood. Kidneys also produce their own complement proteins which may be activated upon infection, kidney disease, and renal transplantations. In some embodiments, complement inhibitor compounds described herein and/or compositions thereof may be used to treat, prevent, or delay development of kidney-related indications, in some cases by inhibiting complement activity. In some cases, complement inhibitor compounds described herein and/or compositions thereof may be used to treat kidney-related indications according to the methods taught by Quigg, J Immunol 2003; 171:3319-24, the contents of which are herein incorporated by reference in their entirety. In some embodiments, C5 inhibitory cyclic polypeptides (e.g., zilucoplan and/or active metabolites or variants thereof) and compositions of the present disclosure may be used to treat, prevent, or delay development of kidney-related indications, in some cases by inhibiting complement activity. In some cases, C5 inhibitory cyclic polypeptides (e.g., zilucoplan and/or active metabolites or variants thereof) and compositions may be used to treat kidney-related indications according to the methods taught by Quigg, J Immunol 2003; 171:3319-24, the contents of which are herein incorporated by reference in their entirety.

### Atypical hemolytic uremic syndrome (aHUS)

Kidney-related indications may include atypical hemolytic uremic syndrome (aHUS). aHUS belongs to the spectrum of thrombotic microangiopathies. aHUS is a condition causing abnormal blood clots formation in small blood vessels of the kidneys. The condition is commonly characterized by hemolytic anemia, thrombocytopenia and kidney failure, and leads to end-stage renal disease (ESRD) in about half of all cases. aHUS has been associated with abnormalities of the alternative pathway of the complement system and may be caused by a genetic mutation in one of the genes that lead to increased activation of the alternative pathway. (Verhave et al., Nephrol Dial Transplant. 2014;29 Suppl 4:iv131-41 and International Publication WO 2016/138520). aHUS may be treated by inhibitors that control the alternative pathway of complement activation, including C5 activation. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent or delay development of aHUS. In some embodiments, C5 inhibitory cyclic polypeptides (e.g., zilucoplan and/or active metabolites or variants thereof) and compositions of the present disclosure may be used to treat, prevent or delay development of aHUS. Methods and compositions for preventing and/or treating aHUS by complement inhibition may include any of those taught by Verhave et al. in Nephrol Dial Transplant. 2014;29 Suppl 4:iv131-41 or International Publication WO 2016/138520, the contents of each of which are herein incorporated by reference in their entirety.

### Lupus Nephritis

Kidney-related indications may include lupus nephritis. Lupus nephritis is a kidney inflammation caused by an autoimmune disease called systemic lupus erythematosus (SLE). Symptoms of lupus nephritis include high blood pressure; foamy urine; swelling of the legs, the feet, the hands, or the face; joint pain; muscle pain; fever; and rash. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of lupus nephritis, in some cases through complement activity inhibition. In some embodiments, C5 inhibitory cyclic polypeptides (e.g., zilucoplan and/or active metabolites or variants thereof) and compositions of the present disclosure may be used to treat, prevent, or delay development of lupus nephritis, in some cases through complement activity inhibition. Related methods may include any of those taught in US publication No. US2013/0345257 or United States Patent No. 8,377,437, the contents of each of which are herein incorporated by reference in their entirety.

### Membranous glomerulonephritis (MGN)

Kidney-related indications may include membranous glomerulonephritis (MGN). MGN is a disorder of the kidney that may lead to inflammation and structural changes. MGN is caused by antibodies binding to a soluble antigen in kidney capillaries (glomerulus). MGN may affect kidney functions, such as filtering fluids and may lead to kidney failure. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of MGN, including by inhibiting complement activity. In some embodiments, C5 inhibitory cyclic polypeptides (e.g., zilucoplan and/or active metabolites or variants thereof) and compositions of the present disclosure may be used to treat, prevent, or delay development of MGN, including by inhibiting complement activity. Related treatment methods may include any of those taught in U.S. publication No. US2010/0015139 or in International publication No. WO2000/021559, the contents of each of which are herein incorporated by reference in their entirety.

### Hemodialysis complications

Kidney-related indications may include hemodialysis complications. Hemodialysis is a medical procedure used to maintain kidney function in subjects with kidney failure. In hemodialysis, the removal of waste products such as creatinine, urea, and free water from blood is performed externally. A common complication of hemodialysis treatment is chronic inflammation caused by contact between blood and the dialysis membrane. Another common complication is thrombosis referring to a formation of blood clots that obstructs the blood circulation. Studies have suggested that these complications are related to complement activation. Hemodialysis may be combined with complement inhibitor therapy to provide means of controlling inflammatory responses and pathologies and/or preventing or treating thrombosis in subjects going through hemodialysis due to kidney failure. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of hemodialysi**s** complications, including by inhibiting complement activation. In some embodiments, C5 inhibitory cyclic polypeptides (e.g., zilucoplan and/or active metabolites or variants thereof) and compositions of the present disclosure may be used to treat, prevent, or delay development of hemodialysis complications, including by inhibiting complement activation. Related methods for treatment of hemodialysis complications may include any of those taught by DeAngelis et al in Immunobiology, 2012, 217(11): 1097-1105 or by Kourtzelis et al. Blood, 2010, 116(4):631-639, the contents of each of which are herein incorporated by reference in their entirety.

### IgA Nephropathy

Kidney-related indications may include IgA nephropathy. IgA nephropathy is the most common cause of glomerulonephritis, affecting 25 in every one million per year. The disease is characterized by mesangial deposits of IgA and complement components in the glomeruli. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of IgA nephropathy by inhibiting the activation of certain complement components. Compounds described herein and/or compositions thereof may be used according to methods of preventing and/or treating IgA nephropathy by complement inhibition taught by Maillard N et al., in J of Am Soc Neph (2015) 26(7):1503-1512, the contents of each of which are herein incorporated by reference in their entirety. In some embodiments, C5 inhibitory cyclic polypeptides (e.g., zilucoplan and/or active metabolites or variants thereof) and compositions of the present disclosure may be used to treat, prevent, or delay development of IgA nephropathy by inhibiting the activation of certain complement components. C5 inhibitory cyclic polypeptides (e.g., zilucoplan and/or active metabolites or variants thereof) and compositions of the disclosure may be used according to methods of preventing and/or treating IgA nephropathy by complement inhibition taught by Maillard N et al., in J of Am Soc Neph (2015) 26(7):1503-1512, the contents of each of which are herein incorporated by reference in their entirety.

### Dense deposit disease/Membranoproliferative Glomerulonephritis type II/C3

### Glomerulopathy

Kidney-related indications may include dense deposit disease, membranoproliferative glomerulonephritis type II, and C3 glomerulopathy. Dense deposit disease (DDD) is a complement-related indication that involves kidney disorder. DDD may include proteinuria, hematuria, reduced amounts of urine, low levels of protein in the blood, and swelling in many areas of the body. DDD can be caused by mutations in the C3 and CFH genes; by both genetic risk factors and environmental triggers; or by the presence of autoantibodies blocking the activity of proteins needed for the body's immune response. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of DDD. In some embodiments, C5 inhibitory cyclic polypeptides (e.g., zilucoplan and/or active metabolites or variants thereof) and compositions of the present disclosure may be used to treat, prevent, or delay development of DDD. Such uses may include reducing and/or blocking complement alternative pathway activity. Such methods may prevent glomerular C3 deposition.

### Focal-segmental glomerulosclerosis

Kidney-related indications may include focal-segmental glomerulosclerosis. Focal-segmental glomerulosclerosis (FSGS) is a common cause of glomerular disease in children and adults and most commonly presents as severe nephrotic syndrome. Diagnosis of FSGS is made based on histopathological findings and exclusion of other diagnoses common in nephrotic syndrome. Many patients will have substantial deposition of IgM and C3 in sclerotic regions on biopsy. Additionally, biomarkers for complement activation (factor B fragments, C4a, soluble MAC) have been detected in plasma and urine from patients with FSGS, with levels of Ba and Bb correlating with disease severity (J. Thurman et al, PLOSone, 2015). In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of FSGS. In some embodiments, C5 inhibitory cyclic polypeptides (e.g., zilucoplan and/or active metabolites or variants thereof) and compositions of the present disclosure may be used to treat, prevent, or delay development of FSGS.

### Diabetes-related indications

Therapeutic indications addressed with compounds (e.g., C5 inhibitors, C5 inhibitor polypeptides, C5 inhibitory cyclic polypeptides, zilucoplan, and/or active metabolites or variants thereof) described herein and/or compositions thereof may include diabetes-related indications. As used herein, the term "diabetes-related indication" refers to any therapeutic indication resulting from or relating to elevated blood sugar. Diabetes-related indications may include complement-related indications. Diabetes-related indications may occur as a result of organ and/or tissue exposure to prolonged hyperglycemia. Prolonged hyperglycemia can result in glycation inactivation of the membrane-associated complement regulatory protein CD59, leaving certain cells and tissues susceptible to complement attack (P. Ghosh et al, 2015. Endocrine Reviews, 36 (3), 2015). Complement-mediated complications from diabetes may include, but are not limited to, diabetic neuropathy, diabetic nephropathy, diabetic cardiovascular disease, and complications resulting from gestational diabetes such as high or low birth weight and resulting complications. In some embodiments, complement inhibitor compounds described herein and/or compositions thereof may be used to treat, prevent, or delay development of diabetes-related indications. In some embodiments, C5 inhibitory cyclic polypeptides (e.g., zilucoplan and/or active metabolites or variants thereof) and compositions of the present disclosure may be used to treat, prevent, or delay development of diabetes-related indications. Such uses may include addressing diabetes-related indications through complement activity inhibition.

### Ocular indications

Therapeutic indications addressed with compounds (e.g., C5 inhibitors, C5 inhibitor polypeptides, C5 inhibitory cyclic polypeptides, zilucoplan, and/or active metabolites or variants thereof) described herein and/or compositions thereof may include ocular indications. As used herein, the term "ocular indication" refers to any therapeutic indication relating to the eye. Ocular indications may include complement-related indications. In a healthy eye the complement system is activated at a low level and is continuously regulated by membrane-bound and soluble intraocular proteins that protect against pathogens. Therefore, the activation of complement plays an important role in several complications related to the eye and controlling complement activation may be used to treat such diseases. In some embodiments, complement inhibitor compounds described herein and/or compositions thereof may be used to treat, prevent, or delay development of ocular indications, including by inhibiting complement activity. In some embodiments, C5 inhibitory cyclic polypeptides (e.g., zilucoplan and/or active metabolites or variants thereof) and compositions of the present disclosure may be used to treat, prevent, or delay development of ocular indications, including by inhibiting complement activity. Related treatment methods may include any of those taught by Jha et al. in Mol Immunol. 2007; 44(16): 3901-3908 or in US Patent No. 8,753,625, the contents of each of which are herein incorporated by reference in their entirety.

Ocular indications may include, but are not limited to, age-related macular degeneration, allergic and giant papillary conjunctivitis, Behcet's disease, choroidal inflammation, complications related to intraocular surgery, corneal transplant rejection, corneal ulcers, cytomegalovirus retinitis, dry eye syndrome, endophthalmitis, Fuch's disease, Glaucoma, immune complex vasculitis, inflammatory conjunctivitis, ischemic retinal disease, keratitis, macular edema, ocular parasitic infestation/migration, retinitis pigmentosa, scleritis, Stargardt disease, subretinal fibrosis, uveitis, vitreo-retinal inflammation, and Vogt-Koyanagi-Harada disease.

### Age-related macular degeneration (AMD)

Ocular indications may include age-related macular degeneration (AMD). AMD is a chronic ocular disease causing blurred central vision, blind spots in central vision, and/or eventual loss of central vision. Central vision affects ability to read, drive a vehicle and/or recognize faces. AMD is generally divided into two types, non-exudative (dry) and exudative (wet). Dry AMD refers to the deterioration of the macula which is the tissue in the center of the retina. Wet AMD refers to the failure of blood vessels under the retina leading to leaking of blood and fluid. Several human and animal studies have identified complement proteins that are related to AMD and novel therapeutic strategies included controlling complement activation pathways, as discussed by Jha et al. in Mol Immunol. 2007; 44(16): 3901-8. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of AMD by inhibiting ocular complement activation. Methods of the present disclosure involving the use of complement inhibitor compounds described herein and/or compositions thereof for prevention and/or treatment of AMD may include any of those taught in US publication Nos. US2011/0269807 or US2008/0269318, the contents of each of which are herein incorporated by reference in their entirety. In some embodiments, C5 inhibitory cyclic polypeptides (e.g., zilucoplan and/or active metabolites or variants thereof) and compositions of the present disclosure may be used to treat, prevent, or delay development of AMD by inhibiting ocular complement activation. Methods of the present disclosure involving the use of C5 inhibitory cyclic polypeptides (e.g., zilucoplan and/or active metabolites or variants thereof) and compositions thereof for prevention and/or treatment of AMD may include any of those taught in US publication Nos. US2011/0269807 or US2008/0269318, the contents of each of which are herein incorporated by reference in their entirety.

### Corneal disease

Ocular indications may include corneal disease. The complement system plays an important role in protection of the cornea from pathogenic particles and/or inflammatory antigens. The cornea is the outermost front part of the eye covering and protecting the iris, pupil and anterior chamber and is therefore exposed to external factors. Corneal diseases include, but are not limited to, keratoconus, keratitis, ocular herpes and/or other diseases. Corneal complications may cause pain, blurred vision, tearing, redness, light sensitivity and/or corneal scarring. The complement system is critical for corneal protection, but complement activation may cause damage to the corneal tissue after an infection is cleared as certain complement compounds are heavily expressed. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of corneal diseases by inhibiting ocular complement activation. In some embodiments, C5 inhibitory cyclic polypeptides (e.g., zilucoplan and/or active metabolites or variants thereof) and compositions of the present disclosure may be used to treat, prevent, or delay development of corneal diseases by inhibiting ocular complement activation. Methods of the present disclosure for modulating complement activity in the treatment of corneal disease may include any of those taught by Jha et al. in Mol Immunol. 2007; 44(16): 3901-8, the contents of which are herein incorporated by reference in their entirety.

### Autoimmune uveitis

Ocular indications may include autoimmune uveitis. Uvea is the pigmented area of the eye including the choroids, iris and ciliary body of the eye. Uveitis causes redness, blurred vision, pain, synechia and may eventually cause blindness. Studies have indicated that complement activation products are present in the eyes of patients with autoimmune uveitis and complement plays an important role in disease development. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of uveitis. In some embodiments, C5 inhibitory cyclic polypeptides (e.g., zilucoplan and/or active metabolites or variants thereof) and compositions of the present disclosure may be used to treat, prevent, or delay development of uveitis. Such treatments may be carried out according to any of the methods identified in Jha et al. in Mol Immunol. 2007. 44(16): 3901-8, the contents of which are herein incorporated by reference in their entirety.

### Diabetic retinopathy

Ocular indications may include diabetic retinopathy, which is a disease caused by changes in retinal blood vessels in diabetic patients. Retinopathy may cause blood vessel swelling and fluid leaking and/or growth of abnormal blood vessels. Diabetic retinopathy affects vision and may eventually lead to blindness. Studies have suggested that activation of complement has an important role in the development of diabetic retinopathy. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of diabetic retinopathy. Complement inhibitor compounds described herein and/or compositions thereof may be used according to methods of diabetic retinopathy treatment described in Jha et al. Mol Immunol. 2007; 44(16): 3901-8, the contents of which are herein incorporated by reference in their entirety. In some embodiments, C5 inhibitory cyclic polypeptides (e.g., zilucoplan and/or active metabolites or variants thereof) and compositions of the present disclosure may be used to treat, prevent, or delay development of diabetic retinopathy. C5 inhibitory cyclic polypeptides (e.g., zilucoplan and/or active metabolites or variants thereof) and compositions thereof may be used according to methods of diabetic retinopathy treatment described in Jha et al. Mol Immunol. 2007; 44(16): 3901-8, the contents of which are herein incorporated by reference in their entirety.

### Stargardt's disease

Ocular indications may include Stargardt's disease. Stargardt's disease, also called recessive Stargardt's macular degeneration is an inherited disease of the eye, with an age of onset within the first two decades of life. Complications from Stargardt's disease may include loss of vision (Radu et al.,J. Biol. Chem, 2011 286(21) 18593-18601). The disease results from a mutation in the *ABCA4* gene. The hallmark of the disease includes accumulation of lipofuscin. Studies have indicated that accumulating lipofuscin activates the complement cascade (Radu et al.,J. Biol. Chem, 2011 286(21) 18593-18601). In addition, studies (Tan et al, PNAS 2016; 113(31) 8789-8794) also show that the *ABCA4* gene mutation that affects organelle transport and results in lipofuscin accumulation, also results in downregulation of CD59 on the RPE cell surface making them susceptible to damage by complement activation. In some embodiments, complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used to treat, prevent, or delay development of Stargardt's disease, e.g., by inhibiting ocular complement activation. In some embodiments, C5 inhibitory cyclic polypeptides (e.g., zilucoplan and/or active metabolites or variants thereof) and compositions of the present disclosure may be used to treat, prevent, or delay development of Stargardt's disease, e.g., by inhibiting ocular complement activation.

### Pregnancy-related indications

Therapeutic indications addressed with compounds and/or compositions of the present disclosure may include pregnancy-related indications. As used herein, the term "pregnancy-related indication" refers to any therapeutic indication involving child birth and/or pregnancy. Pregnancy-related indications may include complement-related indications. Pregnancy-related indications may include pre-eclampsia and/or HELLP (abbreviation standing for syndrome features of 1) hemolysis, 2) elevated liver enzymes and 3) low platelet count) syndrome. Pre-eclampsia is a disorder of pregnancy with symptoms including elevated blood pressure, swelling, shortness of breath, kidney dysfunction, impaired liver function and/or low blood platelet count. Pre-eclampsia is typically diagnosed by a high urine protein level and high blood pressure. HELLP syndrome is a combination of hemolysis, elevated liver enzymes and low platelet conditions. Hemolysis is a disease involving rupturing of red blood cells leading to the release of hemoglobin from red blood cells. Elevated liver enzymes may indicate a pregnancy-induced liver condition. Low platelet levels lead to reduced clotting capability, causing danger of excessive bleeding. HELLP is associated with a pre-eclampsia and liver disorder. HELLP syndrome typically occurs during the later stages of pregnancy or after childbirth. It is typically diagnosed by blood tests indicating the presence of the three conditions it involves. Typically HELLP is treated by inducing delivery.

Studies suggest that complement activation occurs during HELLP syndrome and pre-eclampsia and that certain complement components are present at increased levels during HELLP and pre-eclampsia. Complement inhibitor compounds (e.g., C5 inhibitors, C5 inhibitor polypeptides, C5 inhibitory cyclic polypeptides, zilucoplan, and/or active metabolites or variants thereof) described herein and/or compositions thereof may be used as therapeutic agents to prevent and/or treat these and other pregnancy-related indications. Complement inhibitor compounds described herein and/or compositions thereof may be used according to methods of preventing and/or treating HELLP and pre-eclampsia taught by Heager et al. in Obstetrics & Gynecology, 1992, 79(1):19-26 or in International publication No. WO2014/078622, the contents of each of which are herein incorporated by reference in their entirety. C5 inhibitory cyclic polypeptides (e.g., zilucoplan and/or active metabolites or variants thereof) of the present disclosure may be used as therapeutic agents to prevent and/or treat these and other pregnancy-related indications. C5 inhibitory cyclic polypeptides (e.g., zilucoplan and/or active metabolites or variants thereof) and compositions thereof may be used according to methods of preventing and/or treating HELLP and pre-eclampsia taught by Heager et al. in Obstetrics & Gynecology, 1992, 79(1):19-26 or in International publication No. WO2014/078622, the contents of each of which are herein incorporated by reference in their entirety.

### Dosage and administration

In some embodiments, the present disclosure provides methods of administering active ingredients (e.g., therapeutic agents, C5 inhibitors, C5 inhibitor polypeptides, C5 inhibitory cyclic polypeptides, zilucoplan, and/or active metabolites or variants thereof) described herein and/or compositions thereof. Active ingredients may be administered using any of the sustained release formulations described herein. Sustained release formulations may be administered by subcutaneous injection. Sustained release formulations may be administered weekly or biweekly. Active ingredients may be released from sustained release formulations after administration. Active ingredient concentrations in subject tissues or fluids surrounding sites of sustained release formulation administration may exhibit a burst of less than 5%. Sustained release formulations may be administered at a dose sufficient to administer from about 2 mg/kg to about 20 mg/kg of active ingredient. Where the active ingredient is zilucoplan, sustained release formulations may be administered at doses sufficient to administer from about 100 mg to about 200 mg zilucoplan.

In some embodiments, the present disclosure provides methods of administering C5 inhibitory cyclic polypeptides (e.g., zilucoplan and/or active metabolites or variants thereof). The C5 inhibitory cyclic polypeptides may include zilucoplan and/or metabolites and/or variants thereof. C5 inhibitory cyclic polypeptides may be administered using any of the sustained release formulations described herein. Sustained release formulations may be administered by subcutaneous injection. Sustained release formulations may be administered weekly or biweekly. C5 inhibitory cyclic polypeptides may be released from sustained release formulations after administration. Concentrations of C5 inhibitory cyclic polypeptides in subject tissues or fluids surrounding sites of sustained release formulation administration may exhibit a burst of less than 5%. Sustained release formulations may be administered at a dose sufficient to administer from about 2 mg/kg to about 20 mg/kg of C5 inhibitory cyclic polypeptides (e.g., zilucoplan and/or active metabolites or variants thereof). Sustained release formulations may be administered at doses sufficient to administer from about 100 mg to about 200 mg zilucoplan.

In some embodiments, the present disclosure provides methods of reducing hemolysis in subjects by administering C5 inhibitors (e.g., C5 inhibitor polypeptides, C5 inhibitory cyclic polypeptides, zilucoplan, and/or active metabolites or variants thereof) described herein and/or compositions thereof. The C5 inhibitors may be administered as part of sustained release formulations described herein.

In some embodiments, the present disclosure provides methods of reducing hemolysis in subjects by administering C5 inhibitory cyclic polypeptides (e.g., zilucoplan and/or active metabolites or variants thereof) described herein. The C5 inhibitory cyclic polypeptides (e.g., zilucoplan and/or active metabolites or variants thereof) may be administered as part of sustained release formulations described herein.

C5 inhibitors (e.g., C5 inhibitor polypeptides, C5 inhibitory cyclic polypeptides, zilucoplan, and/or active metabolites or variants thereof) described herein and/or compositions thereof may be provided in a therapeutically effective amount. In some cases, a therapeutically effective amount of a C5 inhibitor may be achieved by administration of a dose of from about 0.1 mg to about 1 mg, from about 0.5 mg to about 5 mg, from about 1 mg to about 20 mg, from about 5 mg to about 50 mg, from about 10 mg to about 100 mg, from about 20 mg to about 200 mg, or at least 200 mg of one or more C5 inhibitors.

C5 inhibitory cyclic polypeptides (e.g., zilucoplan and/or active metabolites or variants thereof) may be provided in a therapeutically effective amount. In some cases, a therapeutically effective amount of C5 inhibitory cyclic polypeptides (e.g., zilucoplan and/or active metabolites or variants thereof) may be achieved by administration of a dose of from about 0.1 mg to about 1 mg, from about 0.5 mg to about 5 mg, from about 1 mg to about 20 mg, from about 5 mg to about 50 mg, from about 10 mg to about 100 mg, from about 20 mg to about 200 mg, or at least 200 mg of one or more C5 inhibitory cyclic polypeptide (e.g., zilucoplan and/or active metabolites or variants thereof).

In some embodiments, subjects may be administered a therapeutic amount of C5 inhibitors (e.g., C5 inhibitor polypeptides, C5 inhibitory cyclic polypeptides, zilucoplan, and/or active metabolites or variants thereof) based on the weight of such subjects. In some cases, C5 inhibitors are administered at a dose of from about 0.001 mg/kg to about 1.0 mg/kg, from about 0.01 mg/kg to about 2.0 mg/kg, from about 0.05 mg/kg to about 5.0 mg/kg, from about 0.03 mg/kg to about 3.0 mg/kg, from about 0.01 mg/kg to about 10 mg/kg, from about 0.1 mg/kg to about 2.0 mg/kg, from about 0.2 mg/kg to about 3.0 mg/kg, from about 0.4 mg/kg to about 4.0 mg/kg, from about 1.0 mg/kg to about 5.0 mg/kg, from about 2.0 mg/kg to about 4.0 mg/kg, from about 1.5 mg/kg to about 7.5 mg/kg, from about 5.0 mg/kg to about 15 mg/kg, from about 7.5 mg/kg to about 12.5 mg/kg, from about 10 mg/kg to about 20 mg/kg, from about 15 mg/kg to about 30 mg/kg, from about 20 mg/kg to about 40 mg/kg, from about 30 mg/kg to about 60 mg/kg, from about 40 mg/kg to about 80 mg/kg, from about 50 mg/kg to about 100 mg/kg, or at least 100 mg/kg. Such ranges may include ranges suitable for administration to human subjects. Dosage levels may be highly dependent on the nature of the condition; drug efficacy; the condition of the patient; the judgment of the practitioner; and the frequency and mode of administration. In some embodiments, zilucoplan and/or active metabolites or variants thereof may be administered at a dose of from about 0.01 mg/kg to about 10 mg/kg. In some cases, zilucoplan and/or active metabolites or variants thereof may be administered at a dose of from about 0.1 mg/kg to about 3 mg/kg.

In some embodiments, subjects may be administered a therapeutic amount of C5 inhibitory cyclic polypeptides (e.g., zilucoplan and/or active metabolites or variants thereof) based on the weight of such subjects. In some cases, C5 inhibitory cyclic polypeptides (e.g., zilucoplan and/or active metabolites or variants thereof) are administered at a dose of from about 0.001 mg/kg to about 1.0 mg/kg, from about 0.01 mg/kg to about 2.0 mg/kg, from about 0.05 mg/kg to about 5.0 mg/kg, from about 0.03 mg/kg to about 3.0 mg/kg, from about 0.01 mg/kg to about 10 mg/kg, from about 0.1 mg/kg to about 2.0 mg/kg, from about 0.2 mg/kg to about 3.0 mg/kg, from about 0.4 mg/kg to about 4.0 mg/kg, from about 1.0 mg/kg to about 5.0 mg/kg, from about 2.0 mg/kg to about 4.0 mg/kg, from about 1.5 mg/kg to about 7.5 mg/kg, from about 5.0 mg/kg to about 15 mg/kg, from about 7.5 mg/kg to about 12.5 mg/kg, from about 10 mg/kg to about 20 mg/kg, from about 15 mg/kg to about 30 mg/kg, from about 20 mg/kg to about 40 mg/kg, from about 30 mg/kg to about 60 mg/kg, from about 40 mg/kg to about 80 mg/kg, from about 50 mg/kg to about 100 mg/kg, or at least 100 mg/kg. Such ranges may include ranges suitable for administration to human subjects. Dosage levels may be highly dependent on the nature of the condition; drug efficacy; the condition of the patient; the judgment of the practitioner; and the frequency and mode of administration. In some embodiments, zilucoplan and/or active metabolites or variants thereof may be administered at a dose of from about 0.01 mg/kg to about 10 mg/kg. In some cases, zilucoplan and/or active metabolites or variants thereof may be administered at a dose of from about 0.1 mg/kg to about 3 mg/kg.

In some cases, C5 inhibitors (e.g., C5 inhibitor polypeptides, C5 inhibitory cyclic polypeptides, zilucoplan, and/or active metabolites or variants thereof) are provided at concentrations adjusted to achieve a desired level of the C5 inhibitor in a sample, biological system, or subject (e.g., plasma level in a subject). In some cases, desired concentrations of C5 inhibitors in a sample, biological system, or subject may include concentrations of from about 0.001 µM to about 0.01 µM, from about 0.005 µM to about 0.05 µM, from about 0.02 µM to about 0.2 µM, from about 0.03 µM to about 0.3 µM, from about 0.05 µM to about 0.5 µM, from about 0.01 µM to about 2.0 µM, from about 0.1 µM to about 50 µM, from about 0.1 µM to about 10 µM, from about 0.1 µM to about 5 µM, from about 0.2 µM to about 20 µM, from about 5 µM to about 100 µM, or from about 15 µM to about 200 µM. In some cases, desired concentrations of C5 inhibitors in subject plasma may be from about 0.1 µg/mL to about 1000 µg/mL. The desired concentration of C5 inhibitors in subject plasma may be from about 0.01 µg/mL to about 2 µg/mL, from about 0.02 µg/mL to about 4 µg/mL, from about 0.05 µg/mL to about 5 µg/mL, from about 0.1 µg/mL to about 1.0 µg/mL, from about 0.2 µg/mL to about 2.0 µg/mL, from about 0.5 µg/mL to about 5 µg/mL, from about 1 µg/mL to about 5 µg/mL, from about 2 µg/mL to about 10 µg/mL, from about 3 µg/mL to about 9 µg/mL, from about 5 µg/mL to about 20 µg/mL, from about 10 µg/mL to about 40 µg/mL, from about 30 µg/mL to about 60 µg/mL, from about 40 µg/mL to about 80 µg/mL, from about 50 µg/mL to about 100 µg/mL, from about 75 µg/mL to about 150 µg/mL, or at least 150 µg/mL. In other embodiments, C5 inhibitors (e.g., C5 inhibitor polypeptides, C5 inhibitory cyclic polypeptides, zilucoplan, and/or active metabolites or variants thereof) described herein and/or compositions thereof are administered at a dose sufficient to achieve a maximum C5 inhibitor serum concentration (Cₘₐₓ) of at least 0.1 µg/mL, at least 0.5 µg/mL, at least 1 µg/mL, at least 5 µg/mL, at least 10 µg/mL, at least 50 µg/mL, at least 100 µg/mL, or at least 1000 µg/mL.

In some cases, C5 inhibitory cyclic polypeptides (e.g., zilucoplan and/or active metabolites or variants thereof) are provided at concentrations adjusted to achieve a desired level of the C5 inhibitor in a sample, biological system, or subject (e.g., plasma level in a subject). In some cases, desired concentrations of C5 inhibitory cyclic polypeptides (e.g., zilucoplan and/or active metabolites or variants thereof) in a sample, biological system, or subject may include concentrations of from about 0.001 µM to about 0.01 µM, from about 0.005 µM to about 0.05 µM, from about 0.02 µM to about 0.2 µM, from about 0.03 µM to about 0.3 µM, from about 0.05 µM to about 0.5 µM, from about 0.01 µM to about 2.0 µM, from about 0.1 µM to about 50 µM, from about 0.1 µM to about 10 µM, from about 0.1 µM to about 5 µM, from about 0.2 µM to about 20 µM, from about 5 µM to about 100 µM, or from about 15 µM to about 200 µM. In some cases, desired concentrations of C5 inhibitory cyclic polypeptides (e.g., zilucoplan and/or active metabolites or variants thereof) in subject plasma may be from about 0.1 µg/mL to about 1000 µg/mL. The desired concentration of C5 inhibitory cyclic polypeptides (e.g., zilucoplan and/or active metabolites or variants thereof) in subject plasma may be from about 0.01 µg/mL to about 2 µg/mL, from about 0.02 µg/mL to about 4 µg/mL, from about 0.05 µg/mL to about 5 µg/mL, from about 0.1 µg/mL to about 1.0 µg/mL, from about 0.2 µg/mL to about 2.0 µg/mL, from about 0.5 µg/mL to about 5 µg/mL, from about 1 µg/mL to about 5 µg/mL, from about 2 µg/mL to about 10 µg/mL, from about 3 µg/mL to about 9 µg/mL, from about 5 µg/mL to about 20 µg/mL, from about 10 µg/mL to about 40 µg/mL, from about 30 µg/mL to about 60 µg/mL, from about 40 µg/mL to about 80 µg/mL, from about 50 µg/mL to about 100 µg/mL, from about 75 µg/mL to about 150 µg/mL, or at least 150 µg/mL. In other embodiments, C5 inhibitory cyclic polypeptides (e.g., zilucoplan and/or active metabolites or variants thereof) are administered at a dose sufficient to achieve a maximum serum concentration (Cₘₐₓ) of at least 0.1 µg/mL, at least 0.5 µg/mL, at least 1 µg/mL, at least 5 µg/mL, at least 10 µg/mL, at least 50 µg/mL, at least 100 µg/mL, or at least 1000 µg/mL.

In some embodiments, C5 inhibitors (e.g., C5 inhibitor polypeptides, C5 inhibitory cyclic polypeptides, zilucoplan, and/or active metabolites or variants thereof) are administered daily at a dose sufficient to deliver from about 0.1 mg/day to about 60 mg/day per kg weight of a subject. In some embodiments, C5 inhibitory cyclic polypeptides (e.g., zilucoplan and/or active metabolites or variants thereof) are administered daily at a dose sufficient to deliver from about 0.1 mg/day to about 60 mg/day per kg weight of a subject. In some cases, the Cₘₐₓ achieved with each dose is from about 0.1 µg/mL to about 1000 µg/mL. In such cases, the area under the curve (AUC) between doses may be from about 200 µg*hr/mL to about 10,000 µg*hr/mL.

According to some methods of the present disclosure, C5 inhibitors (e.g., C5 inhibitor polypeptides, C5 inhibitory cyclic polypeptides, zilucoplan, and/or active metabolites or variants thereof) are provided at concentrations needed to achieve a desired effect. In some cases, compounds and compositions of the disclosure are provided at an amount necessary to reduce a given reaction or process by half. The concentration needed to achieve such a reduction is referred to herein as the half maximal inhibitory concentration, or "IC₅₀." Alternatively, compounds and compositions of the disclosure may be provided at an amount necessary to increase a given reaction, activity or process by half. The concentration needed for such an increase is referred to herein as the half maximal effective concentration or "EC₅₀."

C5 inhibitors (e.g., C5 inhibitor polypeptides, C5 inhibitory cyclic polypeptides, zilucoplan, and/or active metabolites or variants thereof) may be present in amounts totaling 0.1-95% by weight of the total weight of the composition. In some cases, C5 inhibitors are provided by intravenous (IV) administration. In some cases, C5 inhibitors are provided by subcutaneous (SC) administration.

C5 inhibitory cyclic polypeptides (e.g., zilucoplan and/or active metabolites or variants thereof) may be present in amounts totaling 0.1-95% by weight of the total weight of the composition. In some cases, C5 inhibitory cyclic polypeptides (e.g., zilucoplan and/or active metabolites or variants thereof) are provided by IV administration. In some cases, C5 inhibitory cyclic polypeptides (e.g., zilucoplan and/or active metabolites or variants thereof) are provided by SC administration.

SC administration of C5 inhibitors (e.g., C5 inhibitor polypeptides, C5 inhibitory cyclic polypeptides, zilucoplan, and/or active metabolites or variants thereof) may, in some cases, provide advantages over IV administration. SC administration may allow patients to provide self-treatment. Such treatment may be advantageous in that patients could provide treatment to themselves in their own home, avoiding the need to travel to a provider or medical facility. Further, SC treatment may allow patients to avoid long-term complications associated with IV administration, such as infections, loss of venous access, local thrombosis, and hematomas. In some embodiments, SC treatment may increase patient compliance, patient satisfaction, quality of life, reduce treatment costs and/or drug requirements.

In some cases, daily SC administration provides steady-state concentrations of C5 inhibitors (e.g., C5 inhibitor polypeptides, C5 inhibitory cyclic polypeptides, zilucoplan, and/or active metabolites or variants thereof) that are reached within 1-3 doses, 2-3 doses, 3-5 doses, or 5-10 doses. In some cases, daily SC doses of from about 0.1 mg/kg to about 0.3 mg/kg may achieve sustained C5 inhibitor levels greater than or equal to 2.5 µg/mL and/or inhibition of complement activity of greater than 90%.

In some cases, daily SC administration provides steady-state concentrations for C5 inhibitory cyclic polypeptides (e.g., zilucoplan and/or active metabolites or variants thereof) that are reached within 1-3 doses, 2-3 doses, 3-5 doses, or 5-10 doses. In some cases, daily SC doses of from about 0.1 mg/kg to about 0.3 mg/kg may achieve sustained levels of C5 inhibitory cyclic polypeptides (e.g., zilucoplan and/or active metabolites or variants thereof) that are greater than or equal to 2.5 µg/mL and/or inhibition of complement activity of greater than 90%.

C5 inhibitors (e.g., C5 inhibitor polypeptides, C5 inhibitory cyclic polypeptides, zilucoplan, and/or active metabolites or variants thereof) may exhibit slow absorption kinetics (time to maximum observed concentration of greater than 4-8 hours) and high bioavailability (from about 75% to about 100%) after SC administration.

C5 inhibitory cyclic polypeptides (e.g., zilucoplan and/or active metabolites or variants thereof) may exhibit slow absorption kinetics (time to maximum observed concentration of greater than 4-8 hours) and high bioavailability (from about 75% to about 100%) after SC administration.

In some embodiments, dosage and/or administration are altered to modulate half-life (t_{1/2}) levels of C5 inhibitors (e.g., C5 inhibitor polypeptides, C5 inhibitory cyclic polypeptides, zilucoplan, and/or active metabolites or variants thereof) in a subject or in subject fluids (e.g., plasma). In some cases, t_{1/2} is at least 1 hour, at least 2 hrs, at least 4 hrs, at least 6 hrs, at least 8 hrs, at least 10 hrs, at least 12 hrs, at least 16 hrs, at least 20 hrs, at least 24 hrs, at least 36 hrs, at least 48 hrs, at least 60 hrs, at least 72 hrs, at least 96 hrs, at least 5 days, at least 6 days, at least 7 days, at least 8 days, at least 9 days, at least 10 days, at least 11 days, at least 12 days, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, or at least 16 weeks.

In some embodiments, dosage and/or administration are altered to modulate the half-life (t_{1/2}) of C5 inhibitory cyclic polypeptide (e.g., zilucoplan and/or active metabolites or variants thereof) levels in a subject or in subject fluids (e.g., plasma). In some cases, t_{1/2} is at least 1 hour, at least 2 hrs, at least 4 hrs, at least 6 hrs, at least 8 hrs, at least 10 hrs, at least 12 hrs, at least 16 hrs, at least 20 hrs, at least 24 hrs, at least 36 hrs, at least 48 hrs, at least 60 hrs, at least 72 hrs, at least 96 hrs, at least 5 days, at least 6 days, at least 7 days, at least 8 days, at least 9 days, at least 10 days, at least 11 days, at least 12 days, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, or at least 16 weeks.

In some embodiments, C5 inhibitors (e.g., C5 inhibitor polypeptides, C5 inhibitory cyclic polypeptides, zilucoplan, and/or active metabolites or variants thereof) may exhibit long terminal t_{1/2}. Extended terminal t_{1/2} may be due to extensive target binding and/or additional plasma protein binding. In some cases, C5 inhibitors exhibit t_{1/2} values greater than 24 hours in both plasma and whole blood. In some cases, C5 inhibitors do not lose functional activity after incubation in human whole blood at 37°C for 16 hours.

In some embodiments, C5 inhibitory cyclic polypeptides (e.g., zilucoplan and/or active metabolites or variants thereof) may exhibit long terminal t_{1/2}. Extended terminal t_{1/2} may be due to extensive target binding and/or additional plasma protein binding. In some cases, C5 inhibitory cyclic polypeptides (e.g., zilucoplan and/or active metabolites or variants thereof) exhibit t_{1/2} values greater than 24 hours in both plasma and whole blood. In some cases, C5 inhibitory cyclic polypeptides (e.g., zilucoplan and/or active metabolites or variants thereof) do not lose functional activity after incubation in human whole blood at 37°C for 16 hours.

In some embodiments, dosage and/or administration are altered to modulate the steady state volume of distribution of C5 inhibitors (e.g., C5 inhibitor polypeptides, C5 inhibitory cyclic polypeptides, zilucoplan, and/or active metabolites or variants thereof). In some cases, the steady state volume of distribution of C5 inhibitors is from about 0.1 mL/kg to about 1 mL/kg, from about 0.5 mL/kg to about 5 mL/kg, from about 1 mL/kg to about 10 mL/kg, from about 5 mL/kg to about 20 mL/kg, from about 15 mL/kg to about 30 mL/kg, from about 10 mL/kg to about 200 mL/kg, from about 20 mL/kg to about 60 mL/kg, from about 30 mL/kg to about 70 mL/kg, from about 50 mL/kg to about 200 mL/kg, from about 100 mL/kg to about 500 mL/kg, or at least 500 mL/kg. In some cases, the dosage and/or administration of C5 inhibitors is adjusted to ensure that the steady state volume of distribution is equal to at least 50% of total blood volume. In some embodiments, C5 inhibitor distribution may be restricted to the plasma compartment.

In some embodiments, dosage and/or administration are altered to modulate the steady state volume of distribution of C5 inhibitory cyclic polypeptides (e.g., zilucoplan and/or active metabolites or variants thereof). In some cases, the steady state volume of distribution of C5 inhibitory cyclic polypeptides (e.g., zilucoplan and/or active metabolites or variants thereof) is from about 0.1 mL/kg to about 1 mL/kg, from about 0.5 mL/kg to about 5 mL/kg, from about 1 mL/kg to about 10 mL/kg, from about 5 mL/kg to about 20 mL/kg, from about 15 mL/kg to about 30 mL/kg, from about 10 mL/kg to about 200 mL/kg, from about 20 mL/kg to about 60 mL/kg, from about 30 mL/kg to about 70 mL/kg, from about 50 mL/kg to about 200 mL/kg, from about 100 mL/kg to about 500 mL/kg, or at least 500 mL/kg. In some cases, the dosage and/or administration of C5 inhibitory cyclic polypeptides (e.g., zilucoplan and/or active metabolites or variants thereof) is adjusted to ensure that the steady state volume of distribution is equal to at least 50% of total blood volume. In some embodiments, distribution of C5 inhibitory cyclic polypeptides (e.g., zilucoplan and/or active metabolites or variants thereof) may be restricted to the plasma compartment.

In some embodiments, C5 inhibitors (e.g., C5 inhibitor polypeptides, C5 inhibitory cyclic polypeptides, zilucoplan, and/or active metabolites or variants thereof) exhibit a total clearance rate of from about 0.001 mL/hr/kg to about 0.01 mL/hr/kg, from about 0.005 mL/hr/kg to about 0.05 mL/hr/kg, from about 0.01 mL/hr/kg to about 0.1 mL/hr/kg, from about 0.05 mL/hr/kg to about 0.5 mL/hr/kg, from about 0.1 mL/hr/kg to about 1 mL/hr/kg, from about 0.5 mL/hr/kg to about 5 mL/hr/kg, from about 0.04 mL/hr/kg to about 4 mL/hr/kg, from about 1 mL/hr/kg to about 10 mL/hr/kg, from about 5 mL/hr/kg to about 20 mL/hr/kg, from about 15 mL/hr/kg to about 30 mL/hr/kg, or at least 30 mL/hr/kg.

In some embodiments, C5 inhibitory cyclic polypeptides (e.g., zilucoplan and/or active metabolites or variants thereof) exhibit a total clearance rate of from about 0.001 mL/hr/kg to about 0.01 mL/hr/kg, from about 0.005 mL/hr/kg to about 0.05 mL/hr/kg, from about 0.01 mL/hr/kg to about 0.1 mL/hr/kg, from about 0.05 mL/hr/kg to about 0.5 mL/hr/kg, from about 0.1 mL/hr/kg to about 1 mL/hr/kg, from about 0.5 mL/hr/kg to about 5 mL/hr/kg, from about 0.04 mL/hr/kg to about 4 mL/hr/kg, from about 1 mL/hr/kg to about 10 mL/hr/kg, from about 5 mL/hr/kg to about 20 mL/hr/kg, from about 15 mL/hr/kg to about 30 mL/hr/kg, or at least 30 mL/hr/kg.

Time periods for which maximum concentration of C5 inhibitors (e.g., C5 inhibitor polypeptides, C5 inhibitory cyclic polypeptides, zilucoplan, and/or active metabolites or variants thereof) in subjects (e.g., in subject serum) are maintained (Tₘₐₓ values) may be adjusted by altering dosage and/or administration (e.g., subcutaneous administration). In some cases, C5 inhibitors have Tₘₐₓ values of from about 1 min to about 10 min, from about 5 min to about 20 min, from about 15 min to about 45 min, from about 30 min to about 60 min, from about 45 min to about 90 min, from about 1 hour to about 48 hrs, from about 2 hrs to about 10 hrs, from about 5 hrs to about 20 hrs, from about 10 hrs to about 60 hrs, from about 1 day to about 4 days, from about 2 days to about 10 days, or at least 10 days.

Time periods for which maximum concentration of C5 inhibitory cyclic polypeptides (e.g., zilucoplan and/or active metabolites or variants thereof) in subjects (e.g., in subject serum) are maintained (Tₘₐₓ values) may be adjusted by altering dosage and/or administration (e.g., subcutaneous administration). In some cases, C5 inhibitory cyclic polypeptides (e.g., zilucoplan and/or active metabolites or variants thereof) have Tₘₐₓ values of from about 1 min to about 10 min, from about 5 min to about 20 min, from about 15 min to about 45 min, from about 30 min to about 60 min, from about 45 min to about 90 min, from about 1 hour to about 48 hrs, from about 2 hrs to about 10 hrs, from about 5 hrs to about 20 hrs, from about 10 hrs to about 60 hrs, from about 1 day to about 4 days, from about 2 days to about 10 days, or at least 10 days.

In some embodiments, C5 inhibitors (e.g., C5 inhibitor polypeptides, C5 inhibitory cyclic polypeptides, zilucoplan, and/or active metabolites or variants thereof) described herein and/or compositions thereof may be administered without off-target effects. In some cases, C5 inhibitors do not inhibit hERG (human ether-a-go-go related gene), even with concentrations less than or equal to 300 µM. SC injection of C5 inhibitors with dose levels up to 10 mg/kg may be well tolerated and not result in any adverse effects of the cardiovascular system (e.g., elevated risk of prolonged ventricular repolarization) and/or respiratory system.

In some embodiments, C5 inhibitory cyclic polypeptides (e.g., zilucoplan and/or active metabolites or variants thereof) may be administered without off-target effects. In some cases, C5 inhibitory cyclic polypeptides (e.g., zilucoplan and/or active metabolites or variants thereof) do not inhibit hERG, even with concentrations less than or equal to 300 µM. SC injection of C5 inhibitory cyclic polypeptides (e.g., zilucoplan and/or active metabolites or variants thereof) with dose levels up to 10 mg/kg may be well tolerated and not result in any adverse effects of the cardiovascular system (e.g., elevated risk of prolonged ventricular repolarization) and/or respiratory system.

Doses of C5 inhibitors (e.g., C5 inhibitor polypeptides, C5 inhibitory cyclic polypeptides, zilucoplan, and/or active metabolites or variants thereof) may be determined using the no observed adverse effect level (NOAEL) observed in another species. Such species may include, but are not limited to monkeys, rats, rabbits, and mice. In some cases, human equivalent doses (HEDs) may be determined by allometric scaling from NOAELs observed in other species. In some cases, HEDs result in therapeutic margins of from about 2 fold to about 5 fold, from about 4 fold to about 12 fold, from about 5 fold to about 15 fold, from about 10 fold to about 30 fold, or at least 30 fold. In some cases, therapeutic margins are determined by using exposure in primates and estimated human Cₘₐₓ levels in humans.

In some embodiments, C5 inhibitors (e.g., C5 inhibitor polypeptides, C5 inhibitory cyclic polypeptides, zilucoplan, and/or active metabolites or variants thereof) of the present disclosure allow for a rapid washout period in cases of infection where prolonged inhibition of the complement system is detrimental.

Administration of C5 inhibitors (e.g., C5 inhibitor polypeptides, C5 inhibitory cyclic polypeptides, zilucoplan, and/or active metabolites or variants thereof) described herein and/or compositions thereof may be modified to reduce potential clinical risks to subjects. Infection with *Neisseria meningitidis* is a known risk of C5 inhibitors, including eculizumab. In some cases, risk of infection with *Neisseria meningitides* is minimized by instituting one or more prophylactic steps. Such steps may include the exclusion of subjects who may already be colonized by these bacteria. In some cases, prophylactic steps may include coadministration with one or more antibiotics. In some cases, ciprofloxacin may be coadministered. In some cases, ciprofloxacin may be coadministered orally at a dose of from about 100 mg to about 1000 mg (e.g., 500 mg).

In some embodiments, C5 inhibitors (e.g., C5 inhibitor polypeptides, C5 inhibitory cyclic polypeptides, zilucoplan, and/or active metabolites or variants thereof) described herein and/or compositions thereof are administered at a frequency of every hour, every 2 hrs, every 4 hrs, every 6 hrs, every 12 hrs, every 18 hrs, every 24 hrs, every 36 hrs, every 72 hrs, every 84 hrs, every 96 hrs, every 5 days, every 7 days, every 10 days, every 14 days, every week, every two weeks, every 3 weeks, every 4 weeks, every month, every 2 months, every 3 months, every 4 months, every 5 months, every 6 months, every year, or at least every year. In some cases, C5 inhibitors are administered once daily or administered as two, three, or more sub-doses at appropriate intervals throughout the day.

In some embodiments, C5 inhibitory cyclic polypeptides (e.g., zilucoplan and/or active metabolites or variants thereof) are administered at a frequency of every hour, every 2 hrs, every 4 hrs, every 6 hrs, every 12 hrs, every 18 hrs, every 24 hrs, every 36 hrs, every 72 hrs, every 84 hrs, every 96 hrs, every 5 days, every 7 days, every 10 days, every 14 days, every week, every two weeks, every 3 weeks, every 4 weeks, every month, every 2 months, every 3 months, every 4 months, every 5 months, every 6 months, every year, or at least every year. In some cases, C5 inhibitory cyclic polypeptides (e.g., zilucoplan and/or active metabolites or variants thereof) are administered once daily or administered as two, three, or more sub-doses at appropriate intervals throughout the day.

In some embodiments, C5 inhibitors (e.g., C5 inhibitor polypeptides, C5 inhibitory cyclic polypeptides, zilucoplan, and/or active metabolites or variants thereof) described herein and/or compositions thereof are administered in multiple daily doses. In some cases, C5 inhibitors are administered daily for 7 days. In some cases, C5 inhibitors described herein and/or compositions thereof are administered daily for 7 to 100 days. In some cases, C5 inhibitors described herein and/or compositions thereof are administered daily for at least 100 days. In some cases, C5 inhibitors described herein and/or compositions thereof are administered daily for an indefinite period.

In some embodiments, C5 inhibitory cyclic polypeptides (e.g., zilucoplan and/or active metabolites or variants thereof) are administered in multiple daily doses. In some cases, C5 inhibitory cyclic polypeptides (e.g., zilucoplan and/or active metabolites or variants thereof) are administered daily for 7 days. In some cases, C5 inhibitory cyclic polypeptides (e.g., zilucoplan and/or active metabolites or variants thereof) are administered daily for 7 to 100 days. In some cases, C5 inhibitory cyclic polypeptides (e.g., zilucoplan and/or active metabolites or variants thereof) are administered daily for at least 100 days. In some cases, C5 inhibitory cyclic polypeptides (e.g., zilucoplan and/or active metabolites or variants thereof) are administered daily for an indefinite period.

C5 inhibitors (e.g., C5 inhibitor polypeptides, C5 inhibitory cyclic polypeptides, zilucoplan, and/or active metabolites or variants thereof) described herein and/or compositions thereof delivered intravenously may be delivered by infusion over a period of time, such as over a 5 minute, 10 minute, 15 minute, 20 minute, or 25 minute period. The administration may be repeated, for example, on a regular basis, such as hourly, daily, weekly, biweekly (*i.e*., every two weeks), for one month, two months, three months, four months, or more than four months. After an initial treatment regimen, treatments may be administered on a less frequent basis. For example, after biweekly administration for three months, administration may be repeated once per month, for six months or a year or longer. C5 inhibitor administration may reduce, lower, increase or alter binding or any physiologically deleterious process (e.g., in a cell, tissue, blood, urine or other compartment of a patient) by at least 10%, at least 15%, at least 20%, at least 25%, at least 30%, at least 40%, at least 50%, at least 60%, at least 70%, at least 80 % or at least 90% or more.

C5 inhibitory cyclic polypeptides (e.g., zilucoplan and/or active metabolites or variants thereof) delivered intravenously may be delivered by infusion over a period of time, such as over a 5 minute, 10 minute, 15 minute, 20 minute, or 25 minute period. The administration may be repeated, for example, on a regular basis, such as hourly, daily, weekly, biweekly (*i.e*., every two weeks), for one month, two months, three months, four months, or more than four months. After an initial treatment regimen, treatments may be administered on a less frequent basis. For example, after biweekly administration for three months, administration may be repeated once per month, for six months or a year or longer. Administration of C5 inhibitory cyclic polypeptides (e.g., zilucoplan and/or active metabolites or variants thereof) may reduce, lower, increase or alter binding or any physiologically deleterious process (e.g., in a cell, tissue, blood, urine or other compartment of a patient) by at least 10%, at least 15%, at least 20%, at least 25%, at least 30%, at least 40%, at least 50%, at least 60%, at least 70%, at least 80 % or at least 90% or more.

Before administration of a full dose of C5 inhibitors (e.g., C5 inhibitor polypeptides, C5 inhibitory cyclic polypeptides, zilucoplan, and/or active metabolites or variants thereof) described herein and/or compositions thereof, patients can be administered a smaller dose, such as 5% of a full dose, and monitored for adverse effects, such as an allergic reaction or infusion reaction, or for elevated lipid levels or blood pressure. In another example, patients can be monitored for unwanted immunostimulatory effects, such as increased cytokine (*e.g*., TNF-alpha, IL-1, IL-6, or IL-10) levels.

Genetic predisposition plays a role in the development of some diseases or disorders. Therefore, patients in need of C5 inhibitors (e.g., C5 inhibitor polypeptides, C5 inhibitory cyclic polypeptides, zilucoplan, and/or active metabolites or variants thereof) may be identified by family history analysis, or, for example, screening for one or more genetic markers or variants. Healthcare providers (e.g., doctors or nurses) or family members may analyze family history information before prescribing or administering therapeutic compositions of the present disclosure.

### III. Kits and Devices

In some embodiments, the present disclosure provides kits and devices. Such kits and devices may include any of the compounds (e.g., therapeutic agents, C5 inhibitors, C5 inhibitor polypeptides, C5 inhibitory cyclic polypeptides, zilucoplan, and/or active metabolites or variants thereof) described herein and/or compositions thereof. Such kits and devices may include C5 inhibitory cyclic polypeptides (e.g., zilucoplan and/or active metabolites or variants thereof) or compositions described herein. In a non-limiting example, zilucoplan and/or compositions thereof may be included. Such kits may be used to carry out methods of treating complement-related indications described herein.

Kit components may be packaged in liquid (e.g., aqueous or organic) media or in dry (e.g., lyophilized) form. Kits may include containers that may include, but are not limited to vials, test tubes, flasks, bottles, syringes, or bags. Kit containers may be used to aliquot, store, preserve, insulate, and/or protect kit components. Kit components may be packaged together or separately. Some kits may include containers of sterile, pharmaceutically acceptable buffer and/or other diluent (e.g., phosphate buffered saline). In some embodiments, kits include containers of kit components in dry form with separate containers of solution for dissolving dried components. In some embodiments, kits include a syringe for administering one or more kit components.

When polypeptides are provided as a dried powder it is contemplated that between 10 micrograms and 1000 milligrams of polypeptide, or at least or at most those amounts are provided in kits.

Containers may include at least one vial, test tube, flask, bottle, syringe and/or other receptacle, into which polypeptide formulations may be placed, preferably, suitably allocated. Kits may also include containers for sterile, pharmaceutically acceptable buffer and/or other diluent.

Kits may include instructions for employing kit components as well the use of any other reagent not included in the kit. Instructions may include variations that can be implemented.

In some embodiments, the present disclosure provides kits that include syringes with zilucoplan and instructions. Syringes may be self-injection devices. Self-injection devices may include BD ULTRASAFE PLUS^{™} self-administration devices (BD, Franklin Lakes, NJ). Kits may include one or more items for addressing syringe wounds. Such items may include, but are not limited to, alcohol wipes and wound dressings (e.g., cotton balls, mesh pads, bandages, tape, gauze, etc.). Kits may further include disposal containers for disposal of used kit components. Disposal containers may be designed for disposal of sharp objects, such as needles and syringes. Some kits may include instructions for sharp object disposal.

In some embodiments, kits of the present disclosure include zilucoplan in powdered form or in solution. Solutions may be aqueous solutions. Solutions may include PBS. Zilucoplan solutions may include from about 4 mg/ml to about 200 mg/ml zilucoplan. In some embodiments, zilucoplan solutions include about 40 mg/ml zilucoplan. Zilucoplan solutions may include preservatives. In some embodiments, zilucoplan solutions are preservative-free.

### IV. Definitions

*Administered in combination:* As used herein, the term "administered in combination" or "combined administration" means that a subject is simultaneously exposed to two or more agents administered at the same time or within an interval of time such that the subject is at some point in time simultaneously exposed to both and/or such that there may be an overlap in the effect of each agent on the patient. In some embodiments, at least one dose of one or more agents is administered within about 24 hours, 12 hours, 6 hours, 3 hours, 1 hour, 30 minutes, 15 minutes, 10 minutes, 5 minutes, or 1 minute of at least one dose of one or more other agents. In some embodiments, administration occurs in overlapping dosage regimens. As used herein, the term "dosage regimen" refers to a plurality of doses spaced apart in time. Such doses may occur at regular intervals or may include one or more hiatus in administration. *Bioavailability*: As used herein, the term "bioavailability" refers to the systemic availability of a given amount of a compound (e.g., C5 inhibitor) administered to a subject. Bioavailability can be assessed by measuring the area under the curve (AUC) or the maximum serum or plasma concentration (Cₘₐₓ) of the unchanged form of a compound following administration of the compound to a subject. AUC is a determination of the area under the curve when plotting the serum or plasma concentration of a compound along the ordinate (Y-axis) against time along the abscissa (X-axis). Generally, the AUC for a particular compound can be calculated using methods known to those of ordinary skill in the art and/or as described in G. S. Banker, Modern Pharmaceutics, Drugs and the Pharmaceutical Sciences, v. 72, Marcel Dekker, New York, Inc., 1996, the contents of which are herein incorporated by reference in their entirety.

*Biological system*: As used herein, the term "biological system" refers to a cell, a group of cells, a tissue, an organ, a group of organs, an organelle, a biological fluid, a biological signaling pathway (e.g., a receptor-activated signaling pathway, a charge-activated signaling pathway, a metabolic pathway, a cellular signaling pathway, etc.), a group of proteins, a group of nucleic acids, or a group of molecules (including, but not limited to biomolecules) that carry out at least one biological function or biological task within cellular membranes, cellular compartments, cells, cell cultures, tissues, organs, organ systems, organisms, multicellular organisms, biological fluids, or any biological entities. In some embodiments, biological systems are cell signaling pathways that include intracellular and/or extracellular signaling biomolecules. In some embodiments, biological systems include proteolytic cascades (e.g., the complement cascade).

*Buffering agent*: As used herein, the term "buffering agent" refers to a compound used in a solution for the purposes of resisting changes in pH. Such compounds may include, but are not limited to acetic acid, adipic acid, sodium acetate, benzoic acid, citric acid, sodium benzoate, maleic acid, sodium phosphate, tartaric acid, lactic acid, potassium metaphosphate, glycine, sodium bicarbonate, potassium phosphate, sodium citrate, and sodium tartrate.

*Clearance rate*: As used herein, the term "clearance rate" refers to the velocity at which a particular compound is cleared from a biological system or fluid.

*Compound:* As used herein, the term "compound," refers to a distinct chemical entity. In some embodiments, a particular compound may exist in one or more isomeric or isotopic forms (including, but not limited to stereoisomers, geometric isomers and isotopes). In some embodiments, a compound is provided or utilized in only a single such form. In some embodiments, a compound is provided or utilized as a mixture of two or more such forms (including, but not limited to a racemic mixture of stereoisomers). Those of skill in the art will appreciate that some compounds exist in different forms, show different properties and/or activities (including, but not limited to biological activities). In such cases it is within the ordinary skill of those in the art to select or avoid particular forms of a compound for use in accordance with the present disclosure. For example, compounds that contain asymmetrically substituted carbon atoms can be isolated in optically active or racemic forms.

*Cyclic or Cyclized:* As used herein, the term "cyclic" refers to the presence of a continuous loop. The continuous loop may be formed by a chemical bond between different regions of a compound (also referred to herein as a "cyclic bond.") Cyclic molecules need not be circular, only joined to form an unbroken chain of subunits. Cyclic polypeptides may include a "cyclic loop," formed when two amino acids are connected by a bridging moiety. The cyclic loop comprises the amino acids along the polypeptide present between the bridged amino acids. Cyclic loops may include 2, 3, 4, 5, 6, 7, 8, 9, 10 or more amino acids.

*Downstream event*: As used herein, the term "downstream" or "downstream event," refers to any event occurring after and/or as a result of another event. In some cases, downstream events are events occurring after and as a result of C5 cleavage and/or complement activation. Such events may include, but are not limited to, generation of C5 cleavage products, activation of MAC, hemolysis, and hemolysis-related disease (e.g., PNH).

*Equilibrium dissociation constant*: As used herein, the term "equilibrium dissociation constant" or "K*_{D}*" refers to a value representing the tendency of two or more agents (e.g., two proteins) to reversibly separate. In some cases, K_{D} indicates a concentration of a primary agent at which half of the total levels of a secondary agent are associated with the primary agent.

*Half-life*: As used herein, the term "half-life" or "t_{1/2}" refers to the time it takes for a given process or compound concentration to reach half of a final value. The "terminal half-life" or "terminal t_{1/2}" refers to the time needed for the plasma concentration of a factor to be reduced by half after the concentration of the factor has reached a pseudo-equilibrium.

*Identity*: As used herein, the term "identity," when referring to polypeptides or nucleic acids, refers to a comparative relationship between sequences. The term is used to describe the degree of sequence relatedness between polymeric sequences and may include the percentage of matching monomeric components with gap alignments (if any) addressed by a particular mathematical model or computer program (i.e., "algorithms"). Identity of related polypeptides can be readily calculated by known methods. Such methods include, but are not limited to, those described previously by others (Lesk, A. M., ed., Computational Molecular Biology, Oxford University Press, New York, 1988; Smith, D. W., ed., Biocomputing: Informatics and Genome Projects, Academic Press, New York, 1993; Griffin, A. M. et al., ed., Computer Analysis of Sequence Data, Part 1, Humana Press, New Jersey, 1994; von Heinje, G., Sequence Analysis in Molecular Biology, Academic Press, 1987; Gribskov, M. et al., ed., Sequence Analysis Primer, M. Stockton Press, New York, 1991; and Carillo et al., Applied Math, SIAM J, 1988, 48, 1073).

*Inhibitor*: As used herein, the term "inhibitor" refers to any agent that blocks or causes a reduction in the occurrence of a specific event; cellular signal; chemical pathway; enzymatic reaction; cellular process; interaction between two or more entities; biological event; disease; disorder; or condition.

*Initial loading dose*: As used herein, an "initial loading dose" refers to a first dose of a therapeutic agent that may differ from one or more subsequent doses. Initial loading doses may be used to achieve an initial concentration of a therapeutic agent or level of activity before subsequent doses are administered.

*Intravenous*: As used herein, the term "intravenous" refers to the area within a blood vessel. Intravenous administration typically refers to delivery of a compound into the blood through injection in a blood vessel (e.g., vein).

*In vitro*: As used herein, the term "*in vitro*" refers to events that occur in an artificial environment (e.g., in a test tube or reaction vessel, in cell culture, in a Petri dish, *etc*.), rather than within an organism (*e.g*., animal, plant, or microbe).

*In vivo*: As used herein, the term "*in vivo*" refers to events that occur within an organism (e.g., animal, plant, or microbe or cell or tissue thereof).

*Lactam bridge*: As used herein, the term "lactam bridge" refers to an amide bond that forms a bridge between chemical groups in a molecule. In some cases, lactam bridges are formed between amino acids in a polypeptide.

*Linker*: The term "linker" as used herein refers to a group of atoms (*e.g*., 10-1,000 atoms), molecule(s), or other compounds used to join two or more entities. Linkers may join such entities through covalent or non-covalent (e.g., ionic or hydrophobic) interactions. Linkers may include chains of two or more polyethylene glycol (PEG) units. In some cases, linkers may be cleavable.

*Minute volume*: As used herein, the term "minute volume" refers to the volume of air inhaled or exhaled from a subject's lungs per minute.

*Non-proteinogenic*: As used herein, the term "non-proteinogenic" refers to any non-natural proteins, such as those with non-natural components, such as non-natural amino acids.

*Patient:* As used herein, "patient" refers to a subject who may seek or be in need of treatment, requires treatment, is receiving treatment, will receive treatment, or a subject who is under the care of a trained professional for a particular disease or condition.

*Pharmaceutical composition*: As used herein, the term "pharmaceutical composition" refers to a composition with at least one active ingredient (e.g., a C5 inhibitor) in a form and amount that permits the active ingredient to be therapeutically effective.

*Pharmaceutically acceptable*: The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

*Pharmaceutically acceptable excipients:* The phrase "pharmaceutically acceptable excipient," as used herein, refers to any ingredient other than active agents (e.g., active agent zilucoplan and/or active metabolites thereof or variants thereof) present in a pharmaceutical composition and having the properties of being substantially nontoxic and non-inflammatory in a patient. In some embodiments, a pharmaceutically acceptable excipient is a vehicle capable of suspending or dissolving the active agent. Excipients may include, for example: anti adherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration. Exemplary excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E, vitamin C, and xylitol.

*Plasma compartment*: As used herein, the term "plasma compartment" refers to intravascular space occupied by blood plasma.

*Salt*: As used herein, the term "salt" refers to a compound made up of a cation with a bound anion. Such compounds may include sodium chloride (NaCl) or other classes of salts including, but not limited to acetates, chlorides, carbonates, cyanides, nitrites, nitrates, sulfates, and phosphates. The term "salt" may also be used to refer to salt forms of polypeptides described herein (e.g., zilucoplan salt). Such polypeptide salts may include zilucoplan sodium salt.

*Sample*: As used herein, the term "sample" refers to an aliquot or portion taken from a source and/or provided for analysis or processing. In some embodiments, a sample is from a biological source such as a tissue, cell or component part (e.g., a body fluid, including but not limited to blood, mucus, lymphatic fluid, synovial fluid, cerebrospinal fluid, saliva, amniotic fluid, amniotic cord blood, urine, vaginal fluid and semen). In some embodiments, a sample may be or include a homogenate, lysate or extract prepared from a whole organism or a subset of its tissues, cells or component parts, or a fraction or portion thereof, including but not limited to, for example, plasma, serum, spinal fluid, lymph fluid, the external sections of the skin, respiratory, intestinal, and genitourinary tracts, tears, saliva, milk, blood cells, tumors, or organs. In some embodiments, a sample is or includes a medium, such as a nutrient broth or gel, which may contain cellular components, such as proteins. In some embodiments, a "primary" sample is an aliquot of the source. In some embodiments, a primary sample is subjected to one or more processing (e.g., separation, purification, etc.) steps to prepare a sample for analysis or other use.

*Subcutaneous*: As used herein, the term "subcutaneous" refers to the space underneath the skin. Subcutaneous administration is delivery of a compound beneath the skin.

*Subject*: As used herein, the term "subject" refers to any organism to which a compound or method in accordance with the disclosure may be administered or applied, *e.g*., for experimental, diagnostic, prophylactic, and/or therapeutic purposes. Typical subjects include animals (*e.g*., mammals such as mice, rats, rabbits, porcine subjects, non-human primates, and humans).

*Substantially*: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Therapeutically effective amount:* As used herein, the term "therapeutically effective amount" means an amount of an agent to be delivered (*e.g*., C5 inhibitor) that is sufficient, when administered to a subject suffering from or susceptible to a disease, disorder, and/or condition, to treat, improve symptoms of, diagnose, prevent, and/or delay the onset of the disease, disorder, and/or condition.

*Tidal volume*: As used herein, the term "tidal volume" refers to the normal lung volume of air displaced between breaths (in the absence of any extra effort).

*Tₘₐₓ*: As used herein, the term "Tₘₐₓ" refers to the time period for which maximum concentration of a compound in a subject or fluid is maintained.

*Treating*: As used herein, the term "treating" refers to partially or completely alleviating, ameliorating, improving, relieving, delaying onset of, inhibiting progression of, reducing severity of, and/or reducing incidence of one or more symptoms or features of a particular disease, disorder, and/or condition. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition.

*Treatment dose*: As used herein, "treatment dose" refers to one or more doses of a therapeutic agent administered in the course of addressing or alleviating a therapeutic indication. Treatment doses may be adjusted to maintain a desired concentration or level of activity of a therapeutic agent in a body fluid or biological system.

*Volume of distribution*: As used herein, the term "volume of distribution" or "V_{dist}" refers to a fluid volume required to contain the total amount of a compound in the body at the same concentration as in the blood or plasma. The volume of distribution may reflect the extent to which a compound is present in the extravascular tissue. A large volume of distribution reflects the tendency of a compound to bind to tissue components compared with plasma protein components. In a clinical setting, V_{dist} can be used to determine a loading dose of a compound to achieve a steady state concentration of that compound.

### V. Equivalents and scope

While various embodiments of the invention have been particularly shown and described, it will be understood by those skilled in the art that various changes in form and details may be made therein without departing from the spirit and scope of the invention as defined by the appended claims.

Those skilled in the art will recognize or be able to ascertain using no more than routine experimentation, many equivalents to the specific embodiments in accordance with the invention described herein. The scope of the present invention is not intended to be limited to the above description, but rather is as set forth in the appended claims.

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

It is also noted that the term "comprising" is intended to be open and permits but does not require the inclusion of additional elements or steps. When the term "comprising" is used herein, the terms "consisting of" and "or including" are thus also encompassed and disclosed.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

In addition, it is to be understood that any particular embodiment of the present invention that falls within the prior art may be explicitly excluded from any one or more of the claims. Since such embodiments are deemed to be known to one of ordinary skill in the art, they may be excluded even if the exclusion is not set forth explicitly herein. Any particular embodiment of the compositions of the invention (*e.g*., any nucleic acid or protein encoded thereby; any method of production; any method of use; *etc*.) can be excluded from any one or more claims, for any reason, whether or not related to the existence of prior art.

All cited sources, for example, references, publications, databases, database entries, and art cited herein, are incorporated into this application by reference, even if not expressly stated in the citation. In case of conflicting statements of a cited source and the instant application, the statement in the instant application shall control.

Section and table headings are not intended to be limiting.

### EXAMPLES

### Example 1. PLGA particle formation and testing

Studies were carried out to develop zilucoplan-loaded PLGA microspheres particles for drug delivery. Several parameters for particle formation were manipulated to optimize zilucoplan delivery profile. Initial studies utilized both single and double PLGA emulsion formats. RESOMER^{®} 502H (Evonik, Germany), acid terminated formulation with 7,000-17,000 MW and degradation period less than 3 months, was used with different zilucoplan loading percentages.

Zilucoplan (SEQ ID NO: 1), as described in International Publication Numbers WO2017105939 and WO2018106859, was prepared as a cyclic peptide containing 15 amino acids (4 of which are non-natural amino acids), an acetylated N-terminus, and a C-terminal carboxylic acid. The C-terminal lysine of the core peptide has a modified side chain, forming a N-**ε**-(PEG24-γ-glutamic acid-N-α-hexadecanoyl) lysine reside. This modified side chain includes a polyethyleneglycol spacer (PEG24) attached to an L-γ glutamic acid residue that is derivatized with a palmitoyl group. The cyclization of zilucoplan is via a lactam bridge between the side-chains of L-Lys1 and L-Asp6. All of the amino acids in zilucoplan are L-amino acids. Zilucoplan has a molecular weight of 3562.23 g/mol and a chemical formula of C₁₇₂H₂₇₈N₂₄O₅₅.

### PLGA preparations

Zilucoplan was incorporated into poly lactic-co-glycolic acid (PLGA) particles through emulsification using either single emulsion (oil-in-water) or double emulsion (water-in-oil-in-water) format. Generally, droplets of PLGA in organic solvent (dichloromethane) were prepared at a ratio of 1 g PLGA per 10 mL organic solvent. Droplets were emulsified in an aqueous continuous phase with emulsifier [polyvinyl alcohol (PVA)] and zilucoplan present. Resulting emulsions were processed to evaporate organic solvent and form condensed zilucoplan-PLGA particles. Batches were prepared using either quench evaporation (emersion in solution that draws out organic solvent) or rotary evaporation (rotavap) methods for organic solvent evaporation. Some morphologic changes were seen with particles prepared by rotavap, with particles having collapsed or concave sphere structure when viewed by electron microscopy.

Different pore forming excipients (PFE) were also explored in particle formulations to facilitate zilucoplan release. These included 10% of medium chain triglycerides (MCTs), PLURONIC^{®} F-127 (BASF, Florham Park, NJ), or poly (ethylene glycol) MW 3400 (PEG3400). Ultimately, PFEs were excluded as high zilucoplan load formulations were found to yield good drug release without PFEs.

Double emulsion formulations demonstrated 12.5% initial burst with 29% zilucoplan loading, that more than doubled when zilucoplan loading was raised to 32%. Early single emulsion formulations with low zilucoplan loading percentage (11.24%) demonstrated low initial burst percentage (5.6%). Scaling the process to 1 gram yielded an even lower initial burst (5%) with higher zilucoplan loading percentage (14%). Percent zilucoplan loading of 34.3% with initial burst below 5% was achieved by removing DMSO from formulations.

Single emulsion formulations with 36-37.5% zilucoplan loading percentage had the highest allowable load before burst values became excessive (likely due to level of surface associated zilucoplan). These high load formulations were selected for further analysis. Both quench processed and rotavap processed formats were tested. Scanning electron microscopy and energy dispersive X-Ray spectroscopy were used to confirm particle uniformity with homogenous zilucoplan distribution, respectively. Particles were generally spherical, with few internal voids. Elemental mapping of zilucoplan-Na+ indicated homogenous distribution throughout.

### Animal studies

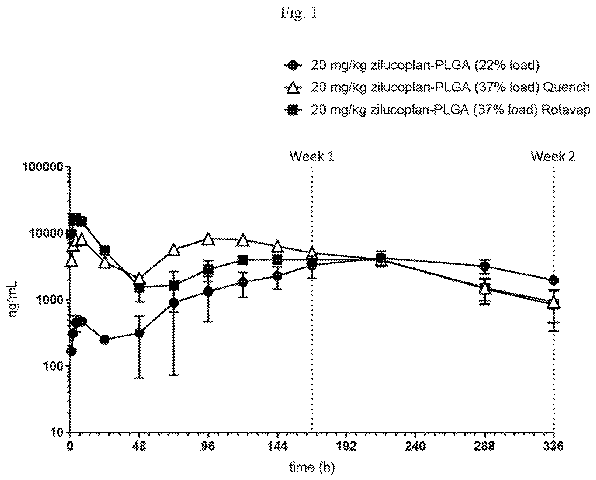

Zilucoplan PLGA formulations with 37% zilucoplan loading prepared by quench processing or rotavap processing were administered to rats and compared to preparations with 22% zilucoplan loading. Formulations were administered to rats by subcutaneous injection and zilucoplan levels were determined by mass spectrometry analysis in rat plasma samples obtained at various time points after administration. Zilucoplan levels were higher in samples obtained through week 1 from rats receiving the higher load formulations (see Fig. 1). Particles prepared by quench processing demonstrated lower initial burst level, but higher sample levels by 48 hours after administration that continued through the end of week 1.

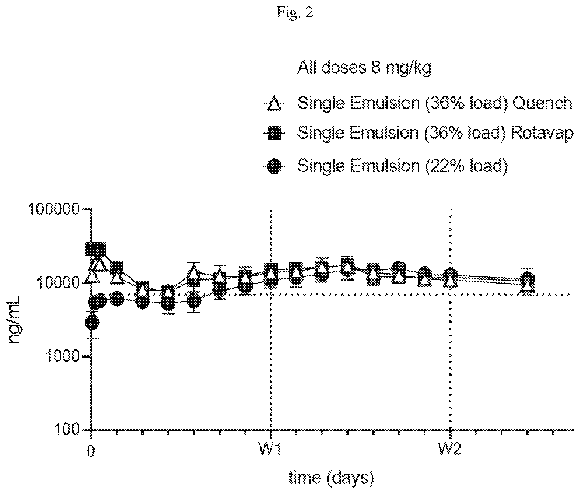

A similar study was carried out in non-human primates (NHPs), but with 36% zilucoplan loading in high load formulations. Formulations were administered to *Cynomolgus* monkeys at a fixed dose of 8 mg/kg. Similar to the rat studies, a higher initial burst was observed with higher zilucoplan loading percentage formulations (see Fig. 2). The observed burst was useful for exceeding the 95% effective concentration (EC95) of 8,000 ng/mL during the first few days. By day 17 after administration, the 22% zilucoplan loading formulation yielded an area under the curve (AUC) of 4,359,795 ng*h/mL, while the higher load quench formulation yielded an AUC of 5,141,124 ng*h/mL and the higher load rotavap formulation yielded an AUC of 5,389,580 ng*h/mL.

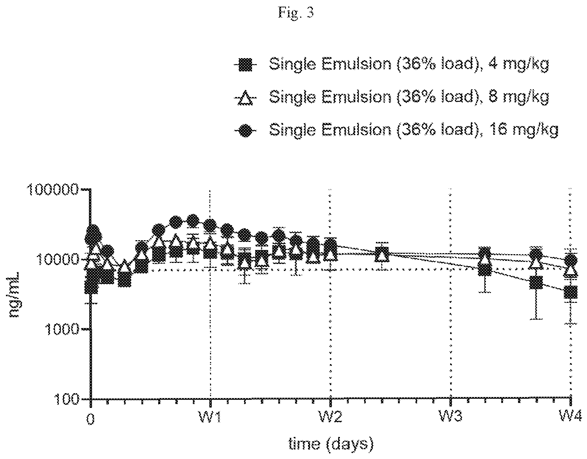

The same study was repeated with varying doses using 36% zilucoplan loading formulations prepared with quench processing. 4, 8, and 16 mg/kg doses were tested to assess dose-exposure relationship. Initial time points out to 4 weeks after administration demonstrated clear dose-related exposures (see Fig. 3). 4 mg/kg dose yielded an AUC of 6,417,235 ng*h/mL, 8 mg/kg dose yielded an AUC of 7,865,963 ng*h/mL, and 16 mg/kg dose yielded an AUC of 11,375,456 ng*h/mL. Comparison between results from the 8 mg/kg dose and the 8 mg/kg dose from the prior study showed similar AUC at day 17 after treatment (5,276,571 ng*h/mL versus 5,141,124 ng*h/mL) and similar maximum concentration (Cmax) for zilucoplan (18,476 ng/mL versus 18,558 ng/mL).

Percent hemolysis associated with plasma samples was determined for samples obtained at multiple time points after subcutaneous zilucoplan-PLGA (formulations with 36% zilucoplan loading, prepared using quench processing) administration in NHPs. *Cynomolgus* monkeys received an initial loading dose of 16 mg/kg zilucoplan-PLGA on day 1 and 10 mg/kg doses on days 8 and 15, thereafter. Greater than 90% levels of hemolysis inhibition were maintained throughout the week following initial administration. Hemolysis levels decreased further after the second dose on day 8. By the end of the second week, hemolysis levels fell to levels consistent with those observed in humans with daily subcutaneous administration of non-extended release formulations (0.3 mg/kg dose zilucoplan formulated at a concentration of 40 mg/mL in buffer with 50 mM sodium phosphate and 75.7 mM sodium chloride at 7.0 pH) for the same time frame (< 95%).

### Example 2. Human dosing predictions

Profiles from zilucoplan-PLGA formulations tested in NHPs (36% zilucoplan loading with quench processing) were used to predict suitable dosing for human studies using a human PK model. The model utilized an input profile based on deconvolution of the NHP study data and assumed a 75 kg human subject with weekly and biweekly dosing up to steady state. Based on the model, expected dose ranges for weekly human dosing were between 100 mg and 200 mg and between 200 mg and 300 mg for biweekly dosing.

100 mg weekly dose of zilucoplan-PLGA was predicted to yield daily zilucoplan plasma concentrations equivalent to those of 0.3 mg/kg daily subcutaneous dosing formulations (40 mg/mL solution with 50 mM sodium phosphate and 75.7 mM sodium chloride at a pH of 7.0), an approximately 35% reduction in amount of zilucoplan administered in comparison to daily dosing. Weekly dosing was predicted to have a narrow range between minimum concentration (Cmin) and Cmax. Without a loading regimen, weekly dosing was predicted to reach effective plasma concentrations (5,000 ng/mL) within 10 days. 200 mg biweekly dose of zilucoplan-PLGA was predicted to maintain effective drug concentrations, with 0.3 mg/kg plasma concentration equivalents for 10 out of 14 days. Overall, zilucoplan-PLGA formulations were predicted to achieve approximately 33% dose efficiency compared to 7 or 14 day doses of standard, immediate release formulations.
The present disclosure also includes the following items.
1. A sustained release formulation comprising:
   a polypeptide, wherein the polypeptide comprises a C5 inhibitory cyclic polypeptide comprising the amino acid sequence of SEQ ID NO: 1; and
   a release modulating matrix comprising a polymer, wherein the polymer comprises poly lactic-co-glycolic acid (PLGA).
2. The sustained release formulation of item 1, wherein the release modulating matrix comprises a particle.
3. The sustained release formulation of item 2, wherein the particle comprises a microparticle.
4. The sustained release formulation of any one of items 1-3, wherein the PLGA comprises a ratio of poly lactic acid to poly glycolic acid of from about 25:75 to about 75:25.
5. The sustained release formulation of item 4, wherein the PLGA comprises a ratio of poly lactic acid to poly glycolic acid of about 50:50.
6. The sustained release formulation of any of items 1-5, wherein the release modulating matrix comprises an emulsion.
7. The sustained release formulation of item 6, wherein the emulsion comprises a single emulsion.
8. The sustained release formulation of item 6, wherein the emulsion comprises a double emulsion.
9. The sustained release formulation of any of items 1-8, wherein the sustained release formulation comprises an excipient.
10. The sustained release formulation of item 9, wherein the excipient comprises a pore forming excipient (PFE).
11. The sustained release formulation of item 10, wherein the PFE is selected from the group consisting of medium chain triglycerides, PLURONIC^{®} F-127, and poly (ethylene glycol) (PEG).
12. The sustained release formulation of any one of items 2-11, wherein the particle comprises a diameter of from about 5 µm to about 200 µm.
13. The sustained release formulation of any one of items 1-12, wherein the polypeptide comprises a cyclic bond.
14. The sustained release formulation of any one of items 1-13, wherein the polypeptide comprises a modified lysine residue.
15. The sustained release formulation of any one of items 1-14, wherein the polypeptide comprises a lipid moiety.
16. The sustained release formulation of any one of items 1-15, wherein the polypeptide comprises a PEG moiety.
17. The sustained release formulation of any one of items 1-16, wherein the polypeptide comprises zilucoplan.
18. The sustained release formulation of any one of items 1-17, wherein the sustained release formulation comprises from about 10% to about 95% of the release modulating matrix by percent weight.
19. The sustained release formulation of item 18, wherein the sustained release formulation comprises from about 50% to about 90% of the release modulating matrix by percent weight.
20. The sustained release formulation of any one of items 1-19, wherein the sustained release formulation comprises from about 10% to about 50% of the polypeptide by percent weight.
21. The sustained release formulation of any one of items 1-20, wherein the polypeptide is uniformly distributed in the release modulating matrix.
22. A method of preparing the sustained release formulation of any one of items 1-21, the method comprising:
   preparing an organic phase solution, wherein the organic phase solution is prepared by combining an organic solvent, PLGA, and the polypeptide;
   preparing an aqueous phase solution, wherein the aqueous phase solution is prepared by combining an aqueous solution with an emulsion stabilizer; and
   preparing the sustained release formulation by preparing an emulsion of the organic phase solution and the aqueous phase solution.
23. The method of item 22, wherein the organic solvent comprises dichloromethane (DCM).
24. The method of item 22 or 23, wherein the aqueous solution comprises phosphate buffered saline.
25. The method of any one of items 22-24, wherein the emulsion stabilizer comprises polyvinyl alcohol (PVA).
26. The method of any one of items 22-24, wherein the organic solvent is removed from the emulsion by evaporation.
27. The method of item 26, wherein the evaporation is carried out by quench evaporation.
28. The method of item 26, wherein the evaporation is carried out by rotary evaporation.
29. The method of any one of items 26-28, wherein a particle is formed as a result of the evaporation.
30. The method of item 29, wherein the polypeptide is uniformly distributed in the particle.
31. The method of any one of items 22-30, wherein the polypeptide is combined in the organic phase solution at a concentration sufficient to yield from about 10% by weight to about 50% by weight of the polypeptide in the sustained release formulation.
32. The method of item 31, wherein the polypeptide is combined in the organic phase solution at a concentration sufficient to yield from about 36% by weight to about 38% by weight of the polypeptide in the sustained release formulation.
33. The method of any one of items 22-32, wherein the polypeptide comprises zilucoplan.
34. The method of item 33, wherein the sustained release formulation comprises a zilucoplan-PLGA particle.
35. A sustained release formulation prepared according to the method of any one of items 23-34.
36. The sustained release formulation of any one of items 1-21 and 35, wherein the sustained release formulation comprises a sustained release profile comprising low initial burst for release of the polypeptide and/or capability of achieving an effective concentration of the polypeptide in a medium where the polypeptide is released from the sustained release formulation.
37. The sustained release formulation of item 36, wherein the capability of achieving the effective concentration of the polypeptide in the medium where the polypeptide is released from the sustained release formulation is extended over a specific period of time.
38. The sustained release formulation of item 36 or 37, wherein the specific period of time comprises from about one week to about three weeks.
39. The sustained release formulation of any one of items 36-38, wherein the effective concentration is from about 4,000 ng/mL to about 12,000 ng/mL.
40. The sustained release formulation of any one of items 36-39, wherein the sustained release profile comprises an initial burst for release of the polypeptide of from about 0% to about 20% of the total amount of the polypeptide included in the sustained release formulation.
41. A method of administering a polypeptide, the method comprising administering the sustained release formulation of any one of items 1-21, and 35-40.
42. The method of item 41, wherein the polypeptide comprises zilucoplan.
43. The method of item 41 or 42, wherein the sustained release formulation is administered by subcutaneous injection.
44. The method of any one of items 41-43, wherein the sustained release formulation is administered weekly or biweekly.
45. The method of any one of items 41-44, wherein the polypeptide is released from the sustained release formulation after administration.
46. The method of item 45, wherein release of the polypeptide exhibits a burst of less than 5%.
47. The method of any one of items 41-46, wherein the sustained release formulation is administered at a dose sufficient to administer from about 2 mg/kg to about 20 mg/kg of the polypeptide.
48. The method of item 47, wherein the polypeptide is zilucoplan and wherein the sustained release formulation is administered at a dose sufficient to administer from about 100 mg to about 200 mg zilucoplan.
49. A method of reducing hemolysis in a subject, the method comprising administering a sustained release formulation according to the sustained release formulation of any one of items 1-21 and 35, wherein the sustained release formulation comprises a C5 inhibitor.
50. The method of item 49, wherein the C5 inhibitor comprises zilucoplan.
51. A method of treating a complement-related indication, the method comprising administering a sustained release formulation according to the sustained release formulation of any one of items 1-21 and 35-40, wherein the sustained release formulation comprises a C5 inhibitor.
52. The method of item 51, wherein the C5 inhibitor comprises zilucoplan.
53. The method of item 51 or 52, wherein the complement-related indication comprises one or more of paroxysmal nocturnal hemoglobinuria, myasthenia gravis, an inflammatory indication, a wound, a burn, an autoimmune indication, a pulmonary indication, a cardiovascular indication, a neurological indication, a kidney-related indication, a diabetes-related indication, an ocular indication, and a pregnancy-related indication.
54. A sustained release formulation comprising:
   an active ingredient; and
   a release modulating matrix comprising a polymer, wherein the polymer comprises PLGA.
55. The sustained release formulation of item 54, wherein the release modulating matrix comprises a particle.
56. The sustained release formulation of item 55, wherein the particle comprises a nanoparticle or a microparticle.
57. The sustained release formulation of any one of items 54-56, wherein the PLGA comprises a ratio of poly lactic acid to poly glycolic acid of from about 25:75 to about 75:25.
58. The sustained release formulation of item 57, wherein the PLGA comprises a ratio of poly lactic acid to poly glycolic acid of about 50:50.
59. The sustained release formulation of any of items 54-58, wherein the release modulating matrix comprises an emulsion.
60. The sustained release formulation of item 59, wherein the emulsion comprises a single emulsion.
61. The sustained release formulation of item 59, wherein the emulsion comprises a double emulsion.
62. The sustained release formulation of any of items 54-61, wherein the sustained release formulation comprises an excipient.
63. The sustained release formulation of item 62, wherein the excipient comprises a PFE.
64. The sustained release formulation of item 63, wherein the PFE is selected from the group consisting of medium chain triglycerides, PLURONIC^{®} F-127, and PEG.
65. The sustained release formulation of any one of items 55-64, wherein the particle comprises a diameter of from about 5 µm to about 200 µm.
66. The sustained release formulation of any one of items 54-65, wherein the active ingredient comprises a therapeutic agent.
67. The sustained release formulation of any one of items 54-66, wherein the active ingredient comprises at least one non-natural amino acid.
68. The sustained release formulation of item 67, wherein the at least one non-natural amino acid is selected from any of those listed in Table 2 of United States Publication Number US 2011/0172126.
69. The sustained release formulation of any one of items 54-68, wherein the active ingredient comprises a cyclic bond.
70. The sustained release formulation of any one of items 54-69, wherein the active ingredient comprises a modified lysine residue.
71. The sustained release formulation of any one of items 54-70, wherein the active ingredient comprises a lipid moiety.
72. The sustained release formulation of any one of items 54-71, wherein the active ingredient comprises a PEG moiety.
73. The sustained release formulation of any one of items 54-72, wherein the active ingredient is a peptidomimetic.
74. The sustained release formulation of any one of items 54-73, wherein the active ingredient is a macrocyclic compound.
75. The sustained release formulation of any one of items 54-74, wherein the active ingredient comprises a C5 inhibitor.
76. The sustained release formulation of item 75, wherein the C5 inhibitor is selected from the group consisting of any of those listed in Table 1 or any of those listed in Table 1 of United States Publication Number US 2017/0137468.
77. The sustained release formulation of any one of items 54-76, wherein the sustained release formulation comprises from about 10% to about 95% of the release modulating matrix by percent weight.
78. The sustained release formulation of item 77, wherein the sustained release formulation comprises from about 50% to about 90% of the release modulating matrix by percent weight.
79. The sustained release formulation of any one of items 54-78, wherein the sustained release formulation comprises from about 10% to about 50% of the active ingredient by percent weight.
80. The sustained release formulation of any one of items 54-79, wherein the active ingredient is uniformly distributed in the release modulating matrix.
81. A method of preparing the sustained release formulation of any one of items 54-80, the method comprising:
   preparing an organic phase solution, wherein the organic phase solution is prepared by combining an organic solvent, PLGA, and the active ingredient;
   preparing an aqueous phase solution, wherein the aqueous phase solution is prepared by combining an aqueous solution with an emulsion stabilizer; and
   preparing the sustained release formulation by preparing an emulsion of the organic phase solution and the aqueous phase solution.
82. The method of item 81, wherein the organic solvent comprises DCM.
83. The method of item 81 or 82, wherein the aqueous solution comprises phosphate buffered saline.
84. The method of any one of items 81-83, wherein the emulsion stabilizer comprises PVA.
85. The method of any one of items 81-83, wherein the organic solvent is removed from the emulsion by evaporation.
86. The method of item 85, wherein the evaporation is carried out by quench evaporation.
87. The method of item 85, wherein the evaporation is carried out by rotary evaporation.
88. The method of any one of items 85-87, wherein a particle is formed as a result of the evaporation.
89. The method of item 88, wherein the active ingredient is uniformly distributed in the particle.
90. The method of any one of items 81-89, wherein the active ingredient is combined in the organic phase solution at a concentration sufficient to yield from about 10% by weight to about 50% by weight of the active ingredient in the sustained release formulation.
91. The method of item 90, wherein the active ingredient is combined in the organic phase solution at a concentration sufficient to yield from about 36% by weight to about 38% by weight of the active ingredient in the sustained release formulation.
92. A sustained release formulation prepared according to the method of any one of items 81-91.
93. The sustained release formulation of any one of items 54-80 and 92, wherein the sustained release formulation comprises a sustained release profile comprising low initial burst for release of the active ingredient and/or capability of achieving an effective concentration of the active ingredient in a medium where the active ingredient is released from the sustained release formulation.
94. The sustained release formulation of item 93, wherein the capability of achieving the effective concentration of the active ingredient in the medium where the active ingredient is released from the sustained release formulation is extended over a specific period of time.
95. The sustained release formulation of item 93 or 94, wherein the specific period of time comprises from about one week to about three weeks.
96. The sustained release formulation of any one of items 93-95, wherein the effective concentration is from about 4,000 ng/mL to about 12,000 ng/mL.
97. The sustained release formulation of any one of items 93-96, wherein the sustained release profile comprises an initial burst for release of the active ingredient of from about 0% to about 20% of the total amount of the active ingredient included in the sustained release formulation.
98. A method of administering an active ingredient, the method comprising administering the sustained release formulation of any one of items 54-80 and 92-97.
99. The method of item 98, wherein the sustained release formulation is administered by subcutaneous injection.
100. The method of item 98 or 99, wherein the sustained release formulation is administered weekly or biweekly.
101. The method of any one of items 98-100, wherein the active ingredient is released from the sustained release formulation after administration.
102. The method of item 101, wherein release of the active ingredient exhibits a burst of less than 5%.
103. The method of any one of items 98-102, wherein the sustained release formulation is administered at a dose sufficient to administer from about 2 mg/kg to about 20 mg/kg of the active ingredient.
104. The method of item 103, wherein the sustained release formulation is administered at a dose sufficient to administer from about 100 mg to about 200 mg of the active ingredient.
105. A method of reducing hemolysis in a subject, the method comprising administering an active ingredient according to the method of any one of items 98-104, wherein the active ingredient comprises a C5 inhibitor.
106. A method of treating a complement-related indication, the method comprising administering an active ingredient according to the method of any one of items 98-104, wherein the active ingredient comprises a C5 inhibitor.
107. The method of item 106, wherein the complement-related indication comprises one or more of paroxysmal nocturnal hemoglobinuria, myasthenia gravis, an inflammatory indication, a wound, a burn, an autoimmune indication, a pulmonary indication, a cardiovascular indication, a neurological indication, a kidney-related indication, a diabetes-related indication, an ocular indication, and a pregnancy-related indication.

## Claims

1. A sustained release formulation comprising:
a) a polypeptide comprising a C5 inhibitory cyclic polypeptide comprising the amino acid sequence of SEQ ID NO: 1; and
b) a release modulating matrix comprising a polymer, wherein the polymer comprises poly lactic-co-glycolic acid (PLGA).

2. The sustained release formulation according to claim 1, wherein the PLGA comprises a ratio of poly lactic acid to poly glycolic acid of from about 25:75 to about 75:25.

3. The sustained release formulation of claim 1 or claim 2, wherein the release modulating matrix comprises an emulsion.

4. The sustained release formulation according to claim 3, wherein the emulsion comprises a single or double emulsion.

5. The sustained release formulation according to any one of claims 1-4, wherein the sustained release formulation comprises a pore forming excipient (PFE).

6. The sustained release formulation according to claim 5, wherein the pore forming excipient (PFE) is selected from the group consisting of medium chain triglycerides, PLURONIC F-127, and poly (ethylene glycol) (PEG).

7. The sustained release formulation according to any one of claims 1-6, wherein the release modulating matrix comprises a particle having a diameter of from about 5 µm to about 200 µm.

8. The sustained release formulation according to any one of claims 1-7, wherein the sustained release formulation comprises from about 10% to about 95% of the release modulating matrix by percent weight.

9. The sustained release formulation according to any one of claims 1-8, wherein the sustained release formulation comprises from about 10% to about 50% of the polypeptide by percent weight.

10. The sustained release formulation according to any one of claims 1-9, wherein the polypeptide is uniformly distributed in the release modulating matrix.

11. The sustained release formulation according to any one of claims 1-10, wherein the sustained release formulation comprises a sustained release profile comprising low initial burst for release of the polypeptide and/or capability of achieving an effective concentration of the polypeptide in a medium where the polypeptide is released from the sustained release formulation.

12. The sustained release formulation according to claim 11, wherein the effective concentration is from about 4,000 ng/mL to about 12,000 ng/mL, and/or the sustained release profile comprises an initial burst for release of the polypeptide of from about 0% to about 20% of the total amount of the polypeptide included in the sustained release formulation.

13. The sustained release formulation according to any one of claims 1-12 for use in a method of treating a complement-related indication, the method comprising administering the sustained release formulation, wherein the C5 inhibitory cyclic polypeptide is zilucoplan.

14. The sustained release formulation for use according to claim 13, wherein:
a) the sustained release formulation is administered by subcutaneous injection; and/or
b) the sustained release formulation is administered weekly or biweekly.

15. The sustained release formulation for use according to claim 13 or claim 14, wherein:
a) the polypeptide is released from the sustained release formulation after administration; and/or
b) the sustained release formulation is administered at a dose sufficient to administer from about 100 mg to about 200 mg of the polypeptide.
